# EUROPÄISCHE PATENTSCHRIFT

(11) **EP 2 729 471 B1**
(45) Veröffentlichungstag und Bekanntmachung des Hinweises auf die Patenterteilung: **06.05.2015**
(21) Anmeldenummer: 12732677.5
(22) Anmeldetag: 06.07.2012
(51) Int. Cl.: C07D 498/04, A61K 31/519, A61P 43/00, A61P 9/04

(54) **CARBONSÄUREDERIVATE MIT EINEM OXAZOLO[5,4-D]PYRIMIDINRING**
CARBOXYLIC ACID DERIVATIVES WITH AN OXAZOLO[5,4-D]PYRIMIDINE RING
DÉRIVÉS D'ACIDE CARBOXYLIQUE AVEC UN ANNEAU D'OXAZOLO[5,4-D]PYRIMIDINE

(30) Priorität: 07.07.2011 EP 11305878; 11.05.2012 EP 12305526
(43) Veröffentlichungstag der Anmeldung: 14.05.2014
(73) Patentinhaber: SANOFI, 75008 Paris (FR)
(72) Erfinder: KADEREIT, Dieter, 65926 Frankfurt am Main (DE); SCHÄFER, Matthias, 65926 Frankfurt am Main (DE); HACHTEL, Stephanie, 65926 Frankfurt am Main (DE); HUEBSCHLE, Thomas, 65926 Frankfurt am Main (DE); HISS, Katrin, 65926 Frankfurt am Main (DE)
(74) Vertreter: Michalski Hüttermann & Partner Patentanwälte mbB
(86) Internationale Anmeldenummer: PCT/EP2012/063298
(87) Internationale Veröffentlichungsnummer: WO 2013/004827

(56) Entgegenhaltungen:
- WO-A1-2010/006704

## Beschreibung

Die vorliegende Erfindung betrifft Carbonsäurederivate mit Oxazolo[5,4-d]Pyrimidinring, sowie deren physiologisch akzeptable Salze.

Es sind bereits strukturähnliche Verbindungen im Stand der Technik beschrieben (siehe WO2009/154775), die zur Behandlung multipler Sklerose geeignet sind. Die Wirkungsweise dieser Verbindungen besteht darin, durch Aktivierung des EDG-1 Rezeptors eine Desensitisierung des EDG-1 Signalweges zu verursachen (sog. Superagonismus), der dann einem funktionellen Antagonismus des EDG-1-Signalweges gleichkommt. Systemisch bedeutet das, daß vor allem auf Lymphozyten der EDG-1 Signalweg dauerhaft unterdrückt wird, wodurch diese Zellen nicht mehr chemotaktisch dem S1 P Gradienten zwischen Blut und Lymphflüssigkeit folgen können. Dies bedingt, dass die betroffenen Lymphozyten nicht mehr das sekundäre lymphatische Gewebe verlassen können (verstärktes Homeing) und die Zahl der frei zirkulierenden Lymphozyten im Plasma stark gesenkt wird. Dieser Mangel an Lymphozyten im Plasma (Lymphopenie) bewirkt eine Immunsuppression, die zwingend für den Wirkmechanismus der in WO 2009/154775 beschriebenen EDG-1-Rezeptor-Modulatoren erforderlich ist.

Der Erfindung lag die Aufgabe zugrunde, Verbindungen zur Verfügung zu stellen, die eine therapeutisch verwertbare Wirkung entfalten. Insbesondere bestand die Aufgabe darin, neue Verbindungen zu finden, die spezifisch zur Wundheilung und insbesondere zur Behandlung von Wundheilungsstörungen von Diabetes Patienten geeignet sind. Weiter war es wünschenswert Verbindungen zur Verfügung zustellen, die zur Behandlung des Diabetische Fußsyndroms (DFS) geeignet sind.

Weiter war es wünschenswert eine wiederholbare Aktivierung des EDG-1-Rezeptor Signalweges zu erreichen, was damit pharmakologisch eine persistente Aktivierung des EDG-1 Signalweges ermöglicht.

Die Erfindung betrifft daher Verbindungen der Formel I 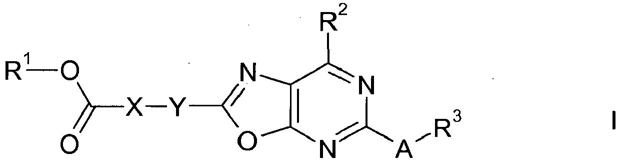 worin A, X, Y, R¹, R² und R³ wie nachstehend definiert sind.

Der Wirkmechanismus der Verbindungen der Formel I beruht nicht auf Desensitisierung des EDG-1-Signalweges und steht somit dem in WO 2009/154775 beschriebenen Wirkmechanismus diametral gegenüber. Die Erfindung betrifft ferner Verfahren zur Herstellung von Verbindungen der Formel I, ihre Verwendung, insbesondere als Wirkstoff in Pharmazeutika, und pharmazeutische Zusammensetzungen, die sie enthalten.

Patienten mit Diabetes haben gegenüber gesunden Menschen eine verzögerte Wundheilung und eine erhöhte Infektionsrate, vor allem bei länger währender Hyperglykämie, zum Beispiel hervorgerufen durch eine schlechte Blutzuckereinstellung. Zu den Ursachen gehören Durchblutungsstörungen, vor allem im Bereich der kleinen Gefäße, die zu einer verschlechterten Sauerstoff- und Nährstoffversorgung des Gewebes führen. Außerdem besteht eine verminderte Zellteilungs- und Zellmigrationsrate von Keratinozyten, Fibroblasten und dermalen Endothelzellen. Zusätzlich ist die Aktivität verschiedener Abwehrzellen (Granulozyten) mit reduzierter Phagozytose (Aufnahme und Zerstörung von Bakterien) eingeschränkt. Auch die Funktion der Antikörper (Immunglobuline) gegen Bakterien ist bei hohen Blutzuckerwerten eingeschränkt. Dementsprechend müssen Wunden und Infektionen bei Diabetes-Patienten besonders versorgt werden.

Der Edg-1-Rezeptor gehört zur Familie der Edg-Rezeptoren (Edg = Endothelial Differentiation Gene) von gegenwärtig acht identifizierten GPCRs (G-Proteingekoppelten Rezeptoren) der Klasse A. Diese Familie kann in Unterfamilien von durch Sphingosin-1-Phosphat (S1P) aktivierten Rezeptoren (fünf Mitglieder) und durch Lysophosphatidsäure (LPA) aktivierte Rezeptoren (drei Mitglieder) unterteilt werden. Der endogene Ligand S1 P ist ein pluripotentes Lysophospholipid, das durch Aktivierung von GPCRs aus der Edg-Rezeptorfamilie, nämlich Edg-1 (= S1P1), Edg-3 (= S1 P3), Edg-5 (= S1P2), Edg-6 (= S1P4) und Edg-8 (S1P5), auf verschiedene Zelltypen wirkt. Wenngleich S1 P auch als intrazellulärer Botenstoff beschrieben wird, werden zahlreiche zelluläre Antworten von S1 P über die Aktivierung von Edg-Rezeptoren - vermittelt werden. S1 P wird durch die Enzymfamilie der Sphingosinkinasen (SPHK) erzeugt und durch verschiedene Phosphatasen oder Lyasen abgebaut.

Bekannte Indikationen von Edg-1-Rezeptor-Agonisten sind beispielsweise Herz-Kreislauf-Erkrankungen, Atherosklerose, Herzversagen, Cardioprotektion, periphere arterielle Verschlußkrankheit, Nierenerkrankungen und Atemwegserkrankungen.

Gegenstand der vorliegenden Erfindung sind Verbindungen der Formel I in einer beliebigen ihrer stereoisomeren Formen oder einer Mischung von stereoisomeren Formen in beliebigem Verhältnis oder ein physiologisch akzeptables Salz davon oder ein physiologisch akzeptables Solvat einer derartigen Verbindung oder eines derartigen Salzes, 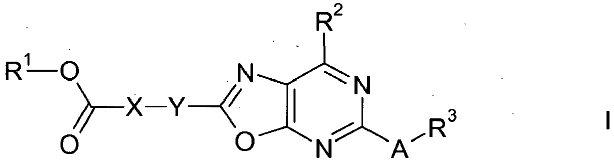 worin
A ausgewählt ist aus Bindung, -CH₂-, NH, O und S;
X ausgewählt ist aus (C₁-C₆)-Alkandiyl, (C₂-C₆)-Alkendiyl, (C₂-C₆)-Alkindiyl, (C₃-C₇)-Cycloalkandiyl, (C₁-C₆)-Alkandiyloxy und (C₃-C₇)-Cycloalkandiyloxy, die alle gegebenenfalls durch einen oder mehrere gleiche oder verschiedene Substituenten substituiert sind, die aus Fluor und Hydroxy ausgewählt sind, wobei das Sauerstoffatom der (C₁-C₆)-Alkandiyloxy- und (C₃-C₇)-Cycloalkandiyloxygruppen an die Gruppe Y gebunden ist;
Y ausgewählt ist aus Phenylen und einem zweiwertigen Rest eines aromatischen, 5-gliedrigen bis 6-gliedrigen monocyclischen Heterocyclus, der 1, 2 oder 3 gleiche oder verschiedene Ringheteroatome enthält, die aus N, O und S ausgewählt sind, wobei eines der Ringstickstoffatome ein Wasserstoffatom oder einen Substituenten R⁴ tragen kann und wobei das Phenylen und der zweiwertige Rest eines aromatischen Heterocyclus gegebenenfalls an einem oder mehreren Ringkohlenstoffatomen durch gleiche oder verschiedene Substituenten R⁵ substituiert sind;
R¹ ausgewählt ist aus Wasserstoff, (C₁-C₄)-Alkyl und (C₃-C₇)-Cycloalkyl-C_{z}H_{2z}-, worin z aus 0, 1 und 2 ausgewählt ist;
R² ausgewählt ist aus (C₁C₆)-Alkyl, (C₃-C₅)-Cycloalkyl-CₓH₂ₓ-, Het¹-CₙH₂ₙ-, worin x und n aus 0, 1 und 2 ausgewählt sind;
R³ ausgewählt ist aus (C₁-C₆)-Alkyl, wobei der Alkylrest gegebenenfalls durch ein oder mehrere Fluoratome substituiert ist, (C₂-C₆)-Alkenyl, (C₂-C₆)-Alkinyl, (C₃-C₇)-Cycloalkyl-CᵤH₂ᵤ- und Het²⁻CᵥH₂ᵥ-, worin u und v aus 1 und 2 ausgewählt sind, oder R³ für einen Rest eines gesättigten oder ungesättigten, 3-gliedrigen bis 10-gliedrigen, monocyclischen oder bicyclischen Rings, der 0, 1, 2, 3 oder 4 gleiche oder verschiedene Ringheteroatome enthält, die aus N, O und S ausgewählt sind, steht, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist;
R⁴ ausgewählt ist aus (C₁-C₄)-Alkyl, (C₃-C₇)-Cycloalkyl-C_{w}H_{2w}- und Oxy, wobei w aus 0, 1 und 2 ausgewählt ist;
R⁵ ausgewählt ist aus Halogen, Hydroxy, (C₁-C₄)-Alkyl-, (C₃-C₅)-Cycloalkyl-C_{z}H_{2z}-, (C₁-C₄)-Alkyloxy, (C₁-C₄)-Alkyl-S(O)ₘ-, Amino, Nitro, Cyano, Hydroxycarbonyl, (C₁-C₄)-Alkyloxycarbonyl, Aminocarbonyl und Aminosulfonyl, wobei z aus 0, 1 und 2 ausgewählt ist;
R³¹ ausgewählt ist aus Halogen, Hydroxy, (C₁-C₄)-Alkyl, (C₁-C₄)-Alkyloxy, (C₃-C₇)-Cycloalkyl, Oxo, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Nitro, Cyano, (C₁-C₄)-Alkylcarbonyl, Aminosulfonyl, (C₁-C₄)-Alkylaminosulfonyl und Di((C₁-C₄)-alkyl)aminosulfonyl;
Het¹ für einen Rest eines gesättigten, 4-gliedrigen bis 6-gliedrigen, monocyclischen gesättigten Heterocyclus steht, der 1 oder 2 gleiche oder verschiedene Ringheteroatome enthält, die unter O und S ausgewählt sind, und der über ein Ringkohlenstoffatom gebunden ist, wobei ein Ringschwefelatom eine oder zwei Oxogruppen tragen können und wobei der Rest eines Heterocyclus gegebenenfalls durch einen oder mehrere gleiche oder verschiedene Substituenten substituiert ist, die unter Fluor und (C₁-C₄)-Alkyl ausgewählt sind;
Het² für einen Rest eines gesättigten, 4-gliedrigen bis 7-gliedrigen, monocyclischen Heterocyclus steht, der 1 oder 2 gleiche oder verschiedene Ringheteroatome enthält, die unter N, O und S ausgewählt sind, und der über ein Ringkohlenstoffatom gebunden ist, wobei der Rest eines Heterocyclus gegebenenfalls durch einen oder mehrere gleiche oder verschiedene Substituenten substituiert ist, die unter Fluor und (C₁-C₄)-Alkyl ausgewählt sind;
m aus 0,1 und 2 ausgewählt ist.

Strukturelemente wie Gruppen, Substituenten, Heteroringglieder, Zahlen oder andere Merkmale, beispielsweise Alkylgruppen, Gruppen wie R⁵, Zahlen wie m, die in den Verbindungen der Formel I mehrmals vorkommen können, können alle unabhängig voneinander eine beliebige der angegebenen Bedeutungen besitzen und in jedem Fall gleich oder voneinander verschieden sein. Beispielsweise können die Alkylgruppen in einer Dialkylaminogruppe gleich oder verschieden sein.

Alkyl-, Alkenyl- und Alkinylgruppen können linear, d.h. geradkettig, oder verzweigt sein. Dies gilt auch, wenn sie Teil anderer Gruppen, beispielsweise Alkyloxygruppen (= Alkoxygruppen, Alkyl-O-Gruppen), Alkyloxycarbonylgruppen oder alkylsubstituierter Aminogruppen, sind oder wenn sie substituiert sind. Je nach der jeweiligen Definition kann die Zahl der Kohlenstoffatome in einer Alkylgruppe 1, 2, 3, 4, 5 oder 6 oder 1, 2, 3 oder 4 oder 1, 2 oder 3 betragen. Beispiele für Alkyl sind Methyl, Ethyl, Propyl einschließlich n-Propyl und Isopropyl, Butyl, einschließlich n-Butyl, sek.-Butyl, Isobutyl und tert.-Butyl, Pentyl einschließlich n-Pentyl, 1-Methylbutyl, Isopentyl, Neopentyl und tert.-Pentyl und Hexyl einschließlich n-Hexyl, 3,3-Dimethylbutyl und Isohexyl. Doppelbindungen und Dreifachbindungen in Alkenylgruppen und Alkinylgruppen können in beliebigen Positionen vorliegen. In einer Ausführungsform der Erfindung enthalten Alkenylgruppen eine Doppelbindung und Alkinylgruppen eine Dreifachbindung. In einer Ausführungsform der Erfindung enthält eine Alkenylgruppe oder Alkinylgruppe mindestens drei Kohlenstoffatome und ist über ein Kohlenstoffatom, das nicht Teil einer Doppelbindung oder Dreifachbindung ist, an den Rest des Moleküls gebunden. Beispiele für Alkenyl und Alkinyl sind Ethenyl, Prop-1-enyl, Prop-2-enyl (= Allyl), But-2-enyl, 2-Methylprop-2-enyl, 3-Methylbut-2-enyl, Hex-3-enyl, Hex-4-enyl, Prop-2-inyl (= Propargyl), But-2-inyl, But-3-inyl, Hex-4-inyl oder Hex-5-inyl. Substituierte Alkylgruppen, Alkenylgruppen und Alkinylgruppen können in beliebigen Positionen substituiert sein, mit der Maßgabe, daß die jeweilige Verbindung ausreichend stabil und für den gewünschten Zweck wie die Verwendung als Arzneistoff geeignet ist. Die Voraussetzung, daß eine spezifische Gruppe und eine Verbindung der Formel I ausreichend stabil und für den gewünschten Zweck wie die Verwendung als Arzneistoff geeignet sind, gilt allgemein in bezug auf die Definitionen aller Gruppen in den Verbindungen der Formel I.

Soweit anwendbar, gelten die vorstehenden Erklärungen bezüglich Alkyl-, Alkenyl- und Alkinylgruppen entsprechend für zweiwertige Alkylgruppen wie die Gruppen Alkandiyl CᵤH₂ᵤ, CᵥH₂ᵥ, C_{w}H_{2w}, CₓH₂ₓ, C_{y}H_{2y} und C_{z}H_{2z} und zweiwertige Alkenylgruppen und Alkinylgruppen, wie die Gruppen Alkendiyl und Alkindiyl, die somit ebenfalls linear und verzweigt sein können. Die Doppelbindungen und Dreifachbindungen in Alkendiyl- und Alkindiylgruppen können in beliebigen Positionen vorliegen. In einer Ausführungsform der Erfindung enthalten Alkendiylgruppen eine Doppelbindung und Alkindiylgruppen eine Dreifachbindung. Beispiele für zweiwertige Alkylgruppen sind -CH₂-(= Methylen), -CH₂-CH₂-, -CH₂-CH₂-CH₂-, -CH₂-CH₂-CH₂-CH₂-, -CH(CH₃)-, -C(CH₃)₂-, -CH(CH₃)-CH₂-, -CH₂-CH(CH₃)-, -C(CH₃)₂-CH₂-, -CH₂-C(CH₃)₂-, Beispiele für zweiwertige Alkenylgruppen sind -CH=CH-, -CH₂-CH=CH-, -CH=CH-CH₂-, -CH₂-CH=CH-CH₂-, -CH₂-CH₂-CH=CH-, -C(CH₃)=C(CH₃)-, und Beispiele für zweiwertige Alkinylgruppen sind -C=C-, -CH₂-C=C-, -C=C-CH₂-, -C(CH₃)₂-C≡C-, -C≡C-C(CH3)₂-, -CH₂-C≡C-CH₂-, -CH₂-CH₂-C≡C-. Wenn eine Zahl in einer zweiwertigen Gruppe, wie beispielsweise die Zahl z in der Gruppe C_{z}H_{2z}, für 0 (= null) steht, sind die beiden Gruppen, die an die in Rede stehende Gruppe, wie C_{z}H_{2z}, gebunden sind, über eine Einfachbindung direkt miteinander verbunden.

Die Zahl der Ringkohlenstoffatome in einer Cycloalkylgruppe kann 3, 4, 5, 6 oder 7 betragen. In einer Ausführungsform der Erfindung beträgt die Zahl der Ringkohlenstoffatome in einer Cycloalkylgruppe unabhängig von der Zahl der Ringkohlenstoffatome in einer anderen Cycloalkylgruppe 3, 4, 5 oder 6, in einer anderen Ausführungsform 3, 4 oder 5, in einer anderen Ausführungsform 3 oder 4, in einer anderen Ausführungsform 3, in einer anderen Ausführungsform 5, 6 oder 7, in einer anderen Ausführungsform 5 oder 6, in einer anderen Ausführungsform 6 oder 7, in einer anderen Ausführungsform 6. Dies gilt entsprechend für zweiwertige Cycloalkylgruppen, d.h. Cycloalkandiylgruppen, die über ein oder zwei beliebige Ringkohlenstoffatome an die benachbarten Gruppen gebunden sein können. Beispiele für Cycloalkylgruppen sind Cyclopropyl, Cyclobutyl, Cyclopentyl, Cyclohexyl und Cycloheptyl. Beispiele für zweiwertige Cycloalkylgruppen sind Cyclopropan-1,1-diyl, Cyclopropan-1,2-diyl, Cyclobutan-1,3-diyl, Cyclopentan-1,1-diyl, Cyclopentan-1,2-diyl, Cyclopentan-1,3-diyl, Cyclohexan-1,1-diyl, Cyclohexan-1,2-diyl, Cyclohexan-1,3-diyl, Cyclohexan-1,4-diyl, Cycloheptan-1,4-diyl. Unabhängig voneinander und unabhängig von anderen Substituenten sind Cycloalkylgruppen und Cycloalkandiylgruppen gegebenenfalls durch einen oder mehrere gleiche oder verschiedene (C₁-C₄)-Alkylsubstituenten substituiert, die in beliebigen Positionen stehen können, d.h. Cycloalkylgruppen können durch Alkylsubstituenten unsubstituiert oder durch Alkylsubstituenten, beispielsweise 1, 2, 3 oder 4 oder 1 oder 2 (C₁-C₄)-Alkylsubstituenten, beispielsweise Methylgruppen, substituiert sein. Beispiele für alkylsubstituierte Cycloalkylgruppen und Cycloalkandiylgruppen sind 4-Methylcyclohexyl, 4-tert.-Butylcyclohexyl oder 2,3-Dimethylcyclopentyl, 2,2-Dimethylcyclopropan-1,1-diyl, 2,2-Dimethylcyclopropan-1,2-diyl, 2,2-Dimethylcyclopentan-1,3-diyl, 6,6-Dimethylcycloheptan-1,4-diyl. Beispiele für Cycloalkylalkylgruppen, die beispielsweise Gruppen wie (C₃-C₇)-Cycloalkyl-C_{z}H_{2z}-repräsentieren können, sind Cyclopropylmethyl, Cyclobutylmethyl, Cyclopentylmethyl, Cyclohexylmethyl, Cycloheptylmethyl, 1-Cyclopropylethyl, 2-Cyclopropylethyl, 1-Cyclobutylethyl, 2-Cyclobutylethyl, 2-Cyclopentylethyl, 2-Cyclohexylethyl, 2-Cycloheptylethyl.

Unabhängig voneinander und unabhängig von anderen Substituenten können Alkylgruppen, zweiwertige Alkylgruppen, Alkenylgruppen, zweiwertige Alkenylgruppen, Alkinylgruppen, zweiwertige Alkinylgruppen, Cycloalkylgruppen und zweiwertige Cycloalkylgruppen gegebenenfalls durch einen oder mehrere Fluorsubstituenten substituiert sein, die in beliebigen Positionen stehen können, d.h. diese Gruppen können durch Fluorsubstituenten unsubstituiert oder durch Fluorsubstituenten, beispielsweise 1, 2, 3, 4, 5, 6, 7, 8, 9, 10, 11, 12 oder 13 oder 1, 2, 3, 4, 5, 6, 7, 8 oder 9 oder 1, 2, 3, 4, 5, 6 oder 7 oder 1, 2, 3, 4 oder 5 oder 1, 2 oder 3 oder 1 oder 2 Fluorsubstituenten, substituiert sein. Beispiele für fluorsubstituierte derartige Gruppen sind Trifluormethyl, 2-Fluorethyl, 2,2,2-Trifluorethyl, Pentafluorethyl, 3,3,3-Trifluorpropyl, 2,2,3,3,3-Pentafluorpropyl, 4,4,4-Trifluorbutyl, Heptafluorisopropyl, -CHF-, -CF₂-, -CF₂-CH₂-, -CH₂-CF₂-, -CF₂-CF₂-, -CF(CH₃)-, -C(CF₃)₂-, 1-Fluorcyclopropyl, 2,2-Difluorcyclopropyl, 3,3-Difluorcyclobutyl, 1-Fluorcyclohexyl, 4,4-Difluorcyclohexyl, 3,3,4,4,5,5-Hexafluorcyclohexyl, 2,2-Difluorcyclopropan-1,2-diyl. Beispiele für Alkyloxygruppen, in denen die Alkylgruppierung fluorsubstituiert ist, sind Trifluormethoxy, 2,2,2-Trifluorethoxy, Pentafluorethoxy und 3,3,3-Trifluorpropoxy. In einer Ausführungsform der Erfindung beträgt die Gesamtzahl der Fluorsubstituenten und (C₁-C₄)-Alkylsubstituenten, die unabhängig von anderen Substituenten gegebenenfalls an Cycloalkylgruppen und Cycloalkandiylgruppen in den Verbindungen der Formel I vorliegen, 1, 2, 3, 4, 5, 6, 7, 8, 9, 10 oder 11, in einer anderen Ausführungsform 1, 2, 3, 4, 5, 6, 7, 8 oder 9, in einer anderen Ausführungsform 1, 2, 3, 4 oder 5, in einer anderen Ausführungsform 1, 2, 3 oder 4.

Gruppen wie Phenyl, Naphthyl (= Naphthalinyl) und Reste von aromatischen Heterocyclen, die gegebenenfalls durch einen oder mehrere Substituenten substituiert sind, können unsubstituiert oder substituiert sein, beispielsweise durch 1, 2, 3, 4 oder 5 oder 1, 2, 3 oder 4 oder 1, 2 oder 3 oder 1 oder 2 oder 1 gleiche oder verschiedene Substituenten, die in beliebigen Positionen stehen können. In einer Ausführungsform der Erfindung ist die Gesamtzahl der Nitrosubstituenten in einer Verbindung der Formel I nicht größer als zwei. Aromatische Stickstoffheterocyclen, die in dem zugrundeliegenden Ringsystem ein Wasserstoffatom an einem Ringstickstoffatom in einem 5-gliedrigen Ring, wie beispielsweise einem Pyrrol-, Imidazol-, Indol- oder Benzoimidazolring, tragen, können an den Kohlenstoffatomen und/oder an derartigen Ringstickstoffatomen substituiert sein. In einer Ausführungsform der Erfindung sind Substituenten an derartigen Ringstickstoffatomen aus (C₁-C₄)-Alkylgruppen ausgewählt, d.h. derartige Ringstickstoffatome in aromatischen Heterocyclen tragen ein Wasserstoffatom oder einen (C₁-C₄)-Alkylsubstituenten. Wenn bezüglich Ringstickstoffatomen in aromatischen Heterocyclen und anderen Heterocyclen angegeben ist, daß sie ein Wasserstoffatom oder einen Substituenten tragen können, so tragen derartige Ringstickstoffatome entweder ein Wasserstoffatom oder einen Substituenten oder nicht. Ringstickstoffatome, die ein Wasserstoffatom oder einen Substituenten tragen, kommen in einem stickstoffhaltigen aromatischen 5-gliedrigen Ring, wie er beispielsweise in Pyrrol, Imidazol, Indol oder Benzoimidazol vorliegt, und in einem nichtaromatischen Ring einschließlich eines gesättigten Rings vor. Ringstickstoffatome, die kein Wasserstoffatom oder keinen Substituenten tragen, sofern sie nicht in positiv geladener Form vorliegen, einschließlich weiterer Ringstickstoffatome neben den Ringstickstoffatomen, die ein Wasserstoffatom oder einen Substituenten tragen, kommen in einem aromatischen Ring, wie er beispielsweise in Thiazol, Imidazol, Pyridin oder Benzoimidazol vorliegt, und in einem nichtaromatischen Ring, in dem sie Brückenkopfatome oder Teil einer Doppelbindung sind, und als Ringstickstoffatome, über die ein Ring gebunden ist, vor. Geeignete Ringstickstoffatome in aromatischen Heterocyclen in den Verbindungen der Formel I, wie das Ringstickstoffatom in einem Pyridinring, spezifisch ein Ringstickstoffatom in einem aromatischen Heterocyclus, der R² repräsentiert, können auch einen Oxysubstituenten -O- tragen und als N-Oxid vorliegen, und derartige Ringstickstoffatome können auch als quartäres Salz, beispielsweise als N-(C₁-C₄)-Alkylsalz wie N-Methylsalz, vorliegen, wobei in einer Ausführungsform der Erfindung das Gegenanion in einem derartigen quartären Salz ein physiologisch akzeptables Anion ist, das sich von einer Säure, die ein physiologisch akzeptables Salz bildet, ableitet. In monosubstituierten Phenylgruppen kann der Substituent in der 2-Position, der 3-Position oder der 4-Position stehen. In disubstituierten Phenylgruppen können die Substituenten in 2,3-Position, 2,4-Position, 2,5-Position, 2,6-Position, 3,4-Position oder 3,5-Position stehen. In trisubstituierten Phenylgruppen können die Substituenten in 2,3,4-Position, 2,3,5-Position, 2,3,6-Position, 2,4,5-Position, 2,4,6-Position oder 3,4,5-Position stehen. Naphthyl kann 1-Naphthyl (= Naphthalin-1-yl) oder 2-naphthyl (= Naphthalin-2-yl) sein. In monosubstituierten 1-Naphthylgruppen kann der Substituent in der 2-, 3-, 4-, 5-, 6-, 7-oder 8-Position stehen. In monosubstituierten 2-Naphthylgruppen kann der Substituent in der 1-, 3-, 4-, 5-, 6-, 7- oder 8-Position stehen. In disubstituierten Naphthylgruppen können die Substituenten ebenfalls in beliebigen Positionen sowohl in dem Ring, über den die Naphthylgruppe gebunden ist, und/oder in dem anderen Ring stehen. Diese Aussage bezüglich der einwertigen Reste gilt entsprechend für die jeweiligen zweiwertigen Reste, wie beispielsweise Phenylengruppen, die R² repräsentieren, die somit ebenfalls unsubstituiert oder substituiert sein können, beispielsweise durch 1, 2, 3 oder 4 oder durch 1, 2 oder 3 oder durch 1 oder 2 oder durch 1 gleiche oder verschiedene Substituenten, die in beliebigen Positionen stehen können.

In aromatischen Heterocyclen, die als Heteroaryl- und Heteroarylengruppen bezeichnet sein können, sowie in allen anderen heterocyclischen Ringen und den nichtaromatischen heterocyclischen Gruppen, sind die Ringheteroatome im allgemeinen aus N, O und S ausgewählt, wobei N Ringstickstoffatome, die ein Wasserstoffatom oder einen Substituenten tragen, sowie Ringstickstoffatome, die kein Wasserstoffatom und keinen Substituenten tragen, einschließt. Ringheteroatome können in beliebigen Positionen stehen, vorausgesetzt, daß das heterocyclische System in der Technik bekannt und stabil und als Untergruppe für den gewünschten Zweck der Verbindung der Formel I wie die Verwendung als Arzneistoff geeignet ist. In einer Ausführungsform der Erfindung können zwei Ringsauerstoffatome nicht in benachbarten Ringpositionen eines Heterocyclus stehen, in einer anderen Ausführungsform können zwei Ringheteroatome, die aus Sauerstoff und Schwefel ausgewählt sind, nicht in benachbarten Ringpositionen eines beliebigen Heterocyclus stehen. Gesättigte Ringe enthalten keine Doppelbindung im Ring. Ungesättigte Ringsysteme können aromatisch oder teilweise ungesättigt einschließlich teilweise aromatisch sein, wobei in letzterem Fall ein Ring in einem bicyclischen Ringsystem aromatisch ist und das Ringsystem über ein Atom im nichtaromatischen Ring gebunden ist. Je nach der jeweiligen Gruppe können ungesättigte Ringe eine, zwei, drei, vier oder fünf Doppelbindungen im Ring enthalten. Aromatische Gruppen enthalten ein cyclisches System von sechs oder zehn delokalisierten pi-Elektronen im Ring. Je nach der jeweiligen Gruppe können gesättigte und nichtaromatisch ungesättigte heterocyclische Ringe einschließlich Het und nichtaromatischen Gruppen, die R³ repräsentieren, 3-gliedrig, 4-gliedrig, 5-gliedrig, 6-gliedrig, 7-gliedrig, 8-gliedrig, 9-gliedrig oder 10-gliedrig sein. In einer Ausführungsform der Erfindung sind aromatische heterocyclische Ringe 5-gliedrige oder 6-gliedrige monocyclische Ringe oder 8-gliedrige, 9-gliedrige oder 10-gliedrige bicyclische Ringe, in einer anderen Ausführungsform 5-gliedrige oder 6-gliedrige monocyclische Ringe oder 9-gliedrige oder 10-gliedrige bicyclische Ringe, in einer anderen Ausführungsform 5-gliedrige oder 6-gliedrige monocyclische Ringe, wobei die 8-gliedrigen, 9-gliedrigen oder 10-gliedrigen bicyclischen Ringe aus zwei anellierten 5-gliedrigen Ringen, einem 5-gliedrigen Ring und einem 6-gliedrigen Ring, die miteinander anelliert sind, bzw. zwei anellierten 6-gliedrigen Ringen zusammengesetzt sind. In bicyclischen aromatischen heterocyclischen Gruppen können ein oder beide Ringe Heteroringglieder enthalten, und ein oder beide Ringe können aromatisch sein. Im allgemeinen werden bicyclische Ringsysteme mit einem aromatischen Ring und einem nichtaromatischen Ring als aromatisch erachtet, wenn sie über ein Kohlenstoffatom im aromatischen Ring gebunden sind, und als nichtaromatisch, wenn sie über ein Kohlenstoffatom im nichtaromatischen Ring gebunden sind. Sofern nicht anders angegeben, können heterocyclische Gruppen einschließlich aromatischer heterocyclischer Gruppen über ein beliebiges geeignetes Ringkohlenstoffatom und im Fall von Stickstoffheterocyclen über ein beliebiges geeignetes Ringstickstoffatom gebunden sein. In einer Ausführungsform der Erfindung ist eine aromatische heterocyclische Gruppe in einer Verbindung der Formel I unabhängig von jeder anderen aromatischen heterocyclischen Gruppe über ein Ringkohlenstoffatom gebunden, in einer anderen Ausführungsformen über ein Ringstickstoffatom. Je nach der Definition der jeweiligen heterocyclischen Gruppe beträgt in einer Ausführungsform der Erfindung die Zahl der Ringheteroatome, die in einer heterocyclischen Gruppe unabhängig von der Zahl von Ringheteroatomen in einer anderen heterocyclischen Gruppe vorliegen kann, 1, 2, 3 oder 4, in einer anderen Ausführungsform 1,2 oder 3, in einer anderen Ausführungsform 1 oder 2, in einer anderen Ausführungsform 1, wobei die Ringheteroatome gleich oder verschieden sein können. Heterocyclische Gruppen, die gegebenenfalls substituiert sind, können unabhängig von jeder anderen heterocyclischen Gruppe unsubstituiert oder durch einen oder mehrere gleiche oder verschiedene Substituenten, beispielsweise 1, 2, 3, 4 oder 5 oder 1, 2, 3 oder 4 oder 1, 2 oder 3 oder 1 oder 2 oder 1 Substituenten, die bei der Definition der jeweiligen Gruppe angegeben sind, substituiert sein. Substituenten an heterocyclischen Gruppen können in beliebigen Positionen stehen. So können Substituenten in einer Pyridin-2-ylgruppe beispielsweise in der 3-Position und/oder 4-Position und/oder 5-Position und/oder 6-Position stehen, in einer Pyridin-3-ylgruppe in der 2-Position und/oder 4-Position und/oder 5-Postion und/oder 6-Position stehen und in einer Pyridin-4-ylgruppe in der 2-Position und/oder 3-Position und/oder 5-Position und/oder 6-Position stehen.

Beispiele für Grundkörper von Heterocyclen, von denen sich heterocyclische Gruppen einschließlich aromatischer heterocyclischer Gruppen, gesättigter heterocyclischer Gruppen und nichtaromatischer ungesättigter heterocyclischer Gruppen ableiten können, sind Azet, Oxet, Pyrrol, Furan, Thiophen, Imidazol, Pyrazol, [1,3]Dioxol, Oxazol (= [1,3]Oxazol), Isoxazol (= [1,2]Oxazol), Thiazol (= [1,3]Thiazol), Isothiazol (= [1,2]Thiazol), [1,2,3]Triazol, [1,2,4]Triazol, [1,2,4]Oxadiazol, [1,3,4]Oxadiazol, [1,2,4]Thiadiazol, [1,3,4]Thiadiazol, Tetrazol, Pyridin, Pyran, Thiopyran, Pyridazin, Pyrimidin, Pyrazin, [1,3]Oxazin, [1,4]Oxazin, [1,3]Thiazin, [1,4]Thiazin, [1,2,3]Triazin, [1,3]Dithiin, [1,4]Dithiin, [1,2,4]Triazin, [1,3,5]Triazin, [1,2,4,5]Tetrazin, Azepin, [1,3]Diazepin, [1,4]Diazepin, [1,3]Oxazepin, [1,4]Oxazepin, [1,3]Thiazepin, [1,4]Thiazepin, Azocin, Azecin, Cyclopenta[b]pyrrol, 2-Azabicyclo[3.1.0]hexan, 3-Azabicyclo[3.1.0]hexan, 2-Oxa-5-azabicyclo[2.2.1]heptan, Indol, Isoindol, Benzothiophen, Benzofuran, [1,3]Benzodioxol (= 1,2-Methylendioxybenzol), [1,3]Benzoxazol, [1,3]Benzothiazol, Benzoimidazol, Thieno[3,2-c]pyridin, Chromen, Isochromen, [1,4]Benzodioxin, [1,4]Benzoxazin, [1,4]Benzothiazin, Chinolin, Isochinolin, Cinnolin, Chinazolin, Chinoxalin, Phthalazin, Thienothiophen, [1,8]Naphthyridin und andere Naphtyridine, Pteridin und die jeweiligen gesättigten und teilweise ungesättigten Heterocyclen, in denen eine oder mehrere, beispielsweise eine, zwei, drei, vier oder alle Doppelbindungen im Ringsystem einschließlich der Doppelbindungen im aromatischen Ring durch Einfachbindungen ersetzt sind, wie beispielsweise Azetidin, Oxetan, Pyrrolidin, Tetrahydrofuran, Tetrahydrothiophen, Imidazolidin, Oxazolidin, Thiazolidin, Dihydropyridin, Piperidin, Tetrahydropyran, Piperazin, Morpholin, Thiomorpholin, Azepan, Chroman, Isochroman, [1,4]Benzodioxan (= 1,2-Ethylendioxybenzol), 2,3-Dihydrobenzofuran, 1,2,3,4-Tetrahydrochinolin, 1,2,3,4-Tetrahydroisochinolin.

Beispiele für Reste von aromatischen Heterocyclen, die in den Verbindungen der Formel I vorkommen können, sind Thiophenyl (= Thienyl) einschließlich Thiophen-2-yl und Thiophen-3-yl, Pyridinyl (= Pyridyl) einschließlich Pyridin-2-yl (= 2-Pyridyl), Pyridin-3-yl (= 3-Pyridyl) und Pyridin-4-yl (= 4-Pyridyl), Imidazolyl einschließlich beispielsweise 1H-Imidazol-1-yl, 1H-Imidazol-2-yl, 1H-Imidazol-4-yl und 1H-Imidazol-5-yl, [1,2,4]Triazolyl einschließlich 1 H-[1,2,4]-Triazol-1-yl und 4H-[1,2,4]-Triazol-3-yl, Tetrazolyl einschließlich 1 H-Tetrazol-1-yl und 1H-Tetrazol-5-yl, Chinolinyl (= Chinolyl) einschließlich Chinolin-2-yl, Chinolin-3-yl, Chinolin-4-yl, Chinolin-5-yl, Chinolin-6-yl, Chinolin-7-yl und Chinolin-8-yl, die alle gegebenenfalls wie in der Definition der jeweiligen Gruppe angegeben substituiert sind. Beispiele für Reste von gesättigten und teilweise ungesättigten Heterocyclen, die in den Verbindungen der Formel I vorkommen können, sind Azetidinyl, Pyrrolidinyl einschließlich Pyrrolidin-1-yl, Pyrrolidin-2-yl und Pyrrolidin-3-yl, 2,5-Dihydro-1H-pyrrolyl, Piperidinyl einschließlich Piperidin-1-yl, Piperidin-2-yl, Piperidin-3-yl und Piperidin-4-yl, 1,2,3,4-Tetrahydropyridinyl, 1,2,5,6-Tetrahydropyridinyl, 1,2-Dihydropyridinyl, Azepanyl, Azocanyl, Azecanyl, Octahydrocyclopenta[b]pyrrolyl, 2,3-Dihydrobenzofuranyl einschließlich 2,3-Dihydrobenzofuran-7-yl, 2,3-Dihydro-1H-indolyl, Octahydro-1 H-indolyl, 2,3-Dihydro-1H-isoindolyl, Octahydro-1H-isoindolyl, 1,2-Dihydrochinolinyl, 1,2,3,4-Tetrahydrochinolinyl, Decahydrochinolinyl, 1,2-Dihydroisochinolinyl, 1,2,3,4-Tetrahydroisochinolinyl, 1,2,3,4-Tetrahydroisochinolinyl, Decahydroisochinolinyl, Decahydroisochinolinyl, 4,5,6,7-Tetrahydrothieno[3,2-c]pyridinyl, Pyrazolidinyl, Imidazolidinyl, Hexahydropyrimidinyl, 1,2-Dihydropyrimidinyl, Piperazinyl, [1,3]Diazepanyl, [1,4]Diazepanyl, Oxazolidinyl, [1,3]Oxazinanyl, [1,3]Oxazepanyl, Morpholinyl einschließlich Morpholin-2-yl, Morpholin-3-yl und Morpholin-4-yl, [1,4]Oxazepanyl, Thiazolidinyl, [1,3]Thiazinanyl, Thiomorpholinyl einschließlich Thiomorpholin-2-yl, Thiomorpholin-3-yl und Thiomorpholin-4-yl, 3,4-Dihydro-2H-[1,4]thiazinyl, [1,3]Thiazepanyl, [1,4]Thiazepanyl, [1,4]Thiazepanyl, Oxetanyl, Tetrahydrofuranyl, Tetrahydrothienyl, Isoxazolidinyl, Isothiazolidinyl, Oxazolidinyl, [1,2,4]-Oxadiazolidinyl, [1,2,4]-Thiadiazolidinyl, [1,2,4]Triazolidinyl, [1,3,4]Oxadiazolidinyl, [1,3,4]Thiadiazolidinyl, [1,3,4]Triazolidinyl, 2,3-Dihydrofuranyl, 2,5-Dihydrofuranyl, 2,3-Dihydrothienyl, 2,5-Dihydrothienyl, 2,3-Dihydropyrrolyl, 2,3-Dihydroisoxazolyl, 4,5-Dihydroisoxazolyl, 2,5-Dihydroisoxazolyl, 2,3-Dihydroisothiazolyl, 4,5-Dihydroisothiazolyl, 2,5-Dihydroisothiazolyl, 2,3-Dihydropyrazolyl, 4,5-Dihydropyrazolyl, 2,5-Dihydropyrazolyl, 2,3-Dihydrooxazolyl, 4,5-Dihydrooxazolyl, 2,5-Dihydrooxazolyl, 2,3-Dihydrothiazolyl, 4,5-Dihydrothiazolyl, 2,5-Dihydrothiazolyl, 2,3-Dihydroimidazolyl, 4,5-Dihydroimidazolyl, 2,5-Dihydroimidazolyl, Tetrahydropyridazinyl, Tetrahydropyrimidinyl, Tetrahydropyrazinyl, Tetrahydro[1,3,5]triazinyl, [1,3]Dithianyl, Tetrahydropyranyl, Tetrahydrothiopyranyl, [1,3]Dioxolanyl, 3,4,5,6-Tetrahydropyridinyl, 4H-[1,3]Thiazinyl, 1,1-Dioxo-2,3,4,5-tetrahydrothienyl, 2-Azabicyclo[3.1.0]hexyl einschließlich 2-Azabicyclo[3.1.0]hex-2-yl, 3-Azabicyclo[3.1.0]hexyl einschließlich 3-Azabicyclo[3.1.0]hex-3-yl, 2-Oxa-5-azabicyclo[2.2.1]heptyl einschließlich 2-Oxa-5-azabicyclo[2.2.1]hept-5-yl, die alle über ein geeignetes Ringkohlenstoffatom oder Ringstickstoffatom gebunden sind und gegebenenfalls wie in der Definition der jeweiligen Gruppe angegebenen substituiert sind.

Halogen steht für Fluor, Chlor, Brom oder lod. In einer Ausführungsform der Erfindung ist jedes Halogen in einer Verbindung der Formel I unabhängig von jedem anderen Halogen aus Fluor, Chlor und Brom ausgewählt, in einer anderen Ausführungsform aus Fluor und Chlor.

Wenn eine Oxogruppe an ein Kohlenstoffatom gebunden ist, ersetzt sie zwei Wasserstoffatome an einem Kohlenstoffatom des zugrundeliegenden Systems. Somit wird eine CH₂-Gruppe in einer Kette oder einem Ring dann, wenn sie durch Oxo, d.h. durch ein doppelt gebundenes Sauerstoffatom, substituiert ist, zu einer C(O)-Gruppe (= C(=O)-Gruppe). Offensichtlich kann eine Oxogruppe nicht als Substituent an einem Kohlenstoffatom in einem aromatischen Ring wie beispielsweise in einer Phenylgruppe vorkommen. Wenn ein Ringschwefelatom in einer heterocyclischen Gruppe eine oder zwei Oxogruppen tragen kann, handelt es sich in dem Fall, daß es keine Oxogruppe trägt, um ein nichtoxidiertes Schwefelatom S, oder in dem Fall, daß es eine Oxogruppe trägt, um eine S(O)-Gruppe (Sulfoxidgruppe, S-Oxid-Gruppe) oder in dem Fall, daß es zwei Oxogruppen trägt, um eine S(O)₂-Gruppe (= Sulfongruppe, S,S-Dioxid-Gruppe).

Die vorliegende Erfindung schließt alle stereoisomeren Formen der Verbindungen der Formel I und ihre Salze und Solvate ein. Bezüglich jedes chiralen Zentrums können die Verbindungen der Formel I unabhängig von jedem anderen chiralen Zentrum in S-Konfiguration oder weitgehend S-Konfiguration oder in R-Konfiguration oder weitgehend R-Konfiguration oder als Mischung des S-Isomers und des R-Isomers in beliebigen Verhältnissen vorliegen. Die Erfindung schließt alle möglichen Enantiomere und Diastereomere und Mischungen von zwei oder mehr Stereoisomeren, zum Beispiel Gemische von Enantiomeren und/oder Diastereomeren, in allen Verhältnissen ein. Somit können erfindungsgemäße Verbindungen, die als Enantiomere existieren können, in enantiomerenreiner Form sowohl als linksdrehende als auch rechtsdrehende Antipoden und in Form von Mischungen der beiden Enantiomere in allen Verhältnissen einschließlich Racematen vorliegen. Im Fall einer E/Z-Isomerie bzw. cis/trans-Isomerie, beispielsweise an Doppelbindungen oder Ringen wie Cycloalkylringen, schließt die Erfindung sowohl die E-Form als auch die Z-Form bzw. die cis-Form und die trans-Form sowie Mischungen dieser Formen in allen Verhältnissen ein. In einer Ausführungsform der Erfindung handelt es sich bei einer Verbindung, die in zwei oder mehr stereoisomeren Formen vorkommen kann, um ein reines oder weitgehend reines einzelnes Stereoisomer. Die Herstellung von einzelnen Stereoisomeren kann beispielsweise durch Trennung einer Mischung von Isomeren nach üblichen Methoden, beispielsweise durch Chromatographie oder Kristallisation, durch Verwendung von stereochemisch einheitlichen Ausgangsstoffen bei der Synthese oder durch stereoselektive Synthese erfolgen. Gegebenenfalls kann vor einer Trennung von Stereoisomeren eine Derivatisierung durchgeführt werden. Die Trennung einer Mischung von Stereoisomeren kann auf der Stufe der Verbindung der Formel I oder auf der Stufe eines Ausgangsstoffs oder eines Zwischenprodukts im Verlauf der Synthese durchgeführt werden. Die vorliegende Erfindung schließt auch alle tautomeren Formen der Verbindungen der Formel I und ihre Salze und Solvate ein.

Wenn die Verbindungen der Formel I eine oder mehrere saure und/oder basische Gruppen, d.h. salzbildende Gruppen, enthalten, schließt die Erfindung auch ihre entsprechenden physiologisch oder toxikologisch akzeptablen Salze, d.h. nichttoxischen Salze, insbesondere ihre pharmazeutisch akzeptablen Salze, ein.

Die vorliegende Erfindung schließt alle Solvate von Verbindungen der Formel I, beispielsweise Hydrate oder Addukte mit Alkoholen wie (C₁-C₄)-Alkanolen, aktive Metaboliten der Verbindungen der Formel I und auch Prodrugs und Derivate der Verbindungen der Formel I, die in vitro nicht unbedingt pharmakologische Wirkung zeigen, aber in vivo in pharmakologisch wirksame Verbindungen umgewandelt werden, beispielsweise Ester oder Amide von Carbonsäuregruppen, ein.

Die Alkandiyl-, Alkendiyl- und Alkindiylgruppen, die in der Gruppe X vorkommen, können linear oder verzweigt sein, wie bereits bezüglich derartiger Gruppen im allgemeinen angegeben, und diese Gruppen sowie Cycloalkandiylgruppen, die X repräsentieren, können über beliebige Positionen an die benachbarten Gruppen, d.h. die Gruppe R¹O-C(O) und die Gruppe Y oder im Fall der Gruppe Alkandiyloxy an das Sauerstoffatom der Alkandiyloxygruppe, gebunden sein. Die benachbarten Gruppen können an das gleiche Kohlenstoffatom oder verschiedene Kohlenstoffatome in der Gruppe X gebunden sein. In einer Ausführungsform besteht die Kette von Kohlenstoffatomen ein einer Alkandiyl-, Alkendiyl- und Alkindiylgruppe, die in der Gruppe X vorkommt, die die Gruppe R¹O-C(O) direkt mit der Gruppe Y oder im Fall der Gruppe Alkandiyloxy mit dem Sauerstoffatom der Alkandiyloxygruppe verbindet, aus 1, 2, 3 oder 4 Kohlenstoffatomen, in einer anderen Ausführungsform aus 1, 2 oder 3 Kohlenstoffatomen, in einer anderen Ausführungsform aus 1 oder 2 Kohlenstoffatomen, in einer anderen Ausführungsform aus 1 Kohlenstoffatom. Im Fall einer X repräsentierenden Cycloalkandiylgruppe sind in einer Ausführungsform die Gruppen R¹O-C(O) und Y an zwei Ringkohlenstoffatome gebunden, die in 1,2-Position, 1,3-Position oder 1,4-Position zueinander stehen, in einer anderen Ausführungsform in 1,2-Position oder 1,3-Position zueinander, in einer anderen Ausführungsform in 1,2-Position zueinander, in einer anderen Ausführungsform in 1,4-Position zueinander. In einer Ausführungsform ist X ausgewählt aus (C₁-C₆)-Alkandiyl, (C₂-C₆)-Alkendiyl, (C₃-C₇)-Cycloalkandiyl und (C₁-C₆)-Alkandiyloxy, in einer anderen Ausführungsform aus (C₁-C₆)-Alkandiyl, (C₂-C₆)-Alkendiyl und (C₁-C₆)-Alkandiyloxy, in einer anderen Ausführungsform aus (C₁-C₆)-Alkandiyl, (C₃-C₇)-Cycloalkandiyl und (C₁-C₆)-Alkandiyloxy, in einer Ausführungsform aus (C₁-C₆)-Alkandiyl und (C₁-C₆)-Alkandiyloxy, in einer anderen Ausführungsform aus (C₁-C₆)-Alkandiyl, (C₂-C₆)-Alkendiyl, (C₂-C₆)-Alkindiyl und (C₃-C₇)-Cycloalkandiyl, in einer anderen Ausführungsform aus (C₁-C₆)-Alkandiyl, (C₂-C₆)-Alkendiyl und (C₃-C₇)-Cycloalkandiyl, in einer anderen Ausführungsform aus (C₁-C₆)-Alkandiyl und (C₂-C₆)-Alkendiyl, in einer anderen Ausführungsform steht X für (C₁-C₆)-Alkandiyl, in einer anderen Ausführungsform steht X für (C₂-C₆)-Alkendiyl, in einer anderen Ausführungsform steht X für (C₃-C₇)-Cycloalkandiyl, und in einer anderen Ausführungsform steht X für (C₁-C₆)-Alkandiyloxy, die alle gegebenenfalls wie angegeben substituiert sind. In einer Ausführungsform ist eine (C₁-C₆)-Alkandiylgruppe, die in X vorkommt, eine (C₁-C₄)-Alkandiylgruppe, in einer anderen Ausführungsform eine (C₁-C₃)-Alkandiyl-gruppe, in einer anderen Ausführungsform eine (C₁-C₂)-Alkandiylgruppe. In einer Ausführungsform sind die (C₂-C₆)-Alkendiyl- und (C₂-C₆)-Alkindiylgruppen, die X repräsentieren, (C₂-C₄)-Alkendiyl- und (C₂-C₄)-Alkindiylgruppen, in einer anderen Ausführungsform (C₂-C₃)-Alkendiyl- und (C₂-C₃)-Alkindiylgruppen. In einer Ausführungsform ist eine (C₃-C₇)-Cycloalkandiylgruppe, die X repräsentiert, eine (C₃-C₆)-Cycloalkandiylgruppe, in einer anderen Ausführungsform eine (C₃-C₄)-Cycloalkandiylgruppe, in einer anderen Ausführungsform eine Cyclopropandiylgruppe, in einer anderen Ausführungsform eine Cyclohexandiylgruppe. Beispiele für Gruppen X, aus denen die jeweilige X repräsentierende Gruppe in den oben aufgeführten Ausführungsformen gewählt sein kann oder aus denen in einer anderen Ausführungsform der Erfindung X ausgewählt sein kann, sind Methylen, -CH(CH₃)-(Ethan-1,1-diyl), -CH₂-CH₂-(Ethan-1,2-diyl, 1,2-Ethylen), -C(CH₃)₂-(1-Methylethan-1,1-diyl),] -CH₂-CH₂-CH₂-(Propan-1,3-diyl, 1,3-Propylen), -CH₂-CH(CH₃)- und -CH(CH₃)-CH₂- (Propan-1,2-diyl, 1,2-Propylen), die die Gruppe (C₁-C₆)-Alkandiyl exemplifizieren, =CH=CH- (Ethen-1,2-diyl), -CH=CH-CH₂- und -CH₂-CH=CH- (Prop-1-en-1,3-diyl und Prop-2-en-1,3-diyl) und -CH=C(CH₃)- und -C(CH₃)=CH- (Prop-1-en-1,2-diyl), die die Gruppe (C₂-C₆)-Alkendiyl exemplifizieren, -C=C- (Ethindiyl) und -CH₂-C≡C- und -C≡C-CH₂- (Prop-1-in-1,3-diyl und Prop-2-in-1,3-diyl), die die Gruppe (C₂-C₆)-Alkindiyl exemplifizieren, Cyclopropan-1,1-diyl, Cyclopropan-1,2-diyl und Cyclohexan-1,4-diyl, die die Gruppe (C₃-C₇)-Cycloalkandiyl exemplifizieren, -CH₂-O- (Methylenoxy), -CH₂-CH₂-O- (Ethan-1,2-diyloxy), -CH(CH₃)-O- (Ethan-1,1-diyloxy), -C(CH₃)₂-O- (1-Methylethan-1,1-diyloxy), -CH₂-CH₂-CH₂-O- (Propan-1,3-diyloxy) und-CH₂-CH₂-CH₂-CH₂-O- (Butan-1,4-diyloxy), die die Gruppe (C₁-C₆)-Alkandiyloxy exemplifizieren, wobei alle diese Gruppen gegebenenfalls wie angegeben substituiert sind. So ist in einer Ausführungsform X ausgewählt aus -CH₂-O-, -CH₂-CH₂-O-, -CH(CH₃)-O- und -C(CH3)₂-O-, in einer anderen Ausführungsform aus -CH₂-O-, -CH₂-CH₂-O- und -CH(CH₃)-O-, in einer anderen Ausführungsform aus -CH₂-O- und - CH(CH₃)-O-, und in einer anderen Ausführungsform steht X für -CH₂=O-, wobei alle diese Gruppen gegebenenfalls wie angegeben substituiert sind und wobei das Sauerstoffatom an die Gruppe Y gebunden ist. In einer Ausführungsform ist die Zahl der Substituenten, die gegebenenfalls in X vorliegen, 1, 2, 3 oder 4, in einer anderen Ausführungsform 1, 2 oder 3, in einer anderen Ausführungsform 1 oder 2, in einer anderen Ausführungsform 1, und in einer anderen Ausführungsform ist die Gruppe X nicht durch aus Fluor und Hydroxy ausgewählte Substituenten substituiert. In einer Ausführungsform ist die Zahl der Hydroxysubstituenten in X nicht größer als 2, in einer anderen Ausführungsform nicht größer als 1. In einer Ausführungsform liegt an einem einzelnen Kohlenstoffatom in X nicht mehr als ein Hydroxysubstituent vor. In einer Ausführungsform liegen an Kohlenstoffatomen, die Teil einer Doppelbindung in der Gruppe (C₂-C₆)-Alkendiyl sind, keine Hydroxysubstituenten vor. In einer Ausführungsform liegen an dem Kohlenstoffatom in der Gruppe (C₁-C₆)-Alkandiyloxy, das an das Sauerstoffatom gebunden ist, keine Hydroxysubstituenten vor, in einer anderen Ausführungsform liegen an dem Kohlenstoffatom in der Gruppe (C₁-C₆)-Alkandiyloxy, das an das Sauerstoffatom gebunden ist, keine Substituenten vor, d.h. in dieser letztgenannten Ausführungsform sind alle Kohlenstoffatome, die nicht an das Sauerstoffatom gebunden sind, gegebenenfalls durch einen oder mehrere gleiche oder verschiedene Substituenten substituiert, die aus Fluor und Hydroxy ausgewählt sind. Die Doppelbindung in der Gruppe (C₂-C₆)-Alkendiyl kann E-Konfiguration oder Z-Konfiguration haben. In einer Ausführungsform hat sie E-Konfiguration, in einer anderen Ausführungsform hat sie Z-Konfiguration.

In einer Ausführungsform der Erfindung ist die Gruppe R¹ aus Wasserstoff und (C₁-C₄)-Alkyl ausgewählt, in einer anderen Ausführungsform ist R¹ aus Wasserstoff, Methyl, Ethyl, n-Propyl, n-Butyl und Isopropyl ausgewählt, in einer anderen Ausführungsform aus Wasserstoff, Methyl und Ethyl, in einer anderen Ausführungsform steht R¹ für Wasserstoff, in einer anderen Ausführungsform steht R¹ für (C₁-C₄)-Alkyl, in einer anderen Ausführungsform steht R¹ für Methyl.

In einer Ausführungsform der Erfindung ist die Zahl der Ringheteroatome in einem aromatischen Heterocyclus, der Y repräsentiert, 1 oder 2, in einer anderen Ausführungsform 1. In einer Ausführungsform der Erfindung ist Y aus Phenylen und einem zweiwertigen Rest eines aromatischen, 6-gliedrigen monocyclischen Heterocyclus, der 1, 2 oder 3 Ringstickstoffatome, in einer anderen Ausführungsform 1 oder 2 Ringstickstoffatome, in einer anderen Ausführungsform 1 Ringstickstoffatom enthält, ausgewählt, wobei eines der Ringstickstoffatome einen Substituenten R⁴ tragen kann, der Oxy ist, d.h. wobei eines der Ringstickstoffatome zum N-Oxid oxidiert sein kann, und wobei das Phenylen und der zweiwertige Rest eines aromatischen Heterocyclus gegebenenfalls an einem oder mehreren Ringkohlenstoffatomen durch gleiche oder verschiedene Substituenten R⁵ substituiert sind. In einer anderen Ausführungsform steht Y für Phenylen, wobei das Phenylen gegebenenfalls an einem oder mehreren Ringatomen durch gleiche oder verschiedene Substituenten R⁵ substituiert ist, und in einer anderen Ausführungsform steht Y für Pyridindiyl, wobei das Ringstickstoffatom einen Substituenten R⁴, der Oxy ist, tragen kann, d.h. wobei das Ringstickstoffatom zum N-Oxid oxidiert sein kann, und wobei das Pyridindiyl gegebenenfalls an einem oder mehreren Ringkohlenstoffatomen durch gleiche oder verschiedene Substituenten R⁵ substituiert ist. In einer anderen Ausführungsform steht Y für einen zweiwertigen Rest eines aromatischen 5-gliedrigen Heterocyclus, der 1, 2 oder 3 gleiche oder verschiedene Ringheteroatome, die aus N, O und S ausgewählt sind, enthält, wobei eines der Ringstickstoffatome ein Wasserstoffatom oder einen Substituenten R⁴ tragen kann und wobei der zweiwertige Rest eines aromatischen Heterocyclus gegebenenfalls an einem oder mehreren Ringkohlenstoffatomen durch gleiche oder verschiedene Substituenten R⁵ substituiert ist. In einer Ausführungsform ist ein zweiwertiger Rest einer aromatischen heterocyclischen Gruppe, die Y repräsentiert, aus Furandiyl, Thiophendiyl, Oxazoldiyl, Thiazoldiyl, Pyridindiyl, Pyridazindiyl, Pyrimidindiyl und Pyrazindiyl ausgewählt, in einer anderen Ausführungsform aus Furandiyl, Thiophendiyl, Thiazoldiyl, Pyridindiyl, Pyridazindiyl, Pyrimidindiyl und Pyrazindiyl, in einer anderen Ausführungsform aus Furandiyl, Thiophendiyl, Pyridindiyl, Pyridazindiyl, Pyrimidindiyl und Pyrazindiyl, in einer anderen Ausführungsform aus Furandiyl, Thiophendiyl, Pyridindiyl und Pyrimidindiyl, in einer anderen Ausführungsform aus Furandiyl, Thiophendiyl und Pyridindiyl, die alle gegebenenfalls wie in bezug auf Y angegeben substituiert sind.

Die Ringkohlenstoffatome, über die die Phenylengruppe und der zweiwertige Rest eines aromatischen Heterocyclus, die bzw. der Y repräsentieren, an den Oxazolopyrimidinring und die Gruppe X gebunden sind, können in beliebigen Positionen vorliegen. Eine Y repräsentierende Phenylengruppe kann 1,2-Phenylen sein, d.h. der Oxazolopyrimidinring und die Gruppe X können in 1,2-Position oder ortho-Position zueinander gebunden sein, sie kann 1,3-Phenylen sein, d.h. der Oxazolopyrimidinring und die Gruppe X können in 1,3-Position oder meta-Position zueinander gebunden sein, und sie kann 1,4-Phenylen sein, d.h. der Oxazolopyrimidinring und die Gruppe X können in 1,4-Position oder para-Position zueinander gebunden sein. In einer Ausführungsform ist eine Y repräsentierende Phenylengruppe ausgewählt aus 1,3-Phenylen und 1,4-Phenylen, in einer anderen Ausführungsform ist sie 1,3-Phenylen, und in einer anderen Ausführungsform ist sie 1,4-Phenylen, wobei alle diese Gruppen gegebenenfalls wie in bezug auf Y angegeben substituiert sind. In einer Ausführungsform ist Y ausgewählt aus einer oder mehreren der Gruppen Phenylen, Furan-2,5-diyl, Thiophen-2,4-diyl, Thiophen-2,5-diyl, Pyridin-2,4-diyl, Pyridin-2,5-diyl, Pyridin-3,5-diyl, Pyridin-2,6-diyl und Pyrimidin-2,5-diyl, in einer anderen Ausführungsform aus den Gruppen Furan-2,5-diyl, Thiophen-2,4-diyl, Thiophen-2,5-diyl, Pyridin-2,4-diyl, Pyridin-2,5-diyl, Pyridin-3,5-diyl, Pyridin-2,6-diyl und Pyrimidin-2,5-diyl, in einer anderen Ausführungsform aus Pyridin-2,4-diyl, Pyridin-2,5-diyl, Pyridin-3,5-diyl und Pyridin-2,6-diyl, in einer anderen Ausführungsform aus Phenylen, Pyridin-2,4-diyl, Pyridin-2,5-diyl, Pyridin-3,5-diyl und Pyridin-2,6-diyl, die alle gegebenenfalls wie in bezug auf Y angegeben substituiert sind. In einer Ausführungsform ist die Zahl der Substituenten R⁵, die gegebenenfalls an Ringkohlenstoffatomen in Y vorliegen können, 1, 2, 3, 4 oder 5, in einer anderen Ausführungsform 1, 2, 3 oder 4, in einer anderen Ausführungsform 1, 2 oder 3, in einer anderen Ausführungsform 1 oder 2, in einer anderen Ausführungsform 1. Ringkohlenstoffatome in Y, die keinen Substituenten R⁵ tragen, tragen ein Wasserstoffatom.

In einer Ausführungsform der Erfindung sind die Substituenten R⁵, die gegebenenfalls an der Gruppe Y vorliegen, aus Halogen, Hydroxy, (C₁-C₄)-Alkyl-, (C₃-C₅)-Cycloalkyl-C_{z}H_{2z}-, (C₁-C₄)-Alkyloxy-, (C₁-C₄)-Alkyl-S(O)ₘ-, Amino, Nitro und Cyano ausgewählt, in einer anderen Ausführungsform aus Halogen, Hydroxy, (C₁-C₄)-Alkyl-, (C₃-C₅)-Cycloalkyl-C_{z}H_{2z}-, (C₁-C₄)-Alkyloxy-, Amino und Cyano, in einer anderen Ausführungsform aus Halogen, Hydroxy, (C₁-C₄)-Alkyl- und (C₁-C₄)-Alkyloxy-, in einer anderen Ausführungsform aus Fluor, Chlor, Hydroxy, (C₁-C₄)-Alkyl- und (C₁-C₄)-Alkyloxy-, in einer anderen Ausführungsform aus Fluor, Chlor und (C₁-C₄)-Alkyl-, und in einer anderen Ausführungsform sind sie (C₁-C₄)-Alkylsubstituenten, wobei z aus 0, 1 und 2 ausgewählt ist.

In einer Ausführungsform sind 1, 2 oder 3 der Substituenten R⁵, in einer anderen Ausführungsform 1 oder 2 der Substituenten R⁵ und in einer anderen Ausführungsform 1 der Substituenten R⁵, die gegebenenfalls an der Gruppe Y vorliegen, wie in der allgemeinen Definition von R⁵ definiert und somit aus Halogen, Hydroxy, (C₁-C₄)-Alkyl-, (C₃-C₅)-Cycloalkyl-C_{z}H_{2z}-, (C₁-C₄)-Alkyloxy-, (C₁-C₄)-Alkyl-S(O)ₘ-, Amino, Nitro, Cyano, Hydroxycarbonyl, (C₁-C₄)-Alkyloxycarbonyl, Aminocarbonyl und Aminosulfonyl ausgewählt, und jegliche weiteren Substituenten R⁵, die gegebenenfalls an der Gruppe Y vorliegen, beispielsweise 1, 2 oder 3 weitere Substituenten R⁵, oder 1 oder 2 weitere Substituenten R⁵ oder 1 weiterer Substituent R⁵, sind aus Halogen, Hydroxy, (C₁-C₄)-Alkyl-, (C₃-C₅)-Cycloalkyl-C_{z}H_{2z}, (C₁-C₄)-Alkyloxy-, (C₁-C₄)-Alkyl-S(O)ₘ-, Amino, Nitro und Cyano ausgewählt, wobei alle Alkylgrupppen unabhängig voneinander gegebenenfalls durch einen oder mehrere Fluorsubstituenten substituiert sind, wie es allgemein für Alkylgruppen gilt. In einer Ausführungsform sind die Substituenten R⁵, die gegebenenfalls an der Gruppe Y vorliegen und in der oben aufgeführten Ausführungsform wie in der allgemeinen Definition von R⁵ definiert sind, beispielsweise 1 oder 2 derartige Substituenten R⁵ oder 1 derartiger Substituent R⁵, aus Halogen, Hydroxy, (C₁-C₄)-Alkyl-, (C₃-C₅)-Cycloalkyl-C_{z}H_{2z}-, (C₁-C₄)-Alkyloxy-, (C₁-C₄)-Alkyl-S(O)ₘ-, Amino und Cyano ausgewählt, wobei z aus 0, 1 und 2 ausgewählt ist. In einer Ausführungsform befinden sich die Substituenten R⁵, die gegebenenfalls an der Gruppe Y vorliegen und in der oben aufgeführten Ausführungsform wie in der allgemeinen Definition von R⁵ definiert sind, beispielsweise 1 oder 2 derartige Substituenten R⁵ oder 1 derartiger Substituent R⁵, nicht an Ringkohlenstoffatomen in der Gruppe Y, die dem Atom, über das die Gruppe Y an den in Formel I dargestellten Oxazolopyrimidinring gebunden ist, benachbart ist. In einer Ausführungsform sind die weiteren Substituenten R⁵, die gegebenenfalls an der Gruppe Y vorliegen, beispielsweise 1, 2 oder 3 weitere Substituenten R⁵ oder 1 oder 2 weitere Substituenten R⁵ oder 1 weiterer Substituent R⁵, aus Halogen, Hydroxy, (C₁-C₄)-Alkyl-, (C₁-C₄)-Alkyloxy-, Amino, Cyano ausgewählt, in einer anderen Ausführungsform aus Halogen, Hydroxy, (C₁-C₄)-Alkyl-, (C₁-C₄)-Alkyloxy-, in einer anderen Ausführungsform aus Halogen, (C₁-C₄)-Alkyl- und (C₁-C₄)-Alkyloxy-, in einer anderen Ausführungsform aus Halogen und (C₁-C₄)-Alkyl-, wobei in allen diesen Ausführungsformen alle Alkylgruppen unabhängig voneinander gegebenenfalls durch einen oder mehrere Fluorsubstituenten substituiert sind.

In einer Ausführungsform der Erfindung ist die Zahl z aus 0 und 1 ausgewählt, in einer anderen Ausführungsform steht sie für 0, in einer anderen Ausführungsform steht sie für 1.

Gegenstand der Erfindung sind alle Verbindungen der Formel I, worin ein oder mehrere Strukturelemente wie Gruppen, Substituenten und Zahlen wie in einer der angegebenen Ausführungsformen oder Definitionen der Elemente definiert sind oder eine oder mehrere der spezifischen Bedeutungen, die hier als Beispiele für Elemente angegeben sind, besitzen, wobei alle Kombinationen einer oder mehrerer angegebener Ausführungsformen und/oder Definitionen und/oder spezifischer Bedeutungen der Elemente Gegenstand der vorliegenden Erfindung sind. Auch in bezug auf alle derartigen Verbindungen der Formel I sind alle ihre stereoisomeren Formen und Mischungen von stereoisomeren Formen in beliebigem Verhältnis und ihre physiologisch annehmbaren Salze und die physiologisch annehmbaren Solvate davon Gegenstand der vorliegenden Erfindung.

Eine weitere Ausführungsform betrifft Verbindungen der Formel I, worin ein oder mehrere Reste die folgenden Bedeutungen haben:
A ausgewählt ist aus Bindung, -CH₂-, NH, O und S;
X ausgewählt ist aus (C₁-C₆)-Alkandiyl, (C₂-C₆)-Alkendiyl, (C₂-C₆)-Alkindiyl, (C₃-C₇)-Cycloalkandiyl, (C₁-C₆)-Alkandiyloxy und (C₃-C₇)-Cycloalkandiyloxy; die alle gegebenenfalls durch einen oder mehrere gleiche oder verschiedene Substituenten substituiert sind, die aus Fluor und Hydroxy ausgewählt sind, wobei das Sauerstoffatom der (C₁-C₆)-Alkandiyloxy- und (C₃-C₇)-Cycloalkandiyloxygruppen an die Gruppe Y gebunden ist;
Y ausgewählt ist aus Phenylen und einem zweiwertigen Rest eines aromatischen, 5-gliedrigen bis 6-gliedrigen monocyclischen Heterocyclus, der 1, 2 oder 3 gleiche oder verschiedene Ringheteroatome enthält, die aus N, O und S ausgewählt sind, wobei eines der Ringstickstoffatome ein Wasserstoffatom oder einen Substituenten R⁴ tragen kann und wobei das Phenylen und der zweiwertige Rest eines aromatischen Heterocyclus gegebenenfalls an einem oder mehreren Ringkohlenstoffatomen durch gleiche oder verschiedene Substituenten R⁵ substituiert sind;
R¹ ausgewählt ist aus Wasserstoff, (C₁-C₄)-Alkyl und (C₃-C₇)-Cycloalkyl-C_{z}H_{2z}-, worin z aus 0, 1 und 2 ausgewählt ist;
R² ausgewählt ist aus (C₁-C₆)-Alkyl, (C₃-C₅)-Cycloalkyl-CₓH₂ₓ- und Het¹-CₙH₂ₙ-, worin x und n aus 0, 1 und 2 ausgewählt sind;
R³ ausgewählt ist aus (C₁-C₆)-Alkyl, wobei der Alkylrest gegebenenfalls durch ein oder mehrere Fluoratome substituiert ist, (C₂-C₆)-Alkenyl, (C₂-C₆)-Alkinyl, (C₃-C₇)-Cycloalkyl-CᵤH₂ᵤ- und Het²-CₙH₂ₙ-, worin u und n aus 1 und 2 ausgewählt sind, oder R³ für einen Rest eines gesättigten oder ungesättigten, 3-gliedrigen bis 10-gliedrigen; monocyclischen oder bicyclischen Rings, der 0, 1, 2, 3 oder 4 gleiche oder verschiedene Ringheteroatome enthält, die aus N, O und S ausgewählt sind, steht, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist;
R⁴ ausgewählt ist aus (C₁-C₄)-Alkyl, (C₃-C₇)-Cycloalkyl-C_{w}H_{2w}- und Oxy, wobei w aus 0, 1 und 2 ausgewählt ist;
R⁵ ausgewählt ist aus Halogen, Hydroxy, (C₁-C₄)-Alkyl-, (C₃-C₅)-Cycloalkyl-C_{z}H_{2z}-, (C₁-C₄)-Alkyloxy, (C₁-C₄)-Alkyl-S(O)ₘ-, Amino, Nitro, Cyano, Hydroxycarbonyl, (C₁-C₄)-Alkyloxycarbonyl, Aminocarbonyl und Aminosulfonyl, wobei z aus 0, 1 und 2 ausgewählt ist;
R³¹ ausgewählt ist aus Halogen, Hydroxy, (C₁-C₄)-Alkyl, (C₁-C₄)-Alkyloxy, (C₃-C₇)-Cycloalkyl, Oxo, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Nitro, Cyano, (C₁-C₄)-Alkylcarbonyl, Aminosulfonyl, (C₁-C₄)-Alkylaminosulfonyl und Di((C₁-C₄)-alkyl)aminosulfonyl;
Het¹ für einen Rest eines gesättigten, 4-gliedrigen bis 6-gliedrigen, monocyclischen gesättigten Heterocyclus steht, der 1 oder 2 Sauerstoffatome enthält, und der über ein Ringkohlenstoffatom gebunden ist, und wobei der Rest eines Heterocyclus gegebenenfalls durch einen oder mehrere gleiche oder verschiedene Substituenten substituiert ist, die unter Fluor und (C₁-C₄)-Alkyl ausgewählt sind;
Het² für einen Rest eines gesättigten, 4-gliedrigen bis 7-gliedrigen, monocyclischen Heterocyclus steht, der 1 oder 2 gleiche oder verschiedene Ringheteroatome enthält, die unter N, O und S ausgewählt sind, und der über ein Ringkohlenstoffatom gebunden ist, wobei der Rest eines Heterocyclus gegebenenfalls durch einen oder mehrere gleiche oder verschiedene Substituenten substituiert ist, die unter Fluor und (C₁-C₄)-Alkyl ausgewählt sind;
m aus 0, 1 und 2 ausgewählt ist.

Eine weitere Ausführungsform betrifft Verbindungen der Formel I, worin ein oder mehrere Reste die folgenden Bedeutungen haben:
A eine Bindung, -CH₂-, NH, O oder S;
X (C₁-C₆)-Alkandiyloxy, wobei das Sauerstoffatom der (C₁-C₆)-Alkandiyloxygruppe an die Gruppe Y gebunden ist;
Y Phenylen, wobei das Phenylen gegebenenfalls an einem oder mehreren Ringkohlenstoffatomen durch gleiche oder verschiedene Substituenten R⁵ substituiert ist;
R¹ Wasserstoff oder (C₁-C₄)-Alkyl;
R² (C₁-C₆)-Alkyl;
R³ (C₁-C₆)-Alkyl, wobei der Alkylrest gegebenenfalls durch ein oder mehrere Fluoratome substituiert ist, (C₂-C₆)-Alkenyl, (C₂-C₆)-Alkinyl, (C₃-C₇)-Cycloalkyl-CᵤH₂ᵤ- oder Het-CᵥH₂ᵥ-, worin u und v aus 1 und 2 ausgewählt sind, oder R³ für einen Rest eines gesättigten oder ungesättigten, 3-gliedrigen bis 10-gliedrigen, monocyclischen oder bicyclischen Rings, der 0, 1, 2, 3 oder 4 gleiche oder verschiedene Ringheteroatome enthält, die aus N, O und S ausgewählt sind, steht, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist;
R⁵ Halogen, Hydroxy, (C₁-C₄)-Alkyl-, (C₃-C₅)-Cycloalkyl-C_{z}H_{2z}-, (C₁-C₄)-Alkyloxy, (C₁-C₄)-Alkyl-S(O)ₘ-, Amino, Nitro, Cyano, Hydroxycarbonyl, (C₁-C₄)-Alkyloxycarbonyl, Aminocarbonyl oder Aminosulfonyl, wobei z aus 0, 1 und 2 ausgewählt ist;
R³¹ Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Nitro, Cyano, (C₁-C₄)-Alkylcarbonyl, Aminosulfonyl, (C₁-C₄)-Alkylaminosulfonyl oder Di((C₁-C₄)-alkyl)aminosulfonyl;
m aus 0, 1 und 2 ausgewählt ist, wobei alle Zahlen m voneinander unabhängig sind.

Eine weitere Ausführungsform betrifft Verbindungen der Formel I, worin ein oder mehrere Reste die folgenden Bedeutungen haben:
A -CH₂- oder O;
X (C₁-C₆)-Alkandiyloxy, wobei das Sauerstoffatom der (C₁-C₆)-Alkandiyloxygruppe an die Gruppe Y gebunden ist;
Y Phenylen, wobei das Phenylen gegebenenfalls an einem oder mehreren Ringkohlenstoffatomen durch gleiche oder verschiedene Substituenten R⁵ substituiert ist;
R¹ Wasserstoff oder (C₁-C₄)-Alkyl,
R² (C₁-C₆)-Alkyl;
R³ (C₁-C₆)-Alkyl, wobei der Alkylrest gegebenenfalls durch ein oder mehrere Fluoratome substituiert ist, oder Phenyl, wobei der Phenylrest gegebenenfalls an einem oder mehreren Ringkohlenstoffatomen durch gleiche oder verschiedene Substituenten R³¹ substituiert ist;
R⁵ Halogen, Hydroxy, (C₁-C₄)-Alkyl-, (C₃-C₅)-Cycloalkyl-C_{z}H_{2z}-, (C₁-C₄)-Alkyloxy, (C₁-C₄)-Alkyl-S(O),-, Amino, Nitro, Cyano, Hydroxycarbonyl, (C₁-C₄)-Alkyloxycarbonyl, Aminocarbonyl oder Aminosulfonyl, wobei z aus 0, 1 und 2 ausgewählt ist;
R³¹ Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Nitro, Cyano, (C₁-C₄)-Alkylcarbonyl, Aminosulfonyl, (C₁-C₄)-Alkylaminosulfonyl oder Di((C₁-C₄)-alkyl)aminosulfonyl;
m aus 0, 1 und 2 ausgewählt ist.

Eine weitere Ausführungsform betrifft Verbindungen der Formel I, worin ein oder mehrere Reste die folgenden Bedeutungen haben:
A -CH₂- oder O;
X (C₁-C₆)-Alkandiyloxy, wobei das Sauerstoffatom der (C₁-C₆)-Alkandiyloxygruppe an die Gruppe Y gebunden ist;
Y Phenylen, wobei das Phenylen gegebenenfalls an einem oder mehreren Ringkohlenstoffatomen durch gleiche oder verschiedene Substituenten R⁵ substituiert ist;
R¹ Wasserstoff;
R² (C₁-C₆)-Alkyl;
R³ (C₁-C₆)-Alkyl, wobei der Alkylrest gegebenenfalls durch ein oder mehrere Fluoratome substituiert ist, oder Phenyl, wobei der Phenylrest gegebenenfalls an einem oder mehreren Ringkohlenstoffatomen durch gleiche oder verschiedene Substituenten R³¹ substituiert ist;
R⁵ (C₁-C₄)-Alkyl;
R³¹ Halogen.

Ebenso gilt auch in bezug auf alle hier offenbarten spezifischen Verbindungen, wie die Beispielverbindungen, die Ausführungsformen der Erfindung repräsentieren, in denen die verschiedenen Gruppen und Zahlen in der allgemeinen Definition der Verbindungen der Formel I die in der jeweiligen spezifischen Verbindung vorliegenden spezifischen Bedeutungen besitzen, daß sie in einer beliebigen ihrer stereoisomeren Formen und/oder einer Mischung von stereoisomeren Formen in beliebigem Verhältnis und in Form ihrer physiologisch akzeptablen Salze und in Form der physiologisch akzeptablen Solvate derartiger Verbindungen oder derartiger Salze Gegenstand der vorliegenden Erfindung sind. Unabhängig davon, ob eine spezifische Verbindung hier als freie Verbindung und/oder als spezifisches Salz offenbart wird, ist sie sowohl in Form der freien Verbindung als auch in Form aller ihrer physiologisch akzeptablen Salze und bei Offenbarung eines spezifischen Salzes zusätzlich in Form dieses spezifischen Salzes und in Form der physiologisch akzeptablen Solvate einer derartigen Verbindung oder derartiger Salze Gegenstand der Erfindung. Gegenstand der Erfindung ist somit auch eine Verbindung der Formel I, die aus einer oder mehreren der hier offenbarten spezifischen Verbindungen der Formel I einschließlich der nachstehend angeführten Beispielverbindungen ausgewählt ist, und die physiologisch akzeptablen Salze davon und die physiologisch akzeptablen Solvate einer derartigen Verbindung oder derartiger Salze, wobei die Verbindung der Formel I in einer beliebigen ihrer stereoisomeren Formen oder als Mischung von stereoisomeren Formen in beliebigem Verhältnis, sofern anwendbar, Gegenstand der Erfindung ist. Als Beispiel genannt ist eine Verbindung der Formel I oder ein physiologisch akzeptables Solvat davon, die ausgewählt ist aus {4-[7-Isobutyl-5-(3,3,3-trifluoro-propoxy)-oxazolo[5,4-d]pyrimidin-2-yl]-2,6-dimethyl-phenoxy}-essigsäure, {4-[5-(3-Chloro-phenoxy)-7-isobutyl-oxazolo[5,4-d]pyrimidin-2-yl]-2,6-dimethyl-phenoxyl-essigsäure, {4-[5-(3-Chloro-phenoxy)-7-propyl-oxazolo[5,4-d]pyrimidin-2-yl]-2,6-dimethyl-phenoxy}-essigsäure, {4-[5-(4-Chloro-benzyl)-7-isobutyl-oxazolo[5,4-d]pyrimidin-2-yl]-2,6-dimethyl-phenoxy}-essigsäure, [4-(5-Benzyl-7-isobutyl-oxazolo[5,4-d]pyrimidin-2-yl)-2,6-dimethyl-phenoxy]-essigsäure und{4-[5-(4-Chloro-benzyl)-7-isopropyl-oxazolo[5,4-d]pyrimidin-2-yl]-2,6-dimethyl-phenoxy}-essigsäure.

Ein weiterer Gegenstand der vorliegenden Erfindung sind Verfahren zur Herstellung der Verbindungen der Formel I und ihrer Salze und Solvate, nach denen die Verbindungen erhältlich sind und die im Folgenden umrissen werden. Bei einem Verfahren setzt man eine Verbindung der Formel II mit einer Verbindung der Formel III zu einer Verbindung der Formel I um, 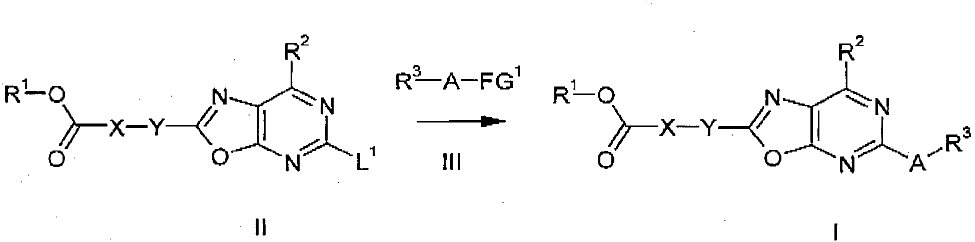 worin die Gruppen A, X, Y, R¹, R² und R³ in den Verbindungen der Formeln II und III wie in den Verbindungen der Formel I definiert sind und zusätzlich funktionelle Gruppen in geschützter Form oder in Form einer Vorläufergruppe, die später in die endgültige Gruppe umgewandelt wird, vorliegen können. Die Gruppe L¹ in den Verbindungen der Formel II ist eine Abgangsgruppe, die in einer, ggf. kataysierten, aromatischen Substitutionsreaktion ausgetauscht werden kann, wie ein Halogenatom, beispielsweise Fluor, Chlor, Brom oder Iod, oder eine Alkylthio- oder eine Sulfoxidgruppe oder eine Sulfongruppe, beispielsweise eine Gruppe der Formel -S-Alk oder -S(O)-Alk oder - S(O)₂-Alk, worin Alk eine (C₁-C₄)-Alkylgruppe, beispielsweise Methyl oder Ethyl, ist. Die Gruppe FG¹ in den Verbindungen der Formel III, ist eine Gruppe, die in einer, ggf. kataysierten, aromatischen Substitutionsreaktion aus dem Reagenz der Formel III abgespalten werden kann und nicht im Produkt der Formel I verbleibt. So kann FG¹ beispielsweise ein Proton sein, insbesondere für Verbindungen der Formel III, in der A für NH, O oder S steht. Alternativ kann FG¹ für einen Boronsäure- oder Boronsäureester-Rest, einen Trialkylstannyl-Rest oder einen Lithium-, Zinkhalogenid oder Magnesiumhalogenid-Rest stehen, insbesondere für Verbindungen der Formel III, in denen A für eine Bindung oder -CH₂- steht.

Die Reaktion der Verbindungen der Formeln II und III ist eine, ggf. katalysierte, aromatische Substitutionsreaktion an dem Kohlenstoffatom in der 6-Position des Oxazolo[5,4-d]pyrimidinrings, d.h. in der Pyrimidingruppierung, und kann unter Standardbedingungen für derartige Reaktionen, die dem Fachmann gut bekannt sind, durchgeführt werden. Die Reaktion kann auch in Gegenwart von Katalysatorsystemen, z.B. Natriumtolylsulfinat oder Eisen-, Kupfer- oder Palladium-salzen oder -komplexen, durchgeführt werden. Im allgemeinen wird die Reaktion in einem inerten Lösungsmittel, beispielsweise einem Kohlenwasserstoff wie Benzol, Toluol, Xylol oder Chlorbenzol, einem Ether wie Tetrahydrofuran (THF), Dioxan, Dibutylether, Diisopropylether oder 1,2-Dimethoxyethan (DME), einem Amin wie N,N-Dimethylformamid (DMF), N,N-Dimethylacetamid (DMA) oder N-Methylpyrrolidin-2-on (NMP) oder einer Lösungsmittelmischung bei Temperaturen von etwa -20°C bis etwa 250°C, beispielsweise bei Temperaturen von etwa 40°C bis etwa 200°C, je nach den Besonderheiten des jeweiligen Falls, durchgeführt. Im allgemeinen ist es günstig, zur Erhöhung der Reaktivität eine Base zuzusetzen, beispielsweise ein tertiäres Amin, wie Triethylamin, Ethyldiisopropylamin oder N-Methylmorpholin, oder eine anorganische Base wie ein Erdalkalimetallhydrid, -hydroxid, -carbonat oder -hydrogencarbonat wie Natriumhydrid, Natriumhydroxid, Kaliumhydroxid, Natriumcarbonat, Kaliumcarbonat, Cäsiumcarbonat oder Natriumhydrogencarbonat oder ein Alkoxid oder Amid wie Natriummethoxid, Natriumethoxid, Kaliummethoxid, Kalium-tert.-butoxid, Natriumamid oder Lithiumdiisopropylamid. Eine Verbindung der Formel III, in der FG¹ für ein Proton steht, kann auch vor der Reaktion mit der Verbindung der Formel II separat mit einer Base behandelt und in ein Salz umgewandelt werden. Wenn die Reaktion in Gegenwart eines Katalysatorsystems durchgeführt wird, können hierbei Katalysatoren zu Einsatz kommen, die ein Metallion oder ein Metall in der Oxidationstufe 0 enthalten können, hierbei kommen bevorzugt Eisen, Kupfer oder Palladium zum Einsatz. Die Katalyse erfordert häufig die Gegenwart bestimmter metall-komplexierender Liganden, die die Bildung einer katalytisch aktiven Spezies erst ermöglichen oder sie stabilisieren. Metall-Ligand-Komplexe können der Reaktion zugegeben werden oder in situ gebildet werden. Beispielsweise können solche Katalysatorsysteme Kupfer oder Kupfer(I)salze, besonders Kupfer(I)hafogenide oder Kupfer(I)carboxylate, insbesondere Kupfer(I)iodid oder Kupfer(I)thiophencarboxylat, oder auch vorgebildete Kupfer(I)-Komlplexe, z.B. Tetrakis(acetnitril)kupfer(I)hexafluorophoshat, allein oder in Gegenwart von Liganden, z.B. Diaminliganden oder 1,10-Phenanthrolin, enthalten. Des weiteren können solche Katalysatorsysteme zum Beispiel aus Palladium-komplexen oder Palladiumsalzen in Gegenwart von Liganden, z. B. aus Palladium(0)komplexen, insbesondere Tris(dibenzylidenaceton)dipalladium(0), oder Palladiumacetat, Palladiumtrifluoracetat oder Palladiumhalogeniden, insbesondere Palladiumchlorid, in Gegenwart von Liganden, insbesondere Diphosphinliganden wie z.B. 2,2'-Bis(dipheynlphosphino)-1-1'-binapthyl oder 4,5-Bis(diphenylphosphino)-9,9-dimethylxanthene oder vorgebildeten wie Bis(tri-tert-butylphosphin)palladium(O) bestehen oder gebildet werden .Des weiteren können solche Kataysatorsysteme auch Eisen(III)-salze, wie z.B. Eisen(III)acetylacetonat enthalten. Außerdem können auch einfache Katalysatoren zum Einsatz kommen, z. B. kann eine nucleophile aromatische Substitution von 2-Pyrimidinhalogeniden, insesondere -chloriden durch substituierte Alkali- oder Erdalkali-Benzolsulfinate, insbesondere durch Natriumtolylsulfinat, katalysiert werden.

Die Ausgangsverbindungen der Formeln II und III sind nach in der Literatur beschriebenen Verfahrensweisen oder in Anlehnung daran erhältlich und in vielen Fällen im Handel erhältlich. Verbindungen der Formel IIa, d.h. Verbindungen der Formel II, in denen L¹ beispielsweise für eine Sulfoxidgruppe der Formel Alk-S(O)- oder eine Sulfongruppe der Formel Alk-S(O)₂- steht, sind durch Umsetzung eines Aminomalonsäureesters der Formel IV mit einem aktivierten Carbonsäurederivat der Formel V zu einer Verbindung der Formel VI, Umsetzung der letzteren Verbindung mit Thioharnstoff der Formel VII zu einer Verbindung der Formel VIII, Alkylierung des Thiols mit einem Alkylierungsreagens der Formel IX zum Thioether der Formel X, Cyclisierung der letzteren Verbindung unter Bildung des Oxazolo[5,4-d]pyrimidinringsystems zur Verbindung der Formel XI, Überführung der letzteren Verbindung in eine Verbindung der Formel XII, wobei der Rest R¹O-C(O)-X- durch Umsetzung mit einer Verbindung der Formel XIII zur Verbindung der Formel XIV eingeführt wird, anschließende Umsetzung der Verbindung der Formel XIV mit einer Verbindung der Formel XV zu einer Verbindung der Formel XVI und Oxidation der Thioethergruppierung in der erhaltenen Verbindung der Formel XVI zu dem entsprechenden Sulfoxid oder Sulfon der Formel IIa erhältlich.

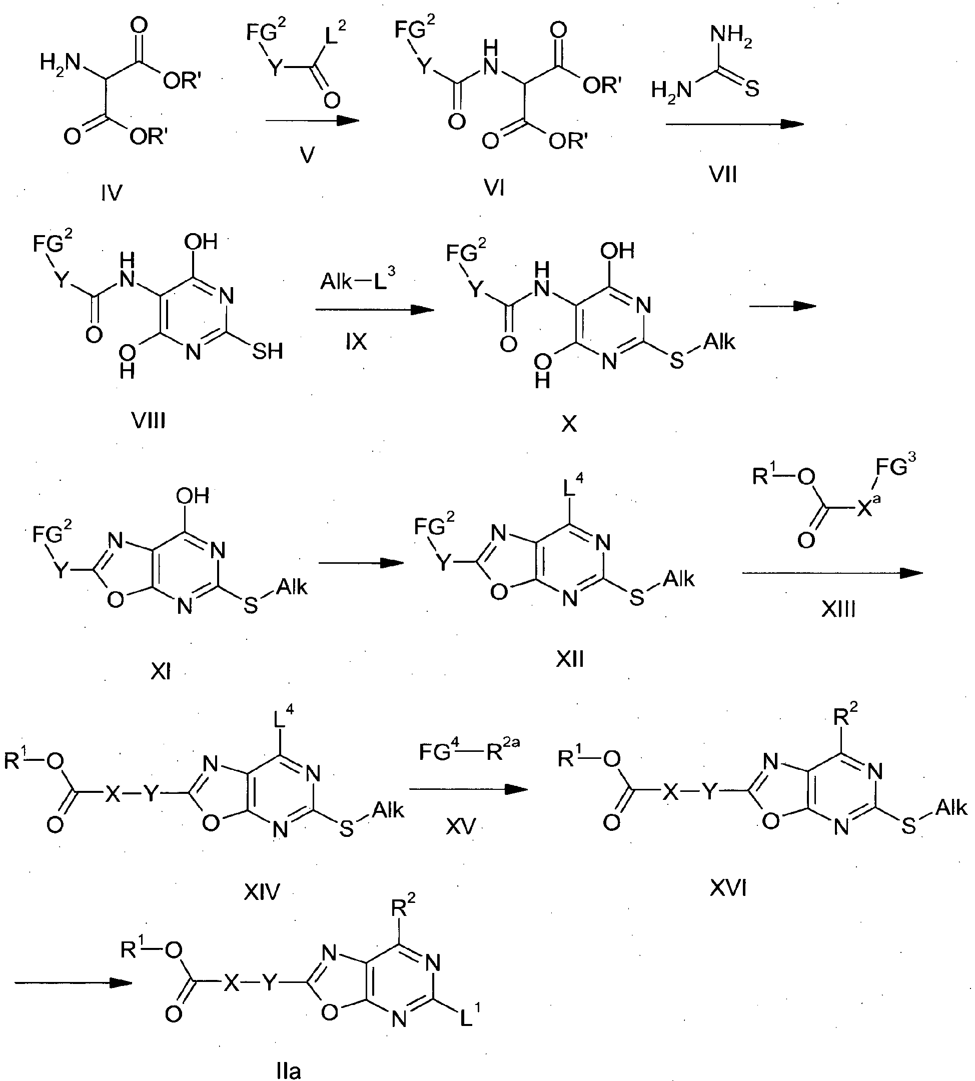

Die Gruppen X, Y, R¹ und R² in den Verbindungen der Formeln IIa, V, VI, VIII, X, XI, XII, XIII, XIV und XVI sind wie in den Verbindungen der Formel I definiert, und zusätzlich können funktionelle Gruppen in geschützter Form oder in Form einer Vorläufergruppe, die später in die endgültige Gruppe umgewandelt wird, vorliegen.

Die Gruppe R^{2a} in der Verbindung der Formel XV ist definiert wie die Gruppe R² in der Verbindung der Formel I, kann aber außerdem eine Doppel-oder Dreifachbindung benachbart zur Bindung zu FG⁴ enthalten.

Die Gruppe X^{a} in den Verbindungen der Formel XIII ist wie die Gruppe X in den Verbindungen der Formel I definiert oder umfaßt einen Teil der Gruppe X in der gewünschten Verbindung der Formel II, so daß nach der Umsetzung der Verbindungen der Formeln XII und XIII die Gruppe X^{a} und jegliche in der Verbindung der Formel XIV verbleibende Teile der Gruppen FG² und FG³ zusammen die gewünschte Gruppe X bilden. So kann beispielsweise in dem Fall, daß die Gruppe X für eine Alkandiyloxygruppe steht, die Gruppe X^{a} in der Verbindung der Formel XIII die gewünschte Alkandiyloxygruppe sein und die Gruppe FG³ kann ein an das Sauerstoffatom gebundenes Wasserstoffatom sein, oder die Gruppe X^{a} kann der Alkandiylteil sein, die Gruppe FG³ ist eine Abgangsgruppe, und die Gruppe FG² in der Verbindung der Formel XII ist eine Hydroxygruppe, deren Sauerstoffatom zusammen mit dem Alkandiylteil dann nach Alkylierung der Verbindung der Formel XII mit der Verbindung der Formel XIII die gewünschte Alkandiyloxygruppe bildet.

Die Gruppen FG² und FG³ in den Verbindungen der Formeln V, VI, VIII, X, XI, XII und XIII sind funktionelle Gruppen, die für den zur Bildung der gewünschten Gruppe X aus der Gruppe X^{a} und jeglichem in der Verbindung der Formel XIV verbleibenden Teil der Gruppen FG² und FG³ verwendeten Kupplungstyp geeignet sind. Beispielsweise kann es sich dann, wenn die Gruppe X^{a} über eine nukleophile Substitutionsreaktion an die Gruppe Y² oder an ein Atom in der Gruppe FG², wie ein Sauerstoffatom in einer Hydroxygruppe, die FG² repräsentiert, wie oben erwähnt, gebunden wird, bei FG³ um eine Abgangsgruppe wie ein Halogenatom wie Chlor, Brom oder Iod oder eine Sulfonyloxygruppe wie Methansulfonyloxy, Trifluormethansulfonyloxy oder Toluolsulfonyloxy handeln. Wenn die Gruppe X^{a} über eine übergangsmetallkatalysierte Reaktion an die Gruppe Y gebunden wird, kann es sich bei FG³ um eine Abgangsgruppe wie eine Boronsäure-, Boronsäureester-, Dialkylboran- oder Trialkylstannylgruppe handeln, und in diesem Fall kann FG² Halogen sein. Bei FG³ kann es sich auch um ein Wasserstoffatom oder ein Kohlenstoffatom, das Teil einer Doppelbindung in einer Alkendiylgruppe, die X^{a} repräsentiert, handeln, wenn zur Verknüpfung von X^{a} mit Y eine Heck-Reaktion verwendet wird, und in diesem Fall kann FG² Halogen sein. Bei Verwendung einer Wittig-Reaktion oder Wittig-Horner-Reaktion zur Verknüpfung von X^{a} mit Y kann es sich bei FG³ um eine Phosphoniogruppe wie Triphenylphosphonio oder eine Phosphonylgruppe wie Diethylphosphonyl handeln, und die Verbindung der Formel XIII kann ein Phosphoniumsalz oder ein Phosphonsäureester sein, und in diesem Fall kann FG² eine Aldehydgruppe -C(O)-H oder Ketongruppe -C(O)-Alkyl sein, und umgekehrt. Im Allgemeinen liegt die Gruppe FG² an dem Kohlenstoffatom in der Phenylengruppe oder heterocyclischen Gruppe, die Y repräsentiert, die in den Verbindungen der Formeln XIV, XVI, IIa und I die Gruppe X trägt, vor. Die Gruppe FG² in den Verbindungen der Formeln V, VI, VIII, X, XI und XII kann auch in geschützter Form oder in Form einer Vorläufergruppe, die später in die Gruppe umgewandelt wird, die in der Verbindung der Formel XII mit der Verbindung der Formel XIII reagiert, vorliegen. So kann beispielsweise eine Hydroxygruppe, die in der Verbindung der Formel XII FG² repräsentiert, in den Verbindungen der Formeln V, VI, VIII, X und XI in geschützter Form vorliegen, beispielsweise in Form einer veretherten Hydroxygruppe wie eines Benzylethers oder eines Alkylethers wie eines Methylethers. Derartige Ether können nach dem Fachmann gutbekannten Methoden gespalten werden. Eine Zusammenfassung von Methoden zur Abspaltung von Schutzgruppen findet sich in der Literatur, beispielsweise in P. J. Kocienski, Protecting Groups (Thieme Verlag, 1994), oder T. W. Greene und P. G. M. Wuts, Protective Groups in Organic Synthesis (John Wiley & Sons, 1999).

Die Gruppe FG⁴ in der Verbindung der Formel XV ist eine funktionelle Gruppe, die für eine Kupplung der Verbindungen XIV und XV zu einer Verbindung der Formel XVI geeignet ist. So kann FG⁴ für einen Lithium-, Zinkhalogenid- oder Magnesiumhalogenid-Rest stehen, wenn die Bildung der Verbindung der Formel XVI über eine ggf. katalysierte nucleophile aromatische Substitution erfolgt, oder es kann sich bei FG⁴ um eine Abgangsgruppe wie eine Boronsäure-, Boronsäureester-, Dialkylboran- oder Trialkylstannylgruppe handeln, wenn es sich bei der Bildung der Verbindung der Formel XVI um einen Suzuki- oder Stille-artigen Kupplungstyp handelt, oder es kann sich bei FG⁴ um ein Proton handeln, wenn R^{2a} eine Doppel- oder Dreifachbindung benachbart zur Bindung zu FG⁴ enthält und die Bildung der Verbindung XVI über eine Heck- oder Sonogashira-artige Kupplungsreaktion mit anschließender Hydrierung erfolgt.

Die Gruppe L¹ in den Verbindungen der Formel IIa ist wie oben angegeben definiert.

Die Gruppe L² in den Verbindungen der Formel V ist eine nukleophil substituierbare Abgangsgruppe und kann insbesondere ein Halogenatom, wie Chlor oder Brom, sein, und die Verbindung der Formel V kann somit ein Carbonsäurehalogenid sein. L² kann auch eine Gruppe der Formel FG²-Y-C(O)-O sein, und die Verbindung der Formel V kann somit beispielsweise ein Carbonsäureanhydrid sein.

Die Gruppe L³ in Verbindungen der Formel IX ist eine Abgangsgruppe, die in einer nukleophilen Substitutionsreaktion ersetzt werden kann, und kann insbesondere ein Halogenatom wie Chlor, Brom oder Iod oder eine Sulfonyloxygruppe wie Methansulfonyloxy, Trifluormethansulfonyloxy oder Toluolsulfonyloxy sein, d.h. die Verbindung der Formel IX kann beispielsweise ein organisches Halogenid oder Sulfonat sein.

Die Gruppe L⁴ in Verbindungen der Formeln XII und XIV ist eine Abgangsgruppe, die in einer aromatischen Substitutionsreaktion ersetzt werden kann, und kann insbesondere ein Halogenatom wie Chlor, Brom oder Iod sein.

Die Gruppe R' in den Verbindungen der Formeln IV und VI kann Alkyl wie beispielsweise (C₁-C₃)-Alkyl, wie Methyl oder Ethyl, sein.

Wie erwähnt, können die Verbindungen der Formel XI auch in einer anderen tautomeren Form vorliegen, beispielsweise in Form der jeweiligen 6H-Oxazolo[5,4-d]pyrimidin-7-one oder 4H-Oxazolo[5,4-d]pyrimidin-7-one. Soweit anwendbar, gilt für alle Verbindungen, die bei der Herstellung der Verbindungen der Formel I vorkommen, daß sie in einer anderen tautomeren Form als der in ihren Formeln dargestellten vorliegen können. Bei den Reaktionen dieses Verfahrens zur Herstellung der Verbindungen der Formel II können wie bei allen anderen Reaktionen, die bei der Herstellung der Verbindungen der Formel I durchgeführt werden, Ausgangsverbindungen auch in Form eines Salzes eingesetzt und/oder Produkte in Form eines Salzes erhalten werden. So können beispielsweise Verbindungen der Formeln IV in Form eines Säureadditionssalzes wie des Hydrochlorids eingesetzt werden.

Die Umsetzung der Verbindungen der Formeln IV und V kann unter Standardbedingungen für die Acylierung eines Amins mit einem aktivierten Carbonsäurederivat wie einem Säurehalogenid oder -anhydrid durchgeführt werden. Im allgemeinen wird die Umsetzung in einem inerten Lösungsmittel, beispielsweise einem Kohlenwasserstoff oder chlorierten Kohlenwasserstoff wie Benzol, Toluol, Xylol, Chlorbenzol, Dichlormethan, Chloroform oder Dichlorethan, einem Ether wie THF, Dioxan, Dibutylether, Diisopropylether oder DME, einem Keton wie Aceton oder Butan-2-on, einem Ester wie Essigsäureethylester oder Essigsäurebutylester oder Wasser, oder einer Mischung von Lösungsmitteln, bei Temperaturen von etwa -10°C bis etwa 40°C, beispielsweise bei Temperaturen von etwa 0°C bis etwa 30°C durchgeführt. Im Allgemeinen wird die Umsetzung unter Zugabe einer Base, beispielsweise eines tertiären Amins, wie Triethylamin, Ethyldiisopropylamin oder N-Methylmorpholin oder einer anorganischen Base wie eines Alkalimetallhydroxids, -carbonats oder - hydrogencarbonats wie Natriumhydroxid, Kaliumhydroxid, Natriumcarbonat, Kaliumcarbonat oder Natriumhydrogencarbonat durchgeführt.

Die Umsetzung der Verbindungen der Formeln VI und VII wird im allgemeinen in einem inerten Lösungsmittel, beispielsweise einem Alkohol, wie Methanol, Ethanol oder Isopropanol, oder einem Ether wie THF, Dioxan oder DME oder einer Mischung von Lösungsmitteln bei Temperaturen von etwa 20°C bis etwa 80°C, beispielsweise Temperaturen von etwa 40°C bis etwa 80°C, in Gegenwart einer Base, beispielsweise eines Alkoxids wie Natriummethoxid, Natriumethoxid, Kaliummethoxid oder Kalium-tert.-butoxid, durchgeführt.

Die Umsetzung der Verbindungen der Formeln VIII und IX ist eine nukleophile Substitutionsreaktion am Kohlenstoffatom in der Gruppe Alk, die die Gruppe L³ trägt, und kann unter Standardbedingungen für derartige Umsetzungen, die dem Fachmann gut bekannt sind, durchgeführt werden. Im allgemeinen wird die Umsetzung je nach den Besonderheiten des jeweiligen Falls in einem inerten Lösungsmittel, beispielsweise einem Kohlenwasserstoff oder chloriertem Kohlenwasserstoff wie Benzol, Toluol, Xylol, Chlorbenzol, Dichlormethan, Chloroform oder Dichlorethan, einem Ether wie THF, Dioxan, Dibutylether, Diisopropylether oder DME, einem Alkohol wie Methanol, Ethanol oder Isopropanol, einem Keton wie Aceton oder Butan-2-on, einem Ester wie Essigsäureethylester oder Essigsäurebutylester, einem Nitril wie Acetonitril, einem Amid wie DMF oder NMP, oder einer Mischung von Lösungsmitteln einschließlich zweiphasiger Mischungen mit wäßrigen Lösungen bei Temperaturen von etwa -20°C bis etwa 100°C, beispielsweise bei Temperaturen von etwa -10°C bis etwa 30°C durchgeführt. Im allgemeinen ist es günstig, zur Erhöhung der Nukleophilie der Verbindung der Formel VIII und/oder zur Bindung einer Säure, die bei der Umsetzung freigesetzt wird, eine Base, beispielsweise ein tertiäres Amin, wie Triethylamin, Ethyldiisopropylamin oder N-Methylmorpholin, oder eine anorganische Base wie ein Alkalimetallhydrid, -hydroxid, -carbonat oder -hydrogencarbonat wie Natriumhydrid, Natriumhydroxid, Kaliumhydroxid, Natriumcarbonat, Kaliumcarbonat, Cäsiumcarbonat oder Natriumhydrogencarbonat oder ein Alkoxid oder Amid wie Natriummethoxid, Natriumethoxid, Kaliummethoxid, Kalium-tert-butoxid, Natriumamid oder Lithiumdiisopropylamid zuzusetzen. Eine Verbindung der Formel VIII kann auch vor der Umsetzung mit der Verbindung der Formel IX separat mit einer Base behandelt und in ein Salz umgewandelt werden.

Die Cyclisierung der Verbindung der Formel X zu der Verbindung der Formel XI kann günstigerweise in Gegenwart eines Phosphorhalogenids, wie Phosphorpentachlorid oder Phosphoroxidchlorid oder einer Mischung davon, in einem inerten Lösungsmittel, beispielsweise einem Kohlenwasserstoff oder chloriertem Kohlenwasserstoff wie Benzol, Toluol, Xylol, Chlorbenzol, Dichlormethan, Chloroform oder Dichlorethan bei Temperaturen von etwa 20°C bis etwa 100°C, beispielsweise Temperaturen von etwa 50°C bis etwa 80°C, durchgeführt werden.

Die Umsetzung der Verbindung der Formel XI zu einer Verbindung der Formel XII kann ebenfalls in Gegenwart eines eines Phosphorhalogenids, wie Phosphorpentachlorid oder Phosphoroxidchlorid oder einer Mischung davon, in einem inerten Lösungsmittel, beispielsweise einem Kohlenwasserstoff oder chloriertem Kohlenwasserstoff wie Benzol, Toluol, Xylol, Chlorbenzol, Dichlormethan, Chloroform oder Dichlorethan bei Temperaturen von etwa 20°C bis etwa 150°C, beispielsweise Temperaturen von etwa 50°C bis etwa 100°C, durchgeführt werden. Die Verbindung der Formel X kann auch ohne Isolierung der Verbindung der Formel XI direkt in die Verbindung der Formel XIII überführt werden.

Die Kupplung von Verbindungen der Formel XII mit Verbindungen der Formel XIII kann durch Reaktionen verschiedener Typen durchgeführt werden, wie oben bereits angegeben, beispielsweise über eine Alkylierungsreaktion. So kann die Gruppe Y beispielsweise dann, wenn sie eine Hydroxygruppe, die FG² repräsentiert, trägt, unter Verwendung einer Verbindung der Formel XIII, in der FG³ für eine für nukleophile Substitutionsreaktionen geeignete Abgangsgruppe wie ein Halogenatom wie Chlor, Brom oder Iod oder eine Sulfonyloxygruppe wie Methansulfonyloxy oder Toluolsulfonyloxy steht, alkyliert werden. Die nukleophile Substitutionsreaktion an dem Kohlenstoffatom der Gruppe XIII, die die Gruppe FG³ trägt, kann unter Standardbedingungen für derartige Reaktionen, die dem Fachmann gut bekannt sind, durchgeführt werden. Im Allgemeinen wird die Umsetzung je nach den Besonderheiten des jeweiligen Falls in einem inerten Lösungsmittel, beispielsweise einem Kohlenwasserstoff oder chlorierten Kohlenwasserstoff wie Benzol, Toluol, Xylol, Chlorbenzol, Dichlormethan, Chloroform oder Dichlorethan, einem Ether wie THF, Dioxan, Dibutylether, Diisopropylether oder DME, einem Alkohol wie Methanol, Ethanol oder Isopropanol, einem Keton wie Aceton oder Butan-2-on, einem Ester wie Essigsäureethylester oder Essigsäurebutylester, einem Nitril wie Acetonitril, einem Amid wie N,N-Dimethylformamid oder N-Methylpyrrolidin-2-on, oder einer Mischung von Lösungsmitteln bei Temperaturen von etwa 20°C bis etwa 100°C, beispielsweise bei Temperaturen von etwa 40°C bis etwa 80°C durchgeführt. Im Allgemeinen ist es günstig, zur Erhöhung der Nukleophilie der Verbindung der Formel XIII und/oder zur Bindung einer Säure, die bei der Umsetzung freigesetzt wird, eine Base, beispielsweise ein tertiäres Amin, wie Triethylamin, Ethyldiisopropylamin oder N-Methylmorpholin, oder eine anorganische Base wie Alkalimetallhydrid, -hydroxid, -carbonat oder -hydrogencarbonat wie Natriumhydrid, Natriumhydrid,Natriumhydroxid, Kaliumhydroxid, Natriumcarbonat, Kaliumcarbonat, Cäsiumcarbonat oder Natriumhydrogencarbonat oder ein Alkoxid oder Amid wie Natriummethoxid, Natriumethoxid, Kaliummethoxid, Kalium-tert.-butoxid, Natriumamid oder Lithiumdiisopropylamid zuzusetzen. Eine Verbindung der Formel XII, worin FG² für Hydroxy steht, kann auch vor der Umsetzung mit der Verbindung der Formel XIII separat mit einer Base behandelt und in ein Salz umgewandelt werden. Eine Verbindung der Formel XII, worin FG² für Hydroxy steht, kann nicht nur durch Umsetzung mit einer Verbindung der Formel XIII, worin FG³ für eine Abgangsgruppe wie angegeben steht, in eine Verbindung der Formel XIV umgewandelt werden, sondern auch durch Umsetzung mit dem entsprechenden Alkohol, d.h. mit einer Verbindung der Formel XIII, worin FG³ für Hydroxy steht, unter den dem Fachmann bekannten Bedingungen der Mitsunobu-Reaktion. Die Kupplung von Verbindungen der Formel XII mit Verbindungen der Formel XIII über eine übergangsmetallkatalysierte Reaktion kann auch unter den Bedingungen von palladiumkatalysierten Kreuzkupplungsreaktionen wie der Heck-, Stille- oder Suzuki-Kupplungsreaktion durchgeführt werden (siehe A. de Meijere und F. Diederich (Hrsg.), Metal-Catalyzed Cross-Coupling Reactions (Wiley-VCH, 2004)).

Die Umsetzung von Verbindungen der Formel XIV mit Verbindungen der Formel XV zu Verbindungen der Formel XVI kann durch Reaktionen verschiedener Typen durchgeführt werden, wie oben bereits angegeben, beispielsweise über eine, ggf. katalysierte, aromatische Substitutionsreaktion, bei der FG⁴ für für einen Lithium-, Zinkhalogenid- oder Magnesiumhalogenid-Rest stehen kann. Im allgemeinen wird die Reaktion in einem inerten Lösungsmittel, beispielsweise einem Kohlenwasserstoff wie Benzol, Toluol, Xylol oder Chlorbenzol, einem Ether wie Tetrahydrofuran (THF), Dioxan, Dibutylether, Diisopropylether oder 1,2-Dimethoxyethan (DME), einem Amin wie N,N-Dimethylformamid (DMF), N,N-Dimethylacetamid (DMA) oder N-Methylpyrrolidin-2-on (NMP) oder einer Lösungsmittelmischung bei Temperaturen von etwa 20°C bis etwa 250°C, beispielsweise bei Temperaturen von etwa 40°C bis etwa 200°C, je nach den Besonderheiten des jeweiligen Falls, durchgeführt. Die Reaktion kann unkatalysiert oder in Gegenwart eines Katalysatorsystems durchgeführt werden, dabei können Katalysatoren zum Einsatz kommen, die ein Metallion oder ein Metall in der Oxidationstufe 0 enthalten können, hierbei kommen bevorzugt Eisen oder Palladium zum Einsatz. Die Katalyse erfordert häufig die Gegenwart bestimmter metallkomplexierender Liganden, die die Bildung einer katalytisch aktiven Spezies erst ermöglichen oder sie stabilisieren. Metall-Ligand-Komplexe können der Reaktion zugegeben werden oder in situ gebildet werden. Beispielsweise können solche Katalysatorsysteme aus Palladium-komplexen oder Palladiumsalzen in Gegenwart von Liganden, z.B. aus Paliadium(0)komplexen, insbesondere Tris(dibenzyliden-aceton)dipalladium(O), oder Palladiumacetat, Palladiumtrifluoracetat oder Palladium-halogeniden, insbsondere Palladiumchlorid, in Gegenwart von Liganden, insbesondere Diphosphinliganden wie z.B. 2,2'-Bis(diphenylphosphino)-1-1'-binaphtyl oder 4,5-Bis(diphenylphosphino)-9,9-dimethylxanthene oder vorgebildeten Komplexen wie Bis(tri-tert-butylphosphin)palladium(0) bestehen oder gebildet werden .Des weiteren können solche Kataysatorsysteme auch Eisen(III)-salze, wie z.B. Eisen(III)acetylacetonat enthalten. Weiterhin kann die Umsetzung von Verbindungen der Formel XIV mit Verbindungen der Formel XV zu Verbindungen der Formel XVI durch eine Suzuki- oder Stille-artige Kupplungsreaktion erfolgen, wobei FG⁴ dann für eine Abgangsgruppe wie eine Boronsäure-, Boronsäureester-, Dialkylboran- oder Trialkylstannylgruppe steht. Weiterhin kann die Umsetzung von Verbindungen der Formel XIV mit Verbindungen der Formel XV zu Verbindungen der Formel XVI durch eine Heck- oder Sonogashira-artige Kupplungsreaktion erfolgen, wobei FG⁴ dann für ein Proton steht und R^{2a} eine Doppel- oder Dreifachbindung benachbart zur Bindung zu FG⁴ enthält und die nach der Kupplungsreaktion resultierende Doppel- oder Dreifachbindung unmittelbar oder auf einer späteren Stufe durch eine Hydrierungsreaktion, die unter dem Fachmann bekannten Standardbedingungen in Gegenwart eines Katalysators wie z.B. Palladium auf Aktivkohle in einem geeigneten inerten Lösungsmittel, wie z.B. Ethanol oder Ethylacetat, durchgeführt werden kann, wieder zu Verbindungen mit einem gesättigten Rest R² führt. Diese Suzuki-, Stille- Heck- und Sonogashira-artigen Kupplungs-reaktionen können beispielsweise unter Palladiumkatalyse bzw. unter Bedingungen, wie sie in der Literatur (siehe A. de Meijere und F. Diederich (Hrsg.), Metal-Catalyzed Cross-Coupling Reactions (Wiley-VCH, 2004)) beschrieben sind, durchgeführt werden. Die Oxidation der Alk-S-Gruppe in den Verbindungen der Formel XVI zur Sulfoxidgruppe oder Sulfongruppe in den Verbindungen der Formel IIa kann mit Hilfe von Wasserstoffperoxid oder einer Persäure wie 3-Chlorperbenzoesäure oder Monoperoxyphthalsäure in einem inerten Lösungsmittel, beispielsweise einem chlorierten Kohlenwasserstoff wie Dichlormethan oder Chloroform oder einem Ester wie Essigsäureethylester oder Essigsäurebutylester, bei Temperaturen von etwa 0°C bis etwa 40°C, beispielsweise bei etwa 20°C, durchgeführt werden.

Man kann auch die Reihenfolge der Schritte bei der Herstellung der Verbindungen der Formel I ändern.

So kann man zum Beispiel zunächst einen Aminomalonsäureester der Formel IV wie den Diethylester in Gegenwart eines Alkalimetallalkoxids wie Natriumethoxid mit Thioharnstoff umsetzen, dann das Schwefelatom alkylieren, beispielsweise mit Iodmethan methylieren, und das erhaltene Produkt mit einer Verbindung der Formel V acylieren, und so zu einer Verbindung der Formel X gelangen.

Des weiteren kann man zum Beispiel eine Verbindung der Formel XII mit einer Verbindung der Formel XV zu einer Verbindung der Formel XVII umsetzen, und durch anschließende Umsetzung der letzeren mit einer Verbindung der Formel XIII zu einer Verbindung der Formel XVI und Oxidation der Thioethergruppierung in der erhaltenen Verbindung der Formel XVI zu dem entsprechenden Sulfoxid oder Sulfon der Formel IIa gelangen.

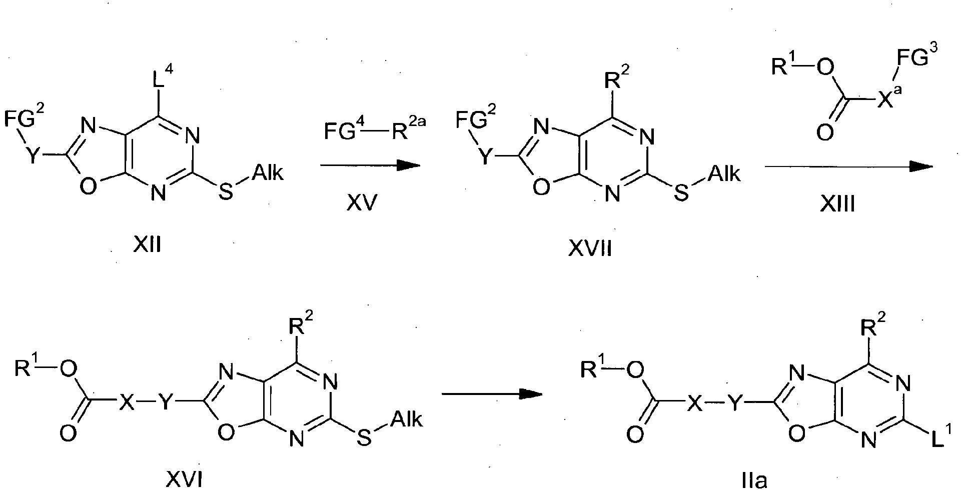

Dabei sind die Gruppen X, Y, R¹ und R² in den Verbindungen der Formeln IIa, XII, XIII, XVI und XVII wie in den Verbindungen der Formel I definiert, und die Gruppen R^{2a}, X^{a}, L¹, L⁴, FG², FG³ und FG⁴ in den Verbindungen der Formeln IIa, XII, XIII, XV und XVII sind wie oben definiert. Die Umsetzungen können unter den oben beschriebenen Bedingungen durchgeführt werden.

Bei einem weiteren Verfahren zur Synthese von Verbindungen der Formel I setzt man eine Verbindung der Formel XVIII mit einer Verbindung der Formel XV zu einer Verbindung der Formel I um, 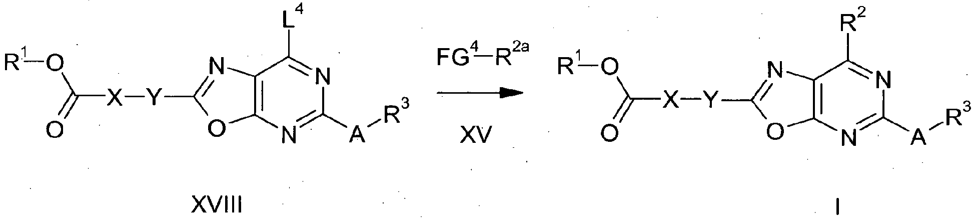 worin die Gruppen A, X, Y, R¹,R² und R³ in den Verbindungen der Formel XVIII wie in den Verbindungen der Formel I definiert sind und zusätzlich funktionelle Gruppen in geschützter Form oder in Form einer Vorläufergruppe, die später in die endgültige Gruppe umgewandelt wird, vorliegen können. Die Gruppen L⁴, FG⁴ und R^{2a} in den Verbindungen der Formeln XV und XVII sind wie oben definiert.

Die Umsetzung von Verbindungen der Formel XVIII mit Verbindungen der Formel XV zu Verbindungen der Formel I kann durch Reaktionen verschiedener Typen erfolgen, wie oben bereits angegeben, beispielsweise über eine ggf. katalysierte aromatische Substitutionsreaktion, bei der FG⁴ für für einen Lithium-, Zinkhalogenid- oder Magnesiumhalogenid-Rest stehen kann. Im allgemeinen wird die Reaktion in einem inerten Lösungsmittel, beispielsweise einem Kohlenwasserstoff wie Benzol, Toluol, Xylol oder Ghlorbenzol, einem Ether wie Tetrahydrofuran (THF), Dioxan, Dibutylether, Diisopropylether oder 1,2-Dimethoxyethan (DME), einem Amin wie N,N-Dimethylform-amid (DMF), N,N-Dimethylacetamid (DMA) oder N-Methylpyrrolidin-2-on (NMP) oder einer Lösungsmittelmischung bei Temperaturen von etwa -20°C bis etwa 250°C, beispielsweise bei Temperaturen von etwa 0°C bis etwa 200°C, je nach den Besonderheiten des jeweiligen Falls, durchgeführt. Die Reaktion kann unkatalysiert oder in Gegenwart eines Katalysatorsystems durchgeführt werden, dabei können Katalysatoren zum Einsatz kommen, die ein Metallion oder ein Metall in der Oxidationstufe 0 enthalten können, hierbei kommen bevorzugt Eisen oder Palladium zum Einsatz. Die Katalyse erfordert häufig die Gegenwart bestimmter metall-komplexierender Liganden, die die Bildung einer katalytisch aktiven Spezies erst ermöglichen oder sie stabilisieren. Metall- Ligand-Komplexe können der Reaktion zugegeben werden oder in situ gebildet werden. Beispielsweise können solche Katalyssatorsysteme aus Palladium-komplexen oder Palladiumsalzen in Gegenwart von Liganden, z.B. aus Palladium(0)komplexen, insbesondere Tris(dibenzylidenaceton)dipalladium(0), oder Palladiumacetat, Palladiumtrifluoracetat oder Palladiumhalogeniden, insbsondere Palladiumchlorid, in Gegenwart von Liganden, insbesondere Diphosphinliganden wie z.B. 2,2'-Bis(dipheynlphosphino)-1-1'-binapthyl oder 4,5-Bis(diphenylphosphino)-9,9-dimethylxanthene oder vorgebildeten Komplexen wie Bis(tri-tert-butylphosphin)-palladium(O) bestehen oder gebildet werden .Des weiteren können solche Kataysatorsysteme auch Eisen(III)-salze, wie z.B. Eisen(III)acetylacetonat enthalten. Weiterhin kann die Umsetzung von Verbindungen der Formel XVIII mit Verbindungen der Formel XV zu Verbindungen der Formel I durch Suzuki- oder Stille-artige Kupplungsreaktionen erfolgen, wobei FG⁴ dann für eine Abgangsgruppe wie eine Boronsäure-, Boronsäureester-, Dialkylboran- oder Trialkylstannylgruppe steht. Weiterhin kann die Umsetzung von Verbindungen der Formel XVIII mit Verbindungen der Formel XV zu Verbindungen der Formel I durch Heck- oder Sonogashira-artige Kupplungsreaktionen erfolgen, wobei FG⁴ dann für ein Proton steht und R^{2a} eine Doppel- oder Dreifachbindung benachbart zur Bindung zu FG⁴ enthält und die nach der Kupplungsreaktion resultierende Doppel- oder Dreifachbindung unmittelbar durch eine Hydrierungsreaktion, die unter dem Fachmann bekannten Standardbedingungen in Gegenwart eines Katalysators wie z.B. Palladium auf Aktivkohle in einem geeigneten inerten Lösungsmittel, wie z.B. Ethanol oder Ethylacetat, durchgeführt werden kann, wieder zu Verbindungen mit einem gesättigten Rest R² führt. Diese Suzuki-, Stille- Heck- und Sonogashira-artigen Kupplungsreaktionen können beispielsweise unter Palladiumkatalyse bzw. unter Bedingungen, wie sie in der Literatur (siehe A. de Meijere und F. Diederich (Hrsg.), Metal-Catalyzed Cross-Coupling Reactions (Wiley-VCH, 2004)) beschrieben sind, durchgeführt werden.

Die Ausgangsverbindungen der Formeln XV und XVIII sind nach in der Literatur beschriebenen Verfahrensweisen oder in Anlehnung daran erhältlich und in vielen Fällen im Handel erhältlich. Verbindungen der Formel XVIII sind durch Umsetzung einer Vebindung der Formel XIV, die wie oben beschrieben hergestellt werden kann, durch Oxidation der Thioethergruppierung in der Verbindung der Formel XIV zu einem Sulfoxid oder Sulfon der Formel IXX und anschließende Umsetzung der letzteren mit einer Verbindung der Formel III erhältlich, 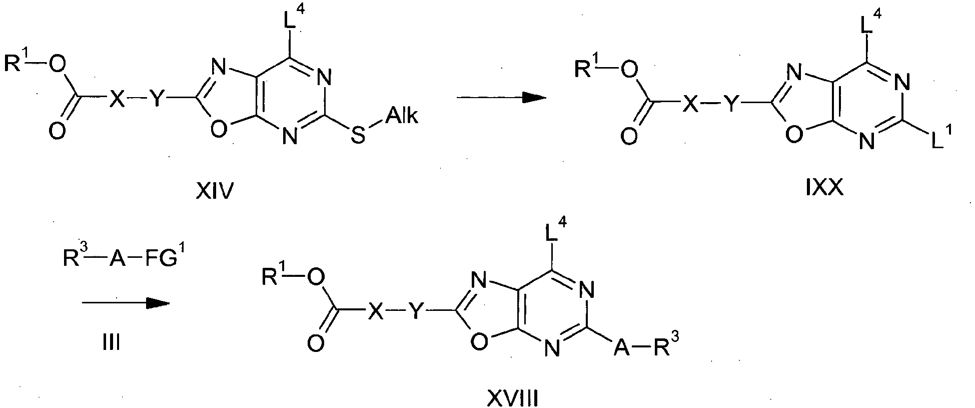 worin die Gruppen A, X, Y, R¹ und R³ in den Verbindungen der Formeln III; XIV, XVIII und IXX wie in den Verbindungen der Formel I definiert sind und zusätzlich funktionelle Gruppen in geschützter Form oder in Form einer Vorläufergruppe, die später in die endgültige Gruppe umgewandelt wird, vorliegen können. Die Gruppe L¹ in der Verbindung IXX ist wie in der Verbindung IIa definiert und die Gruppen L⁴ und FG¹ in den Verbindungen der Formeln III, XIV, XVIII und IXX sind wie oben definiert.

Die Oxidation der Alk-S-Gruppe in den Verbindungen der Formel XIV zur Sulfoxidgruppe oder Sulfongruppe in den Verbindungen der Formel IX kann mit Hilfe von Wasserstoffperoxid oder einer Persäure wie 3-Chlorperbenzoesäure oder Monoperoxyphthalsäure in einem inerten Lösungsmittel, beispielsweise einem chlorierten Kohlenwasserstoff wie Dichlormethan oder Chloroform oder einem Ester wie Essigsäureethylester oder Essigsäurebutylester, bei Temperaturen von etwa 0°C bis etwa 40°C, beispielsweise bei etwa 20°C, durchgeführt werden.

Die Reaktion der Verbindungen der Formeln IXX und III ist eine, ggf. kataysierte, aromatische Substitutionsreaktion an dem Kohlenstoffatom in der 6-Position des Oxazolo[5,4-d]pyrimidinrings, d.h. in der Pyrimidingruppierung, und kann unter Standardbedingungen für derartige Reaktionen, die dem Fachmann gut bekannt sind, durchgeführt werden. Die Reaktion kann auch in Gegenwart von Katalysatoren, z.B. substituierten Alkali- oder Erdalkali-Benzolsulfinaten, insbesondere Natriumtolylsulfinat, durchgeführt werden. Im allgemeinen wird die Reaktion in einem inerten Lösungsmittel, beispielsweise einem Kohlenwasserstoff wie Benzol, Toluol, Xylol oder Chlorbenzol, einem Ether wie Tetrahydrofuran (THF), Dioxan, Dibutylether, Diisopropylether oder 1,2-Dimethoxyethan (DME), einem Amin wie N,N-Dimethylformamid (DMF), N,N-Dimethylacetamid (DMA) oder N-Methylpyrrolidin-2-on (NMP) oder einer Lösungsmittelmischung bei Temperaturen von etwa -80°C bis etwa 250°C, beispielsweise bei Temperaturen von etwa 0°C bis etwa 200°C, je nach den Besonderheiten des jeweiligen Falls, durchgeführt. Im allgemeinen ist es günstig, zur Erhöhung der Reaktivität eine Base zuzusetzen, beispielsweise ein tertiäres Amin, wie Triethylamin, Ethyldiisopropylamin oder N-Methylmorpholin, oder eine anorganische Base wie ein Erdalkalimetallhydrid, -hydroxid, -carbonat oder-hydrogencarbonat wie Natriumhydrid, Natriumhydroxid, Kaliumhydroxid, Natriumcarbonat, Kaliumcarbonat, Cäsiumcarbonat oder Natriumhydrogencarbonat oder ein Alkoxid oder Amid wie Natriummethoxid, Natriumethoxid, Kaliummethoxid, Kalium-tert.-butoxid, Natriumamid oder Lithiumdiisopropylamid. Eine Verbindung der Formel III, in der FG¹ für ein Proton steht, kann auch vor der Reaktion mit der Verbindung der Formel II separat mit einer Base behandelt und in ein Salz umgewandelt werden. Eine Reaktion mit Verbindungen der Formel III, in der FG¹ für einen einenLithium-, Zinkhalogenid oder Magnesiumhalogenid-und A für eine Bindung oder -CH₂- stehen, kann auch durch Eisen(III)-salze, wie z.B. Eisen(III)acetylacetonat, katalysiert werden.

Alternativ ist auch eine direkte Umsetzung von Verbindungen der Formel XIV mit Verbindungen der Formel III zu Verbindungen der Formel XVIII möglich, wenn FG¹ für einen Boronsäure- oder Boronsäureester-Rest oder einen Trialkylstannyl-und A für eine Bindung oder -CH₂- steht. Diese können durch Katalysatorsysteme katalysiert werden, die Kupfer oder Kupfer(I)salze, besonders Kupfer(I)halogenide oder Kupfer(I)carboxylate, insbesondere Kupfer(i)iodid oder Kupfer(I)thiophencarboxylat, oder auch vorgebildete Kupfer(I)-Komplexe, z.B. Tetrakis(acetnitril)kupfer(I)hexafluorophoshat, allein oder in Gegenwart von Liganden, z.B. Diaminliganden oder 1,10-Phenanthrolin, enthalten. Im allgemeinen wird die Reaktion in einem inerten Lösungsmittel, beispielsweise einem Kohlenwasserstoff wie Benzol, Toluol, Xylol oder Chlorbenzol, einem Ether wie Tetrahydrofuran (THF), Dioxan, Dibutylether, Diisopropylether oder 1,2-Dimethoxyethan (DME), einem Amin wie N,N-Dimethylformamid (DMF), N,N-Dimethylacetamid (DMA) oder N-Methylpyrrolidin-2-on (NMP) oder einer Lösungsmittelmischung bei Temperaturen von etwa 20°C bis etwa 250°C, beispielsweise bei Temperaturen von etwa 40°C bis etwa 200°C, je nach den Besonderheiten des jeweiligen Falls, durchgeführt.

Man kann auch die Reihenfolge der Schritte bei der Herstellung der Verbindungen der Formel I ändern.

So kann man zum Beispiel zunächst eine Verbindung der Formel XII zu einer Verbindung der Formel XX umsetzen, anschließend diese Verbindung mit einer Verbindung der Formel III zu einer Verbindung der Formel XXI umsetzen, die letztere dann mit einer Verbindung der Formel XIII zur Reaktion bringen, um eine Verbindung der Formel XVIII zu erhalten.

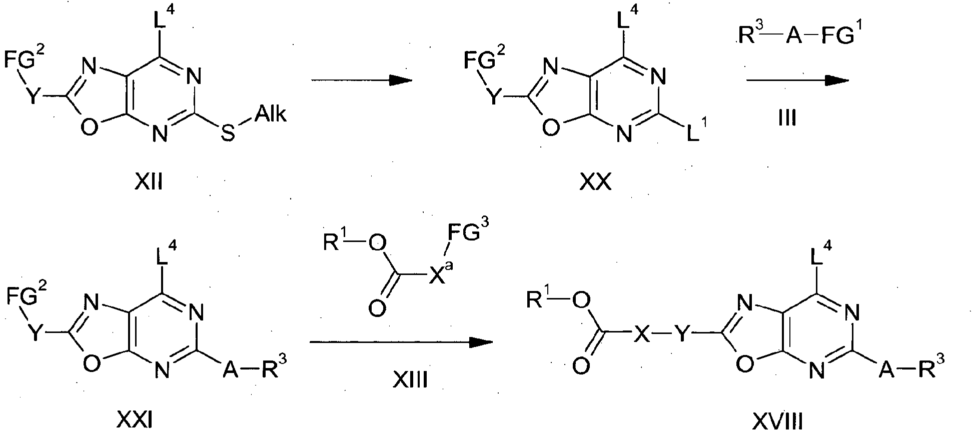

Dabei sind die Gruppen A, X, Y, R¹ und R³ in den Verbindungen der Formeln III, XII, XIII, XVIII, XX und XXI wie in den Verbindungen der Formel I definiert, die Gruppe L¹ in der Verbindung der Formel XX ist wie in der Verbindung IIa definiert, und die Gruppen X^{a}, L⁴, FG¹, FG² und FG³ in den Verbindungen der Formeln III, XII, XIII, XVIII, XX und XXI sind wie oben definiert. Die Umsetzungen können unter den oben beschriebenen Bedingungen durchgeführt werden. Alternativ ist auch, wie oben beschrieben eine direkte Umsetzung von Verbindungen der Formel XII mit Verbindungen der Formel III zu Verbindungen der Formel XXI möglich, wenn FG¹ für einen Boronsäure- oder Boronsäureester-Rest oder einen Trialkylstannyl-und A für eine Bindung oder -CH₂-steht.

Alternativ sind Verbindungen der Formel XVIIIa, d.h. Verbindungen, in denen A für eine Bindung oder für eine CH₂-Gruppe steht, durch Umsetzung eines Aminomalonsäureesters der Formel IV mit einem aktivierten Carbonsäurederivat der Formel V zu einer Verbindung der Formel VI, Umsetzung der letzteren Verbindung mit einem Amidin der Formel XXIIa zu einer Verbindung der Formel XXIIIa, Cyclisierung der letzteren Verbindung unter Bildung des Oxazolo[5,4-d]pyrimidinringsystems zur Verbindung der Formel XXIVa, Überführung der letzteren Verbindung in eine Verbindung der Formel XXIa, und anschließende Einführung des Restes R¹O-C(O)-X- durch Umsetzung mit einer Verbindung der Formel XIII erhältlich.

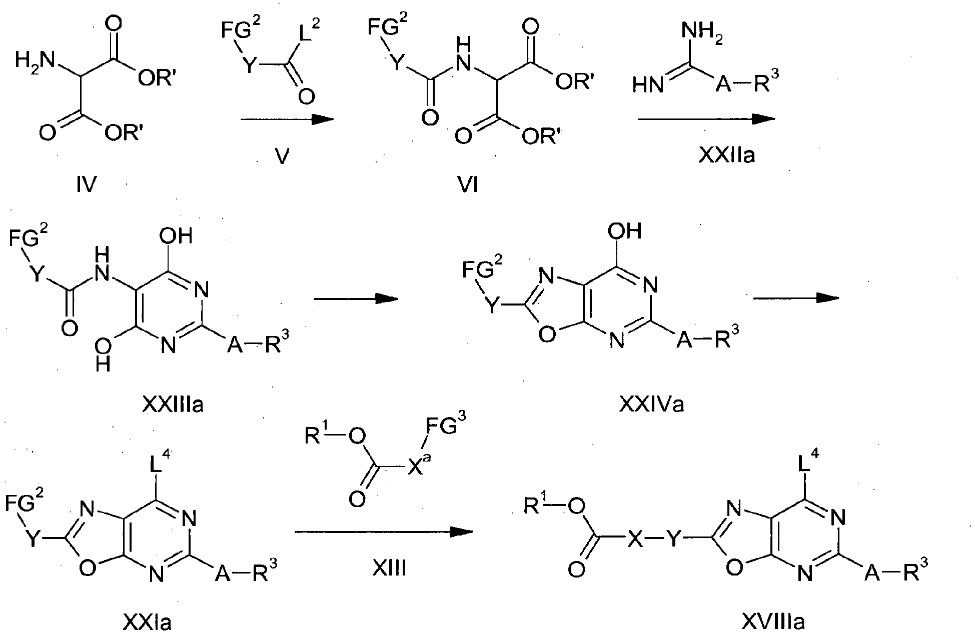

Dabei sind die Gruppen X, Y, R¹ und R³ in den Verbindungen der Formeln V,VI, XIII, XVIIIa, XXIa, XXIIa, XXIIIa und XXIVa sind wie in den Verbindungen der Formel I definiert, die Gruppe A in den Verbindungen der Formeln XVIIIa, XXIa, XXIIa, XXIIIa und XXIVa steht für eine Bindung oder eine CH₂-Gruppe, und die Bedeutungen der Gruppen R', X^{a}, L², L⁴, FG² und FG³ in den Verbindungen der Formeln IV, V,VI, XIII, XVIIIa, XXIa, XXIIa, XXIIIa und XXIVa sind wie oben definiert.

Die Umsetzung der Verbindungen IV und V zur Verbindung VI erfolgt, wie oben bereits beschrieben. Die Umsetzung der Verbindungen der Formel VI mit Verbindungen der Formel XXIIa wird im allgemeinen in einem inerten Lösungsmittel, beispielsweise einem Alkohol, wie Methanol, Ethanol oder Isopropanol, oder einem Ether wie THF, Dioxan oder DME oder einer Mischung von Lösungsmitteln bei Temperaturen von etwa 20°C bis etwa 200°C, beispielsweise Temperaturen von etwa 20°C bis etwa 100°C, in Gegenwart einer Base, beispielsweise eines Alkoxids wie Natriummethoxid, Natriumethoxid, Kaliummethoxid oder Kalium-tert.-butoxid, durchgeführt. Dabei kann das Amidin der Formel XXIIa auch als Salz, beispielsweise als Hydrochlorid, eingesetzt werden. Die Cyclisierung der Verbindungen der Formel XXIIIa zu Verbindungen der Formel XXIVa und die Überführung in die Verbindungen der Formel XXIa kann unter Bedingungen erfolgen, wie sie für die Umsetzung der Verbindungen der Formel X zu Verbindungen der Formel XI und die Überführung der Verbindungen der Formel XI in die Verbindungen der Formel XII oben beschrieben wurden. Die Umsetzung der Verbindungen der Formel XXIa mit der Verbindungen der Formel XIII zu Verbindungen der Formel XVIII kann unter Bedingungen erfolgen, die für die Umsetzung der Verbindungen der Formel XII mit Verbindungen der Formel XIII zu Verbindungen der Formel XIV oben beschrieben wurden.

Bei einem weiteren Verfahren zur Synthese von Verbindungen der Formel I setzt man eine Verbindung der Formel XXV mit einer Verbindung der Formel XIII zu einer Verbindung der Formel I um, 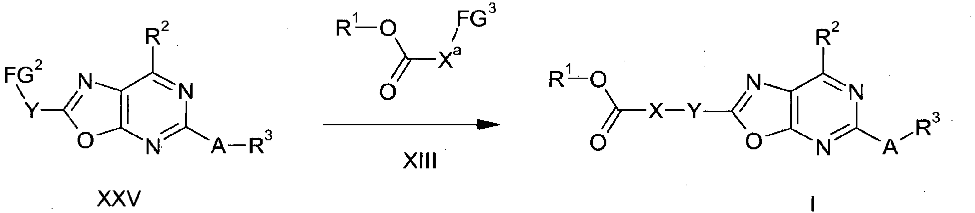 worin die Gruppen A, X, Y, R¹, R² und R³ in den Verbindungen der Formeln XIII und XXV wie in den Verbindungen der Formel I definiert sind und zusätzlich funktionelle Gruppen in geschützter Form oder in Form einer Vorläufergruppe, die später in die endgültige Gruppe umgewandelt wird, vorliegen können. Die Gruppen X^{a}, FG² und FG³ in den Verbindungen der Formeln XIII und XXV sind wie oben definiert.

Die Umsetzung von Verbindungen der Formel XXV zu Verbindungen der Formel I kann unter Bedingungen erfolgen, wie sie für die Umsetzung von Verbindungen der Formel XII zu Verbindungen der Formel XIV bereits oben beschrieben sind.

Die Ausgangsverbindungen der Formeln XXV und XIII sind nach in der Literatur beschriebenen Verfahrensweisen oder in Anlehnung daran erhältlich und in vielen Fällen im Handel erhältlich. Verbindungen der Formel XXV sind durch Überführung einer Vebindung der Formel XVII, die wie oben beschrieben hergestellt werden kann, in eine Verbindung der Formel XXVI und anschließende Umsetzung der letzteren mit einer Verbindung der Formel III erhältlich, 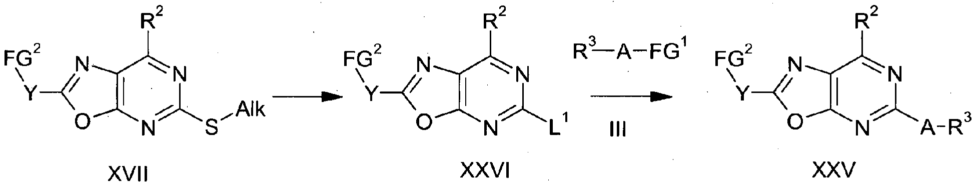 worin die Gruppen A, Y, R² und R³ in den Verbindungen der Formeln III; XVII, XXV und XXVI wie in den Verbindungen der Formel I definiert sind und zusätzlich funktionelle Gruppen in geschützter Form oder in Form einer Vorläufergruppe, die später in die endgültige Gruppe umgewandelt wird, vorliegen können. Die Gruppe L¹ in der Verbindung XXVI ist wie in der Verbindung IIa definiert und die Gruppen FG¹ und FG² in den Verbindungen der Formeln III; XVII, XXV und XXVI sind wie oben definiert.

Die Überführung einer Vebindung der Formel XVII in eine Verbindung der Formel XXVI kann dabei unter Bedingungen durchgeführt werden, wie sie für die Umsetzung von Verbindungen der Formel XVI zu Verbindungen der Formel IIa bereits oben beschrieben wurden. Die Umsetzung von Verbindungen der Formel XXVI mit Verbindungen der Formel III zu Verbindungen der Formel XXV kann unter Bedingungen erfolgen, wie sie für die Umsetzung von Verbindungen der Formel IIa zu Verbindungen der Formel I bereits oben beschrieben wurden.

Des weiteren sind Verbindungen der Formel XXV durch Umsetzung einer Vebindung der Formel XXI, die wie oben beschrieben hergestellt werden kann, mit einer Verbindung der Formel XV erhältlich, 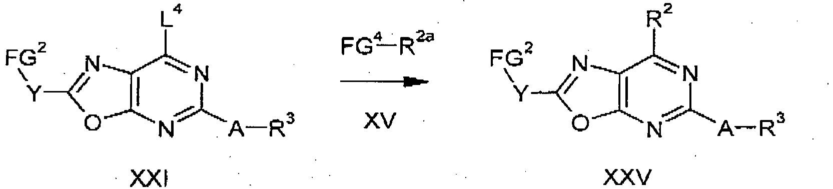 worin die Gruppen A, Y, R² und R³ in den Verbindungen der Formeln XXI und XXV wie in den Verbindungen der Formel I definiert sind und zusätzlich funktionelle Gruppen in geschützter Form oder in Form einer Vorläufergruppe, die später in die endgültige Gruppe umgewandelt wird, vorliegen können. Die Gruppen L⁴, R^{2a}, FG² und FG⁴ in den Verbindungen der Formeln XV, XXI und XXV sind wie oben definiert.

Die Umsetzung von Verbindungen der Formel XXI mit Verbindungen der Fomel XV zu Verbindungen der Formel XXV kann dabei unter Bedingungen erfolgen, wie sie für die Umsetzung von Verbindungen der Formel XIV zu Verbindungen der Formel XVI bereits oben beschrieben wurden.

Weitere Verbindungen der Formel I sind aus geeigneten, nach den oben beschriebenen Verfahren hergestellten Verbindungen durch Funktionalisierung oder Modifizierung von enthaltenen funktionellen Gruppen nach Standardverfahrensweisen erhältlich, beispielsweise durch Veresterung, Amidierung, Hydrolyse, Veretherung, Alkylierung, Acylierung, Sulfonylierung, Reduktion, Oxidation, Umwandlung in Salze u.a. So kann beispielsweise eine Hydroxygruppe, die aus einer Ethergruppe durch Etherspaltung, beispielsweise mit Hilfe von Bortribromid, oder aus einer geschützten Hydroxygruppe durch Entschützung freigesetzt werden kann, zu einem Carbonsäureester oder einem Sulfonsäureester verestert oder verethert werden. Veretherungen von Hydroxygruppen können günstigerweise durch Alkylierung mit der jeweiligen Halogenverbindung, beispielsweise einem Bromid oder Iodid, in Gegenwart einer Base, beispielsweise eines Alkalimetallcarbonats wie Kaliumcarbonat oder Cäsiumcarbonat, in einem inerten Lösungsmittel, beispielsweise einem Amid wie DMF oder NMP oder einem Keton wie Aceton oder Butan-2-on oder mit dem jeweiligen Alkohol unter den oben angesprochenen Bedingungen der Mitsunobu-Reaktion durchgeführt werden. Eine Hydroxygruppe kann durch Behandlung mit einem Halogenierungsmittel in ein Halogenid umgewandelt werden. Ein Halogenatom kann in einer Substitutionsreaktion, bei der es sich auch um eine übergangsmetallkatalysierte Reaktion handeln kann, durch verschiedene Gruppen ersetzt werden. Eine Nitrogruppe kann zu einer Aminogruppe reduziert werden, beispielsweise durch katalytische Hydrierung. Eine Aminogruppe kann unter Standardbedingungen für die Alkylierung, beispielsweise durch Umsetzung mit einer Halogenverbindung oder durch reduktive Aminierung einer Carbonylverbindung, oder für die Acylierung oder Sulfonylierung, beispielsweise durch Umsetzung mit einem reaktiven Carbonsäurederivat wie einem Säurechlorid oder Anhydrid oder einem Sulfonsäurechlorid oder mit einer aktivierten Carbonsäure, die aus der Carbonsäure beispielsweise durch Behandlung mit einem Kupplungsmittel wie N,N'-Carbonyldiimidazol (CDI), einem Carbodiimid wie 1,3-Dicyclohexylcarbodiimid (DCC) oder 1-(3-Dimethylaminopropyl)-3-ethylcarbodiimid-hydrochlorid (EDC), O-(7-Azabenzotriazol-1-yl)-N,N,N',N'-tetramethyluroniumhexafluorophosphat (HATU), O-(Cyano(ethoxycarbonyl)methylenamino)-N,N,N',N'-tetramethyluroniumtetrafluoroborat (TOTU) oder [(Benzotriazol-1-yloxy)dimethylaminomethylen]dimethylammonium-tetrafluoroborat (TBTU) erhältlich ist, modifiziert werden. Eine Carbonsäureestergruppe kann unter sauren oder basischen Bedingungen zu einer Carbonsäure hydrolysiert werden. Eine Carbonsäuregruppe kann wie oben erwähnt aktiviert oder in ein reaktives Derivat umgewandelt und mit einem Alkohol oder einem Amin oder Ammoniak zu einem Ester oder Amid umgesetzt werden. Ein primäres Amid kann zu einem Nitril dehydratisiert werden. Ein Schwefelatom, beispielsweise in einer Alkyl-S-Gruppe oder in einem heterocyclischen Ring, kann mit einem Peroxid wie Wasserstoffperoxid oder einer Persäure zu einer Sulfoxidgruppierung S(O) oder einer Sulfongruppierung S(O)₂ oxidiert werden. Eine Carbonsäuregruppe, eine Carbonsäureestergruppe und eine Ketongruppe können zu einem Alkohol reduziert werden, beispielsweise mit Hilfe eines komplexen Hydrids wie Lithiumaluminiumhydrid, Lithiumborhydrid oder Natriumborhydrid. Eine Verbindung der Formel I oder ein Zwischenprodukt wie eine Verbindung der Formel II, die bzw. das eine Doppelbindung oder eine Dreifachbindung in der Gruppe X enthält, die über eine übergangsmetallkatalysierte Kupplungsreaktion leicht aus einer Verbindung der Formel XIV mit einer Doppel- oder Dreifachbindung in der Gruppe X^{a} und einer Verbindung der Formel XIII wie oben beschrieben erhältlich ist, kann durch Hydrierung in Gegenwart von Hydrierkatalysator wie einem Palladiumkatalysator in eine Verbindung überführt werden, in der X für eine gesättigte Gruppe steht.

Alle bei den oben beschriebenen Synthesen der Verbindungen der Formel I verwendeten Reaktionen sind dem Fachmann an sich gut bekannt und können unter Standardbedingungen gemäß oder in Analogie zu in der Literatur, beispielsweise in Houben-Weyl, Methoden der Organischen Chemie (Methods of Organic Chemistry), Thieme-Verlag, Stuttgart, oder Organic Reactions, John Wiley & Sons, New York, beschriebenen Verfahrensweisen durchgeführt werden. Falls gewünscht, können die erhaltenen Verbindungen der Formel I sowie etwaige Zwischenverbindungen nach herkömmlichen Reinigungsverfahrensweisen gereinigt werden, beispielsweise durch Umkristallisieren oder Chromatographie. Wie bereits erwähnt, können alle bei den oben beschriebenen Synthesen eingesetzten Ausgangsverbindungen und Zwischenprodukte, die eine saure oder basische Gruppe enthalten, auch in Form von Salzen eingesetzt werden und alle Zwischenprodukte und entgültigen Zielverbindungen können auch in Form von Salzen erhalten werden. Wie ebenfalls oben erwähnt, kann es je nach den Umständen des jeweiligen Falls zur Vermeidung eines unerwünschten Verlaufs einer Reaktion oder von Nebenreaktionen im Lauf der Synthese einer Verbindung im allgemeinen erforderlich oder vorteilhaft sein, funktionelle Gruppen durch die Einführung von Schutzgruppen zeitweilig zu blockieren und sie in einer späteren Stufe der Synthese wieder zu entschützen oder funktionelle Gruppen in Form von Vorläufergruppen, die später in die gewünschten funktionellen Gruppen umgewandelt werden, einzuführen. Als Beispiele für Schutzgruppen seien Aminoschutzgruppen genannt, bei denen es sich um Acylgruppen oder Alkyloxycarbonylgruppen, beispielsweise eine tert.-Butyloxycarbonylgruppe (=Boc), die durch Behandung mit Trifluoressigsäure (=TFA) abgespalten werden kann, eine Benzyloxycarbonylgruppe, die durch katalytische Hydrierung abgespalten werden kann, oder eine Fluoren-9-ylmethoxycarbonylgruppe,die durch Behandlung mit Piperidin abgespalten werden kann, handeln kann, und Schutzgruppen von Carbonsäuregruppen, die als Estergruppen, wie tert.-Butylester, die durch Behandlung mit Trifluoressigsäure entschützt werden können, oder Benzylester, die durch katalytische Hydrierung entschützt werden können, geschützt werden können. Als Beispiel für eine Vorläufergruppe sei die Nitrogruppe genannt, die durch Reduktion, beispielsweise durch katalytische Hydrierung, in eine Aminogruppe umgewandelt werden kann. Derartige Synthesestrategien und Schutzgruppen und Vorläufergruppen, die in einem bestimmten Fall geeignet sind, sind dem Fachmann bekannt.

Ein weiterer Gegenstand der vorliegenden Erfindung sind die neuen Ausgangsverbindungen und Zwischenprodukte, die bei der Synthese der Verbindungen der Formel I vorkommen, einschließlich der Verbindungen der Formeln II, III, IV, V, VI, VII, VIII, IX, X, XI, XII, XIII, XIV, XV, XVI, XVII, XVIII, IXX, XX, XXI, XXII, XXIII, XXIV, XXV und XXVI worin Alk, A, X, X^{a}, R¹, R², R^{2a}, R³, FG¹, FG², FG³, FG⁴, L¹, L², L³ und L⁴ wie oben definiert sind, in einer beliebigen ihrer stereoisomeren Formen oder einer Mischung von stereoisomeren Formen in beliebigem Verhältnis und ihre Salze und Solvate derartiger Verbindungen oder derartiger Salze und ihre Verwendung als Zwischenprodukte. Die Erfindung schließt auch alle tautomeren Formen der Zwischenprodukte und Ausgangsverbindungen ein. Alle oben bezüglich der Verbindungen der Formel I angegebenen Erklärungen und Ausführungsformen gelten entsprechend auch für die Zwischenprodukte und Ausgangsverbindungen. Gegenstand der Erfindung sind insbesondere die hier offenbarten neuen spezifischen Ausgangsverbindungen und Zwischenprodukte. Unabhängig davon, ob sie als freie Verbindung und/oder als spezifisches Salz offenbart sind, sind sie sowohl in Form der freien Verbindungen als auch in Form ihrer Salze und im Fall der Offenbarung eines spezifischen Salzes zusätzlich in Form dieses spezifischen Salzes und in Form von Solvaten derartiger Verbindungen oder derartiger Salze Gegenstand der Erfindung.

Die Verbindungen der Formel I, gegebenenfalls in Kombination mit anderen pharmakologisch wirksamen Verbindungen, können Tieren, vorzugsweise Säugetieren einschließlich Menschen, als Pharmazeutika für sich alleine, in Mischungen miteinander oder in Form pharmazeutischer Zusammensetzungen verabreicht werden. Die Verabreichung kann oral, beispielsweise in Form von Tabletten, Filmtabletten, Dragees, Granulaten, Hart- und Weichgelatinekapseln, Lösungen einschließlich wäßriger, alkoholischer und öliger Lösungen, Säften, Tropfen, Sirupen, Emulsionen oder Suspensionen, rektal, beispielsweise in Form von Suppositorien, oder parenteral, beispielsweise in Form von Lösungen zur subkutanen, intramuskulären oder intravenösen Injektion oder Infusion, insbesondere wäßrigen Lösungen, durchgeführt werden. Die Verbindungen der Formel I können des weiteren in Modi der lokalen Arzneistoffzufuhr verwendet werden, beispielsweise in beschichteten Stents zur Verhinderung oder Verringerung der In-Stent-Restenose oder durch lokale Anwendung mit Hilfe eines Katheters. Die geeignete Verabreichungsform hängt u.a. von der zu behandelnden Erkrankung und ihrer Schwere ab.

Die Verabreichung der Verbindungen der Formel I, kann auch topisch erfolgen. Geeignete pharmazeutische Zusammensetzungen für die topische Anwendung auf der Haut liegen als Salbe, Creme, Lotion, Paste, Gel, Hydrogel, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im Allgemeinen in einer Konzentration von 0,0001 bis 15 Gew.-% der Zusammensetzung vorhanden, beispielsweise von 0,0005 bis 2%.
In einer Ausführungsform liegt die topische Zubereitung als Gel vor.

In einer weiteren Ausführungsform liegt die topische Formulierung als Hydrogel vor.

Unter einem Hydrogel wird ein Wasser enthaltendes, aber wasserunlösliches Polymer, dessen Moleküle chemisch, z. B. durch kovalente oder ionische Bindungen, oder physikalisch, z. B. durch Verschlaufen der Polymerketten, zu einem dreidimensionalen Netzwerk verknüpft sind verstanden. Durch eingebaute hydrophile Polymerkomponenten quellen sie in Wasser unter beträchtlicher Volumenzunahme, ohne aber ihren stofflichen Zusammenhalt zu verlieren. Ein Hydrogel besteht beispielsweise aus einem hydrophilen Lösungsmittel (z.B. Wasser), einem Feuchthaltemittel (z.B. Glycerol) und einem Gelbildner (z.B. Croscarmellose-Natrium).

Die folgenden Beispiele zeigen geeignete Gelzubereitungen:

**Zubereitungsbeispiel 1**

| | |
|---|---|
| Verbindung aus Beispiel 1 | 0.0004% |
| Glycerol 85% | 10% |
| Methylparaben | 0.2% |
| Propylparaben | 0.03% |
| Croscarmellose-Natrium | 4% |
| HCl / NaOH | qs (zum Einstellen auf pH 7.5) |
| Wasser | ad 100% |

**Zubereitungsbeispiel 2**

| | |
|---|---|
| Verbindung aus Beispiel 1 | 0.04% |
| Glycerol 85% | 10% |
| Methylparaben | 0.2% |
| Propylparaben | 0.03% |
| Croscarmellose-Natrium | 4% |
| HCl / NaOH | qs (zum Einstellen auf pH 7.5) |
| Wasser | ad 100% |

**Zubereitungsbeispiel 3**

| | |
|---|---|
| Verbindung aus Beispiel 1 | 0.0004% |
| PEG400 | 10% |
| Methylparaben | 0.2% |
| Propylparaben | 0.03% |
| Croscarmellose-Natrium | 4% |
| HCl / NaOH | qs (zum Einstellen auf pH 7.5) |
| Wasser | ad 100% |

**Zubereitungsbeispiel 4**

| | |
|---|---|
| Verbindung aus Beispiel 1 | 0.04% |
| PEG400 | 10% |
| Methylparaben | 0.2% |
| Propylparaben | 0.03% |
| Croscarmellose-Natrium | 4% |
| HCl / NaOH | qs (zum Einstellen auf pH 7.5) |
| Wasser | ad 100% |

Die Hydrogele sind Zubereitung zur dermalen Anwendung. Die Hydrogele können auf offene Wundareale aufgetragen werden. Die Hydrogele enthalten den Arzneistoff in gelöster Form, wodurch eine schnelle Haut- und Gewebepentration gewährleistet ist. Durch einen aseptischen Herstellprozess wird gewährleistet, dass durch die Applikation des Arzneimittels keine zusätzlichen mikrobiologischen Verunreinigungen in die Wunde gelangen. In einer Ausführungsform werden zusätzlich in das Hydrogel Konservierungsmittel (Methyl- und Propylparabene) eingearbeitet, um die Keimbelastung gering zu halten.

In einer Ausführungsform enthält das Hydrogel die Verbindungen der Formel I, in Konzentrationen von 0.04 - 0.0004% (m/m).

Das aseptische Hydrogel wird in geigneten sterilen Behältern gelagert. In einer Ausführungsform wird das Hydrogel in sterilen Behältern aus Polypropylen gelagert.

Die Menge einer Verbindung der Formel I und/oder ihrer physiologisch akzeptablen Salze und/oder Solvate in den pharmazeutischen Zusammensetzungen liegt normalerweise im Bereich von etwa 0,2 bis etwa 800 mg, beispielsweise von etwa 0,5 bis etwa 500 mg, beispielsweise von etwa 1 bis etwa 200 mg, pro Einheitsdosis, kann aber je nach Art der pharmazeutischen Zusammensetzung auch höher sein. Die pharmazeutischen Zusammensetzungen enthalten in der Regel etwa 0,5 bis etwa 90 Gew.-% der Verbindung der Formel I und/oder ihrer physiologisch akzeptablen Salze und/oder Solvate. Die pharmazeutischen Zusammensetzungen können auf an sich bekannte Art und Weise hergestellt werden. Hierzu bringt man eine oder mehrere Verbindungen der Formel I und/oder ihre physiologisch akzeptablen Salze und/oder Solvate zusammen mit einer oder mehreren festen oder flüssigen pharmazeutischen Trägersubstanzen oder Vehikeln und/oder Additiven oder Hilfssubstanzen und dann, wenn ein Kombinationsmedikament gewünscht ist, anderen pharmakologisch wirksamen Verbindungen mit therapeutischer oder prophylaktischer Wirkung in eine für die Verabreichung und Dosierung geeignete Form, die dann in der Human- oder Tiermedizin verwendet werden kann. Als Trägersubstanzen und Additive können geeignete organische und anorganische Substanzen verwendet werden, die mit den Verbindungen der Formel I oder ihren physiologisch akzeptablen Salzen oder Solvaten nicht in unerwünschter Weise reagieren. Als Beispiele für Additivtypen, die in den pharmazeutischen Zusammensetzungen und Medikamenten enthalten sein können, seien Gleitmittel, Konservierungsstoffe, Verdicker, Stabilisatoren, Sprengmittel, Netzmittel, Mittel zur Erzielung eines Depoteffekts, Emulgatoren, Salze, beispielsweise zur Beeinflussung des osmotischen Drucks, Puffersubstanzen, Farbmittel, Geschmacksstoffe und aromatische Substanzen genannt. Beispiele für Trägersubstanzen und Additive sind Wasser, physiologische Natriumchloridlösung, Pflanzenöle, Wachse, Alkohole wie Ethanol, Isopropanol, 1,2-Propandiol, Benzylalkohole oder Glycerin, Polyole, Mannit, Polyethylenglykole, Polypropylenglykole, Glycerintriacetat, Polyvinylpyrrolidon, Gelatine, Cellulose, Kohlenhydrate wie Lactose, Glucose, Saccharose oder Stärke wie Maisstärke, Stearinsäure und Stearinsäuresalze wie Magnesiumstearat, Talk, Lanolin, Vaseline oder Mischungen davon, beispielsweise Mischungen von Wasser mit einem oder mehreren organischen Lösungsmitteln wie Mischungen von Wasser mit Alkoholen. Man kann die Verbindungen der Formel I und ihre physiologisch akzeptablen Salze und Solvate auch lyophilisieren und die erhaltenen Lyophilisate beispielsweise zur Herstellung von Injektionszusammensetzungen verwenden.

Die Dosierung einer zu verabreichenden Verbindung der Formel I und/oder eines physiologisch akzeptablen Salzes und/oder Solvats davon hängt vom Einzelfall ab und ist wie üblich zur Erzielung einer optimalen Wirkung vom Arzt nach den üblichen Regeln und Verfahrensweisen den individuellen Gegebenheiten anzupassen. So hängt sie beispielsweise ab von der Art und Schwere der zu behandelnden Störung, von Geschlecht, Alter, Gewicht und individueller Ansprechbarkeit des zu behandelnden Menschen oder Tiers, von der Effizienz und Wirkdauer der verwendeten Verbindung, davon, ob die Behandlung für die Therapie einer akuten oder chronischen Erkrankung oder prophylaktisch ist, oder davon, ob neben einer Verbindung der Formel I weitere Wirkstoffe verabreicht werden. Im allgemeinen ist eine Tagesdosis von beispielsweise etwa 0,01 mg/kg bis etwa 100 mg/kg oder von etwa 0,1 mg/kg bis etwa 10 mg/kg oder von etwa 0,3 mg/kg bis etwa 5 mg/kg (jeweils mg pro kg Körpergewicht) zur Verabreichung an einen 75 kg schweren Erwachsenen zur Erzielung der gewünschten Ergebnisse angemessen. Die Tagesdosis kann dabei als Einzeldosis verabreicht oder, insbesondere bei Verabreichung größerer Mengen, in mehrere, beispielsweise zwei, drei oder vier, Einzeldosen aufgeteilt werden. Die Verabreichung kann auch kontinuierlich durchgeführt werden, beispielsweise durch kontinuierliche Infusion oder Injektion. Im Einzelfall kann es je nach dem individuellen Verhalten erforderlich sein, von den angegebenen Dosierungen nach oben oder nach unten abzuweichen.

Die folgenden Beispiele erläutern die Erfindung.

Wenn Beispielsverbindungen mit einer basischen Gruppe durch präparative Hochdruck-Flüssigkeitschromatographie (HPLC) an Umkehrphasen-Säulenmaterial (RP-Säulenmaterial) gereinigt wurden und es sich bei dem Elutionsmittel wie üblich um eine Gradientenmischung von Wasser und Acetonitril mit Trifluoressigsäure (TFA) handelte, wurden sie je nach den Einzelheiten der Aufarbeitung wie Verdampfungs- oder Lyophilisierungsbedingungen zum Teil in Form ihres Säureadditionssalzes mit Trifluoressigsäure erhalten. In den Namen der Beispielverbindungen und ihren Strukturformeln ist jegliche derartige enthaltene Trifluoressigsäure nicht angegeben.

Die hergestellten Verbindungen wurden im allgemeinen durch spektroskopische Daten und chromatografische Daten, insbesondere Massenspektrum (MS) und HPLC-Retentionszeiten (Rt; in min), die durch kombinierte analytische HPLC/MS-Charakterisierung (LC/MS) erhalten wurden, und/oder NMR-Spektren (NMR=kernmagnetische Resonanz), charakterisiert. Bei der NMR-Charakterisierung sind die chemische Verschiebung δ (in ppm), die Zahl der Wasserstoffatome und die Multiplizität (s = Singulett, d = Dublett, dd = doppeltes Dublett, t = Triplett, dt = doppeltes Triplett, q = Quartett, m = Multiplett; br = breit) der Signale angegeben. Bei der MS-Charakterisierung ist im allgemeinen die Massenzahl (m/z) des Peaks des Molekülions M, z.B. M⁺, oder eines verwandten Ions wie des Ions M+1, z.B. [M+1]⁺, d.h. des protonierten Molekülions [M+H]⁺, das je nach der verwendeten Ionisierungsmethode gebildet wurde, angegeben. Bei der Ionisierungsmethode handelte es sich im allgemeinen um Elektrospray-lonisierung (ESI). Es wurden die folgenden LC/MS-Bedingungen verwendet.

### Methode LC1

Säule: Phenomenex, 4 µM, 10 x 2 mm, 1,7 µm; Durchfluß: 1,1 ml/min; Elutionsmittel A: Wasser + 0,05% Trifluoressigsäure; Elutionsmittel B: Acetonitril; Gradient: von 93% A + 7% B bis 5% A + 95% B in 1,2 min, dann 5% A + 95% B für 0,2 min; MS-Ionisationsmethode: ESI⁺

### Methode LC2

Säule: UPLC BEH C18, 50 x 2,1 mm, 1,7 µm; Durchfluß: 0,9 ml/min; Elutionsmittel A: Wasser + 0,1 % Ameisensäure; Elutionsmittel B: Acetonitril + 0,08% Ameisensäure; Gradient: von 95% A + 5% B bis 5% A + 95% B in 1,1 min, dann 5% A + 95% B für 0,6 min; MS-Ionisationsmethode: ESI⁺

### Methode LC3

Säule: UPLC BEH C18, 50 x 2,1 mm, 1,7 µm; Durchfluß: 0,9 ml/min; Elutionsmittel A: Wasser + 0,05% Ameisensäure; Elutionsmittel B: Acetonitril + 0,035 % Ameisensäure; Gradient: von 95% A + 5% B bis 5% A + 95% B in 1,1 min, dann 5% A + 95% B für 0,6 min; MS-Ionisationsmethode: ESI⁺

### Methode LC4

Säule: Phenomenex, 4 µM, 10 x 2 mm, 1,7 µm; Durchfluß: 1,1 ml/min; Elutionsmittel A: Wasser + 0,05% Trifluoressigsäure; Elutionsmittel B: Acetonitril; Gradient: von 80% A + 20% B bis 5% A + 95% B in 0,8 min, dann 5% A + 95% B für 0,6 min; MS-Ionisationsmethode: ESI⁺

### Beispiel 1

### {4-[5-(2,5-Difluoro-phenoxy)=7-propyl-okazolo[5,4-d]pyrimidin-2-yl]-2,6-dimethyl-phenoxy}-essigsäure

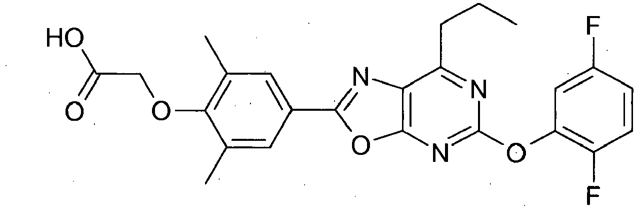

(a) 2-(4-Methoxy-3,5-dimethyl-benzoylamino)-malonsäure diethyl ester 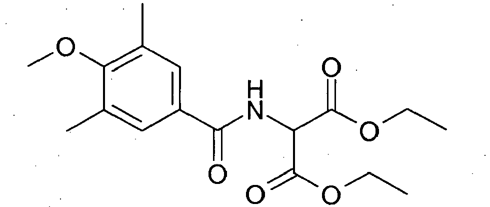 58,4 g Aminomalonsäurediethylester-Hydrochlorid wurden in 300 ml Dichlormethan suspendiert und unter Eiskühlung mit 115 ml Triethylamin versetzt. Bei 0°C wurden dann 54,8 g 4-Methoxy-3,5-dimethylbenzoylchlorid in 250 ml Dichlormethan zugetropft. Nach 2 Stunden bei 0°C wurden 100 ml Wasser zugetropft, die wässrige Phase abgetrennt und mit 200 ml Dichlormethan extrahiert. Die vereinigten organischen Phasen wurden erst mit 2M wässriger Salzsäure, dann mit Wasser ausgeschüttelt, über Natriumsulfat getrocknet, filtriert und eingeengt. Der Rückstand wurde mit Methyl-tert-Butylether verrieben, abgesaugt und im Vakuum getrocknet. Man erhielt 89,3 g (96 %) der Titelverbindung, die ohne weitere Aufreinigung weiter umgesetzt wurde.
   LC/MS (Methode LC1): Rt = 0,94 min; m/z = 338,10 [M+H]⁺
(b) Natrium 4,6-dihydroxy-5-(4-methoxy-3,5-dimethyl-benzoylamino)-pyrimidin-2-thiolat 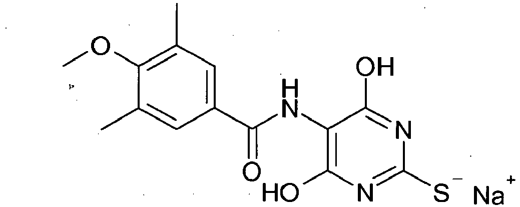 20,6 g Thioharnstoff wurden in 900 ml trockenem Ethanol suspendiert und 75 ml Natriummethylat-Lösung (30%ig in Methanol) zugegeben. Nach 15 Min. wurden 91.0 g 2-(4-Methoxy-3,5-dimethyl-benzoylamino)-malonsäure diethyl ester portionsweise eingetragen. Es entstand zunächst eine klare Lösung, aus der bald ein blassgelber Niederschlag ausfiel. Anschließend wurde der Ansatz 3 Stunden bei 60°C gerührt. Bei Raumtemperatur wurde der ausgefallene Feststoff abgesaugt und mit ca. 100 ml Ethanol, dann mit Diethylether gewaschen und im Vakuum getrocknet. Man erhielt 78.3 g (84 %) der Titelverbindung, die ohne weitere Aufreinigung weiter umgesetzt wurde.
   LC/MS (Methode LC1): Rt = 0,41 min; m/z = 322,05 [M-Na+2H]⁺
(c) N-(4,6-Dihydroxy-2-methylsulfanyl-pyrimidin-5-yl)-4-methoxy-3,5-dimethyl-benzamid 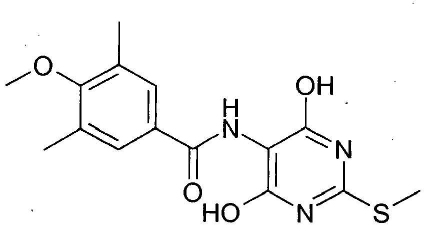 22,78 g Natriumhydroxid wurden in 735 ml Wasser vorgelegt auf 0°C gekühlt, dann wurden 78.21 g Natrium 4,6-dihydroxy-5-(4-methoxy-3,5-dimethyl-benzoylamino)-pyrimidin-2-thiolat und 365 ml N-Methyl-2-pyrrolidon zugegeben und nach 30 Min. bei 0 °C 14.2 ml lodmethan zugetropft. Nach 2 Stunden bei 0°C wurde der Reaktionsansatz vorsichtig mit konzentrierter wäßriger Salzsäure auf pH 2 gebracht. Es wurden ca. 200 ml Wasser zugegeben und der ausgefällte Feststoff wurde abgesaugt, mit Wasser neutral gewaschen und im Vakuum getrocknet. Man erhielt 50.89 g (67 %) der Titelverbindung, die ohne weitere Aufreinigung weiter umgesetzt wurde.
   LC/MS (Methode LC1): Rt = 0,66 min; m/z = 336,05 [M+H]⁺
(d) 7-Chloro-2-(4-methoxy-3,5-dimethyl-phenyl)-5-methylsulfanyl-oxazolo[5,4-d]pyrimidin 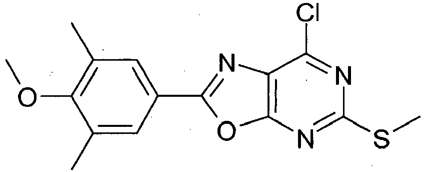 45,95 g N-(4,6-Dihydroxy-2-methylsulfanyl-pyrimidin-5-yl)-4-methoxy-3,5-dimethyl-benzamid wurden bei Raumtemperatur unter Rühren in 125 ml Phosphoroxychlorid suspendiert und für 36 Stunden auf 70°C erwärmt. Nach Abkühlen wurde das Reaktionsgemisch durch eine Glasfritte gesaugt und ein gelber Feststoff erhalten, der unter Rühren in gesättigte wässrige Natriumhydrogensulfatlösung eingetragen und für 10 min gerührt wurde. Der Feststoff wurde dann abgesaugt, neutral gewaschen und getrocknet. Man erhielt so 10,47 g (22 %) der Titelverbindung, die ohne weitere Aufreinigung weiter umgesetzt wurde.
   LC/MS (Methode LC4): Rt = 0,88 min; m/z = 336,00 [M+H]⁺
(e) 4-(7-Chloro-5-methylsulfanyl-oxazolo[5,4-d]pyrimidin-2-yl)-2,6-dimethyl-phenol 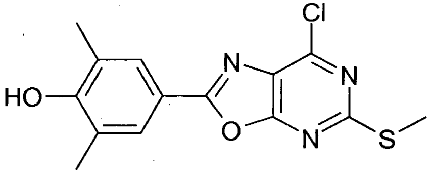 Zu einer Lösung von 6,72 g 7-Chloro-2-(4-methoxy-3,5-dimethyl-phenyl)-5-methyl-sulfanyl-oxazolo[5,4-d]pyrimidin in 140 ml Dichlormethan wurden bei -20 °C 5,30 g Bortribromid unter Rühren zugetropft. Man ließ den Reaktionsansatz auf Raumtemperatur kommen und rührte 2 Stunden. Dann wurden erneut unter Eiskühlung 1,33 g Bortribromid zugetropft. Nach weiteren 2 Stunden wurde der Ansatz erneut auf -20°C abgekühlt und vorsichtig ges. wässrige Natriumhydrogencarbonat-Lösung zugetropft. Bei Raumtemperatur wurden die Phasen getrennt. Die organische Phase wurde mit Wasser gewaschen, über Natriumsulfat getrocknet, filtriert und im Vakuum eingeengt. Der Rückstand wurde mit Diisopropylether verrieben, abgesaugt und an der Luft getrocknet. Man erhielt 6,20 g (96 %) der Titelverbindung, die ohne weitere Aufreinigung weiter umgesetzt wurde.
   LC/MS (Methode LC1): Rt = 1,12 min; m/z = 322,10 [M+H]⁺
(f) [4-(7-Chloro-5-methylsulfanyl-oxazolo[5,4-d]pyrimidin-2-yl)-2,6-dimethyl-phenoxy]-essigsäure tert-butyl ester 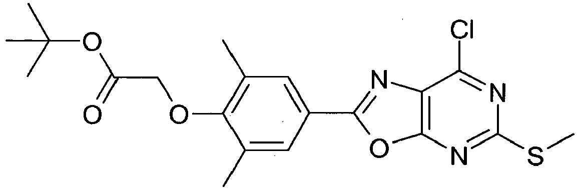 Eine Lösung von 4,34 g 4-(7-Chloro-5-methylsulfanyl-oxazolo[5,4-d]pyrimidin-2-yl)-2,6-dimethyl-phenol in 45 ml Dimethylformamid wurde mit 7,46 g Kaliumcarbonat versetzt, und dann wurden 2,90 g Bromessigsäure-tert.-butylester zugegeben. Die Mischung wurde 1 h bei 60 °C gerührt. Dann wurde der abgekühlte Reaktionsansatz auf Eiswasser gegeben und der ausgefallene Feststoff wurde abgesaugt und mit Wasser gewaschen. Der Feststoff wurde in Dichlormethan aufgenommen, über Natriumsulfat getrocknet, mit Aktivkohle versetzt, filtriert und im Vakuum eingeengt. Man erhielt 4,20 g (71 %) der Titelverbindung, die ohne weitere Aufreinigung weiter umgesetzt wurde.
   LC/MS (Methode LC1): Rt = 1,28 min; m/z = 436,10 [M+H]⁺
(g) [2,6-Dimethyl-4-(5-methylsulfanyl-7-propyl-oxazolo[5,4-d]pyrimidin-2-yl)-phenoxy]-essigsäure tert-butyl ester 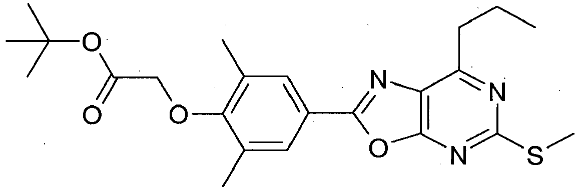 Zu einer Lösung von 1.09 g [4-(7-Chloro-5-methylsulfanyl-oxazolo[5,4-d]pyrimidin-2-yl)-2,6-dimethyl-phenoxy]-essigsäure tert-butyl ester und 44 mg Eisen(III)acetylacetonat in 100 ml trockenem Tetrahydrofuran wurden unter Argon und bei -78 °C 1.38 ml einer 2.0 M Lösung von n-Propylmagnesiumbromid in Tetrahydrofuran langsam zugetropft. Man ließ den Ansatz innerhalb einer Stunde auf Raumtemperatur kommen und rührte zwei weitere Stunden. Danach wurde Diethylether zu der Reaktionsmischung gegeben und unter Eiskühlung wurde er mit 10 %iger wässriger Natriumhydrogensulfat-Lösung versetzt. Die Phasen wurden getrennt, und die wässrige Phase wurde zweimal mit Diethylether extrahiert. Die vereinigten organischen Phasen wurden mit Wasser gewaschen, über Natriumsulfat getrockent, filtriert und im Vakuum eingeengt. Aus dem Rohprodukt wurden nach Chromatogarphie an Kieselgel (Heptan/Ethylacetat) 0.11 g (10%) der Titelverbindung isoliert.
   LC/MS (Methode LC2): Rt = 1,55 min; m/z = 444,20 [M+H]⁺
(h) [4-(5-Methansulfonyl-7-propyl-oxazolo[5,4-d]pyrimidin-2-yl)-2,6-dimethyl-phenoxy]-essigsäure tert-butyl ester 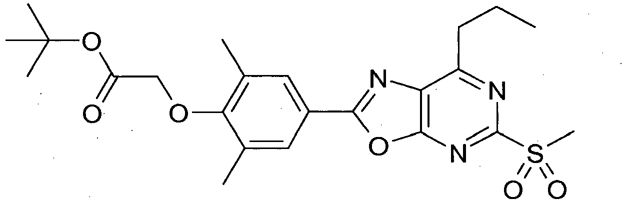 105 mg [2,6-Dimethyl-4-(5-methyl.sulfanyl-7-propyl-oxazolo[5,4-d]pyrimidin-2-yl)-phenoxy]-essigsäure tert-butyl ester wurden in 1,2 ml Dichlormethan vorgelegt und bei Raumtemperatur wurden 128 mg 3-Chlorperbenzoesäure zugegeben. Nach 1 h wurde die Reaktion mit Dichlormethan verdünnt und zweimal mit 1 M wässriger Natronlauge, dann mit ges. wässriger Natriumsulfit-Lösung und schließlich mit Wasser gewaschen. Die org. Phase wurde über Natriumsulfat getrocknet, filtriert und eingeengt, und man erhielt 107 mg (98 %) der Titelverbindung, die ohne weitere Aufreinigung weiter umgesetzt wurde.
   LC/MS (Methode LC2): Rt = 1,41 min; m/z = 476,30 [M+H]⁺
(i) {4-[5-(2,5-Difluoro-phenoxy)-7-propyl-oxazolo[5,4-d]pyrimidin-2-yl]-2,6-dimethyl-phenoxy}-essigsäure tert-butyl ester 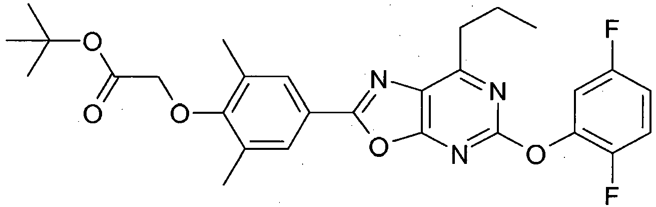 Eine Lösung von 107 mg [4-(5-Methansulfonyl-7-propyl-oxazolo[5,4-d]pyrimidin-2-yl)-2,6-dimethyl-phenoxy]-essigsäure tert-butyl ester in 1,1 ml NMP wurde mit 68 mg Kaliumcarbonat versetzt, und dann wurden 32 mg 2,5-Difluorphenol zugegeben. Die Reaktionsmischung wurde in einem Mikrowellensynthesizer für 5 min auf 110 °C erhitzt, dann wurde sie abgekühlt und auf Eis gegossen. Die ausgefallene Verbindung wurde in Ethylacetat aufgenommen, mit Wasser neutral gewaschen, über Natriumsulfat getrocknet, filtiret und engeengt. Das Rohprodukt wurde über Kieselgel (Heptan/Ethyl-acetat) chromatographiert und man erhielt 45 mg (38 %) der Titelverbindung.
   LC/MS (Methode LC2): Rt = 1,53 min; m/z = 526,30 [M+H]⁺
(j) {4-[5-(2,5-Difluoro-phenoxy)-7-propyl-oxazolo[5,4-d]pyrimidin-2-yl]-2,6-dimethyl-phenoxy}- essigsäure 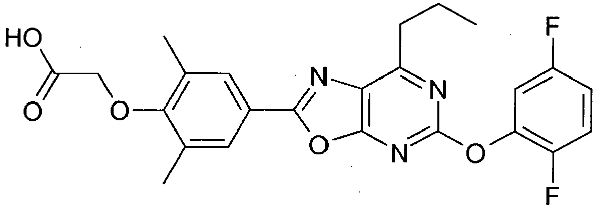 Eine Lösung von 39 mg {4-[5-(2,5-Difluoro-phenoxy)-7-propyl-oxazolo[5,4-d]pyrimidin-2-yl]-2,6-dimethyl-phenoxy}-essigsäure tert-butyl ester in 0,4 ml Dichlormethan wurde mit 55 µl Trifluoressigsäure versetzt und 16 h bei Raumtemperatur gerührt. Dann wurde der Ansatz im Vakuum eingeengt, der Rückstand mit Diisopropylether verrieben, abgesaugt und getrocknet. Man erhielt 21 mg (60 %) der Titelverbindung.
   LC/MS (Methode LC2): Rt = 1,38 min; m/z = 470,20 [M+H]⁺

### Beispiel 2

### {2,6-Dimethyl-4-[7-propyl-5-(3,3,3-trifluoro-propoxy)-oxazolo[5,4-d]pyrimidin-2-yl]-phenoxy}-essigsäure

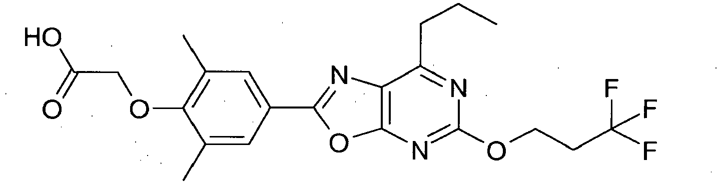

(a) {2,6-Dimethyl-4-[7-propyl-5-(3,3,3-trifluoro-propoxy)-oxazolo[5,4-d]pyrimidin-2-yl]-phenoxy}-essigsäure tert-butyl ester 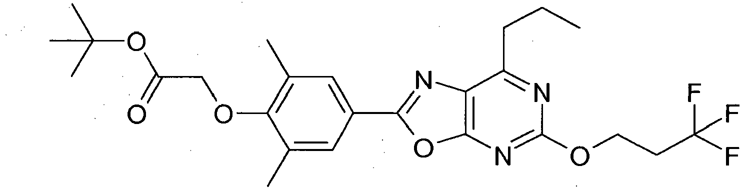 Zu 46 mg Natriumhydrid (60 %ig in Mineralöl) in 1,5 ml trockenem N,N-Dimethylformamid wurden 116 mg 3,3,3-Trifluorpropan-1-ol zugegeben. Nach 5 min wurden 111 mg [4-(5-Methansulfonyl-7-propyl-oxazolo[5,4-d]pyrimidin-2-yl)-2,6-dimethyl-phenoxy]-essigsäure tert-butyl ester zugesetzt und die Reaktion zunächst für 2 h bei Raumtemperatur und anschließend für 15 min bei 80°C gerührt. Zur Aufarbeitung wurde der Ansatz mit gesättigter wässriger Zitronensäure-Lösung versetzt und dreimal mit Ethylacetat extrahiert. Die vereinigten organischen Phasen wurden mit Wasser gewaschen, über Natriumsulfat getrocknet, filtriert und im Vakuum eingeengt. Nach Aufreinigung mittels präparativer HPLC erhielt man 12 mg (10 %) der Titelverbindung.
   LC/MS (Methode LC4): Rt = 0,93 min; m/z = 510.25 [M+H]⁺
(b) {2,6-Dimethyl-4-[7-propyl-5-(3,3,3-trifluoro-propoxy)-oxazolo[5,4-d]pyrimidin-2-yl]-phenoxy}-essigsäure 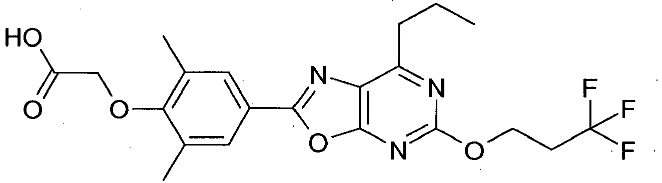 In Analogie zu Beispiel 1 (j) erhielt man durch Umsetzen von 12 mg {2,6-Dimethyl-4-[7-propyl-5-(3,3,3-trifluoro-propoxy)-oxazolo[5,4-d]pyrimidin-2-yl]-phenoxy}-essigsäure tert-butyl mit Trifluoressigsäure 11 mg (83 %) der Titelverbindung.
   LC/MS (Methode LC2): Rt = 1,36 min; m/z = 454,10 [M+H]⁺

### Beispiel 3

### {4-[5-(2-Fluoro-phenoxy)-7-isobutyl-oxazolo[5,4-d]pyrimidin-2-yl]-2,6-dimethyl-phenoxy}-essigsäure

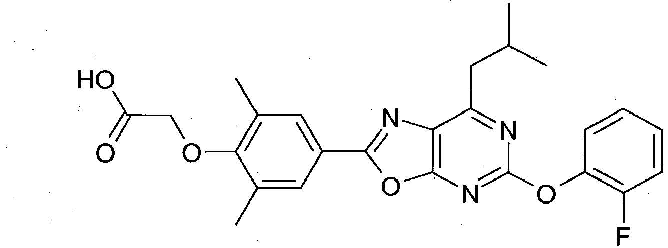

(a) [4-(7-Isobutyl-5-methylsulfanyl-oxazolo[5,4-d]pyrimidin-2-yl)-2,6-dimethyl-phenoxy]-essigsäure tert-butyl ester 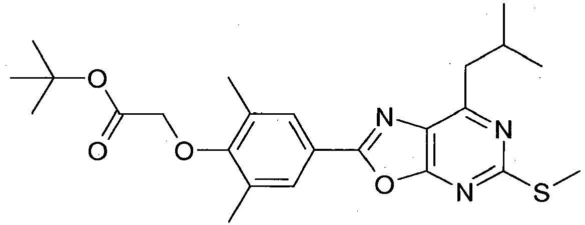 In Analogie zu Beispiel 1 (g) erhielt man aus der Umsetzung von 1.50 g [4-(7-Chloro-5-methylsulfanyl-oxazolo[5,4-d]pyrimidin-2-yl)-2,6-dimethyl-phenoxy]-essigsäure tert-butyl ester mit iso-Butylmagnesiumbromid 0,31 g (20%) der Titelverbindung.
   LC/MS (Methode LC2): Rt = 1,44 min; m/z = 458,30 [M+H]⁺
(b) [4-(7-Isobutyl-5-Methansulfonyl-oxazolo[5,4-d]pyrimidin-2-yl)-2,6-dimethyl-phenoxy]-essigsäure tert-butyl ester 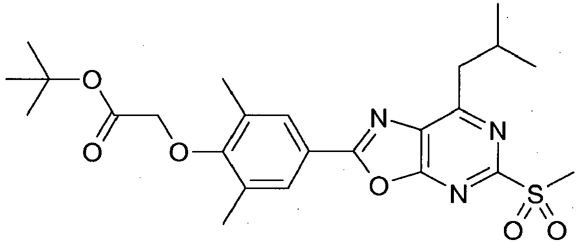 In Analogie zu Beispiel 1 (h) erhielt man aus der Umsetzung von 0,31 g [4-(7-Isobutyl-5-methylsulfanyl-oxazolo[5,4-d]pyrimidin-2-yl)-2,6-dimethyl-phenoxy]-essigsäure tert-butyl ester mit 3-Chlorperbenzoesäure 0,31 g (93%) der Titelverbindung.
   LC/MS (Methode LC2): Rt = 1,43 min; m/z = 490,20 [M+H]⁺
(c) {4-[5-(2-Fluoro-phenoxy)-7-isobutyl-oxazolo[5,4-d]pyrimidin-2-yl]-2,6-dimethyl-phenoxy}-essigsäure tert-butyl ester 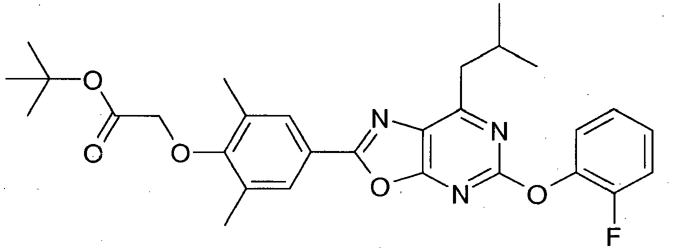 In Analogie zu Beispiel 1 (i) erhielt man aus der Umsetzung von 60 mg [4-(7-Isobutyl-5-Methansulfonyl-oxazolo[5,4-d]pyrimidin-2-yl)-2,6-dimethyl-phenoxy]-essigsäure tert-butyl ester mit 2-Fluorphenol 64 mg (100 %) der Titelverbindung.
   LC/MS (Methode LC4): Rt = 1,01 min; m/z = 522,25 [M+H]⁺
(d) {4-[5-(2-Fluoro-phenoxy)-7-isobutyl-oxazolo[5,4-d]pyrimidin-2-yl]-2,6-dimethyl-phenoxy}-essigsäure 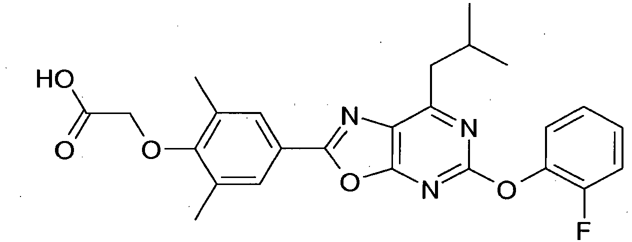 In Analogie zu Beispiel 1 (j) erhielt man aus der Umsetzung von 64 mg {4-[5-(2-Fluoro-phenoxy)-7-isobutyl-oxazolo[5,4-d]pyrimidin-2-yl]-2,6-dimethyl-phenoxy}-essigsäure tert-butyl ester mit Trifluoressigsäure 33 mg (56 %) der Titelverbindung.
   LC/MS (Methode LC2): Rt = 1,40 min; m/z = 466,20 [M+H]⁺

### Beispiel 4

### {4-[7-Isobutyl-5-(3,3,3-trifluoro-propoxy)-oxazolo[5,4-d]pyrimidin-2-yl]-2,6-dimethyl-phenoxy}-essigsäure

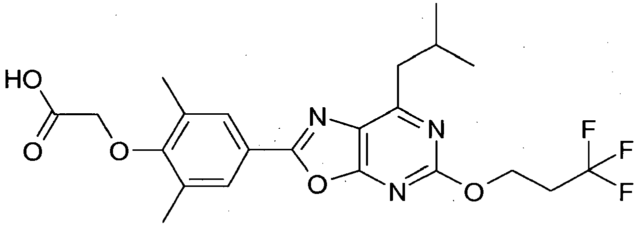

(a) {4-[7-Isobutyl-5-(3,3,3-trifluoro-propoxy)-oxazolo[5,4-d]pyrimidin-2-yl]-2,6-dimethyl-phenoxy}-essigsäure tert-butylester 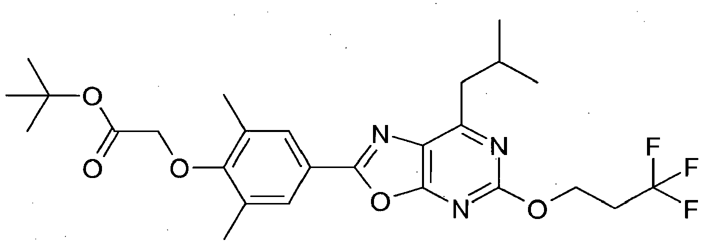 In Analogie zu Beispiel 2 (a) erhielt man aus der Umsetzung von 95 mg [4-(7-Isobutyl-5-Methansulfonyl-oxazolo[5,4-d]pyrimidin-2-yl)-2,6-dimethyl-phenoxy]-essigsäure tert-butyl ester mit 3,3,3-Trifluorpropan-1-ol 10 mg (10 %) der Titelverbindung.
   LC/MS (Methode LC4): Rt = 0,96 min; m/z = 524,25 [M+H]⁺
(b) {4-[7-Isobutyl-5-(3,3,3-trifluoro-propoxy)-oxazolo[5,4-d]pyrimidin-2-yl]-2,6-dimethyl-phenoxy}-essigsäure 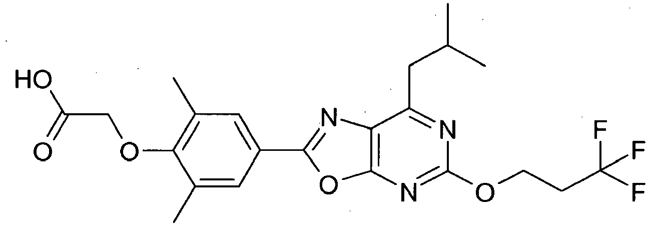 In Analogie zu Beispiel 1 (j) erhielt man aus der Umsetzung von 8 mg {4-[7-Isobutyl-5-(3,3,3-trifluoro-propoxy)-oxazolo[5,4-d]pyrimidin-2-yl]-2,6-dimethyl-phenoxy}-essigsäure tert-butyl ester mit Trifluoressigsäure 4 mg (56 %) der Titelverbindung.
   LC/MS (Methode LC4): Rt = 0,80 min; m/z = 468,15 [M+H]⁺

### Beispiel 5

### {4-[5-(3-Chloro-phenoxy)-7-isobutyl-oxazolo[5,4-d]pyrimidin-2-yl]-2,6-dimethyl-phenoxy}-essigsäure

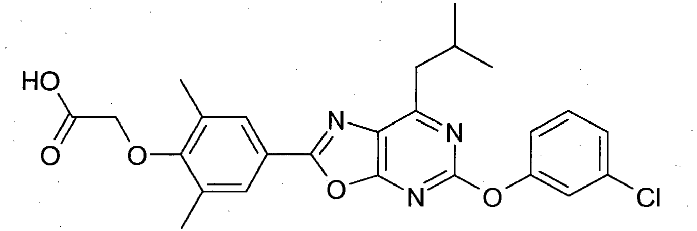

(a) {4-[5-(3-Chloro-phenoxy)-7-isobutyl-oxazolo[5,4-d]pyrimidin-2-yl]-2,6-dimethyl-phenoxy}-essigsäure tert-butyl ester 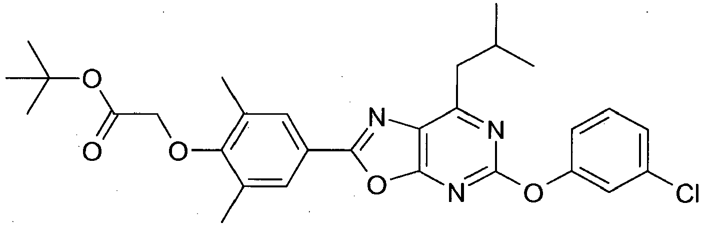 In Analogie zu Beispiel 1 (i) erhielt man aus der Umsetzung von 60 mg [4-(7-Isobutyl-5-Methansulfonyl-oxazolo[5,4-d]pyrimidin-2-yl)-2,6-dimethyl-phenoxy]-essigsäure tert-butyl ester mit 3-Chlorphenol 65 mg (100 %) der Titelverbindung.
   LC/MS (Methode LC4): Rt = 1,04 min; m/z = 538,25 [M+H]⁺
(b) {4-[5-(3-Chloro-phenoxy)-7-isobutyl-oxazolo[5,4-d]pyrimidin-2-yl]-2,6-dimethyl-phenoxy}-essigsäure 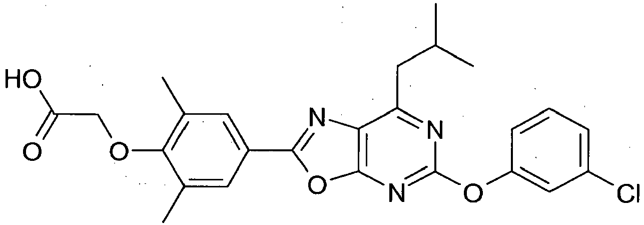 In Analogie zu Beispiel 1 (j) erhielt man aus der Umsetzung von 62 mg {4-[5-(3-Chloro-phenoxy)-7-isobutyl-oxazolo[5,4-d] pyrim id in-2-yl]-2,6-d imethyl-phenoxy}-essigsäure tert-butyl ester mit Trifluoressigsäure 31 mg (45 %) der Titelverbindung.
   LC/MS (Methode LC2): Rt = 1,44 min; m/z = 482,14 [M+H]⁺

### Beispiel 6

### {4-[5-(3-Chloro-phenoxy)-7-propyl-oxazolo[5,4-d]pyrimidin-2-yl]-2,6-dimethyl-phenoxy}-essigsäure

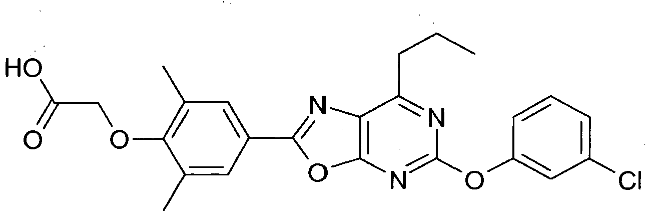

(a) {4-[5-(3-Chloro-phenoxy)-7-propyl-oxazolo[5,4-d]pyrimidin-2-yl]-2,6-dimethyl-phenoxy}-essigsäure tert-butyl ester 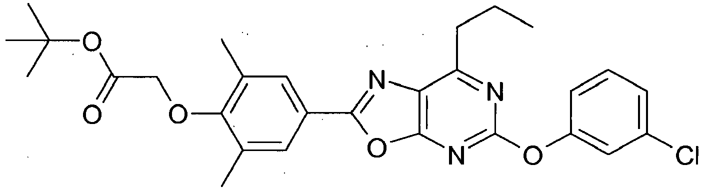 In Analogie zu Beispiel 1 (i) erhielt man aus der Umsetzung von 104 mg [4-(5-Methansulfonyl-7-propyl-oxazolo[5,4-d]pyrimidin-2-yl)-2,6-dimethyl-phenoxy]-essigsäure tert-butyl ester mit 3-Chlorphenol 115 mg (100 %) der Titelverbindung.
   LC/MS (Methode LC2): Rt = 1,42 min; m/z = 524,30 [M+H]⁺
(b) {4-[5-(3-Chloro-phenoxy)-7-propyl-oxazolo[5,4-d]pyrimidin-2-yl]-2,6-dimethyl-phenoxy}-essigsäure 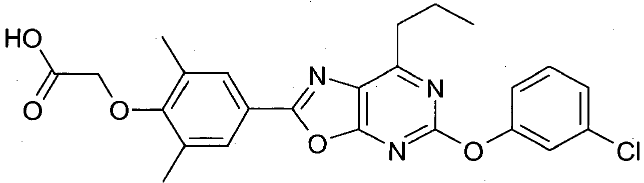 In Analogie zu Beispiel 1 (j) erhielt man aus der Umsetzung von 115 mg {4-[5-(3-Chloro-phenoxy)-7-propyl-oxazolo[5,4-d]pyrimidin-2-yl]-2,6-dimethyl-phenoxy}-essigsäure tert-butyl ester mit Trifluoressigsäure 44 mg (43 %) der Titelverbindung.
   LC/MS (Methode LC2): Rt = 1,29 min; m/z = 468,20 [M+H]⁺

### Beispiel 7

### {4-[5-(4-Chloro-benzyl)-7-isobutyl-oxazolo[5,4-d]pyrimidin-2-yl]-2,6-dimethyl-phenoxy}-essigsäure

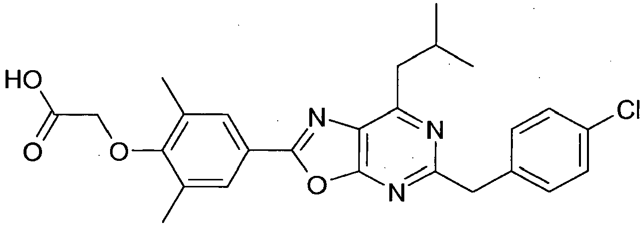

(a) N-[2-(4-Chloro-benzyl)-4,6-dihydroxy-pyrimidin-5-yl]-4-methoxy-3,5-dimethyl-benzamid 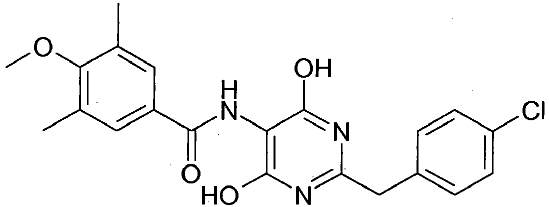 25,6 g 2-(4-Chlorphenyl)-acetamidin-Hydrochlorid wurden in 425 ml trockenem Ethanol gelöst und 70 ml Natriummethylat zugegeben. Nach 15 Min. wurden 42.2 g 2-(4-Methoxy-3,5-dimethyl-benzoylamino)-malonsäure diethylester portionsweise eingetragen, und die Reaktion dann für 2 h bei 80 °C gerührt. Nach Abkühlen auf Raumtemperatur wurde der ausgefallene Feststoff abgesaugt und mit wenig Ethanol und Tetrahydrofuran gewaschen und im Vakuum getrocknet.
   Man erhielt 41.5 g (85 %) der Titelverbindung, die ohne weitere Aufreinigung weiter umgesetzt wurde.
   LC/MS (Methode LC1): Rt = 0,81 min; m/z =414,1 [M+H]⁺
(b) 5-(4-Chloro-benzyl)-2-(4-methoxy-3,5-dimethyl-phenyl)-oxazolo[5,4-d]pyrimidin-7-ol 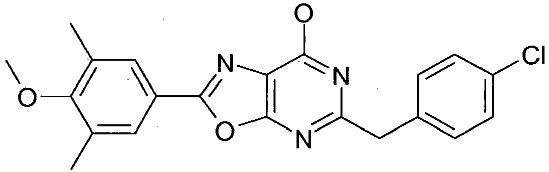 40 g N-[2-(4-Chloro-benzyl)-4,6-dihydroxy-pyrimidin-5-yl]-4-methoxy-3,5-dimethyl-benzamid und 150 ml Phosphoroxychlorid wurden für 1,5 h auf 70 °C erhitzt. Dann ließ man die Mischung abkühlen. Der entstandene Feststoff wurde abgesaugt, mit Diethylether gewaschen und getrocknet. Man erhielt 21,1g (55%) der Titelverbindung.
   LC/MS (Methode LC2): Rt = 1,24 min; m/z = 396,0 [M+H]⁺
(c) 7-Chloro-5-(4-chloro-benzyl)-2-(4-methoxy-3,5-dimethyl-phenyl)-oxazolo[5,4-d]pyrimidin 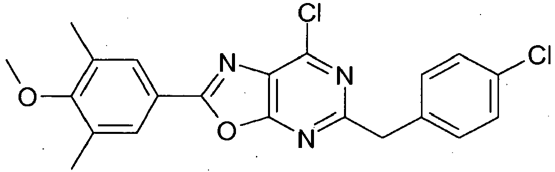 10 g 5-(4-Chloro-benzyl)-2-(4-methoxy-3,5-dimethyl-phenyl)-oxazolo[5,4-d]pyrimidin-7-ol und 50 ml Phsophoroxychlorid wurden für 5 h auf 90 °C erhitzt. Dann ließ man die Mischung abkühlen. Der entstandene Feststoff wurde abgesaugt, mit Diethylether gewaschen und getrocknet. Man erhielt 10,4 g (99%) der Titelverbindung.
   LC/MS (Methode LC2): Rt = 1,09 min; m/z = 414,1 [M+H]⁺
(d) 5-(4-Chloro-benzyl)-7-isobutyl-2-(4-methoxy-3,5-dimethyl-phenyl)-oxazolo[5,4-d]pyrimidin 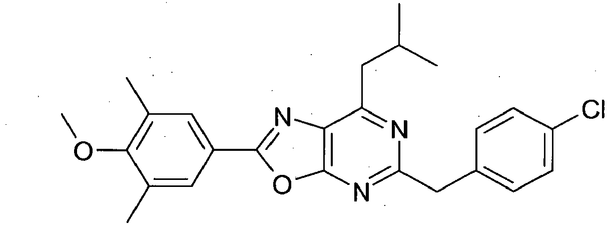 Zu einer entgasten Lösung von 1.50 g 7-Chloro-5-(4-chloro-benzyl)-2-(4-methoxy-3,5-dimethyl-phenyl)-oxazolo[5,4-d]pyrimidin und 65 mg Eisen(III)acetylacetonat in 20 ml trockenem Tetrahydrofuran und 2 ml N-Methylpyrolidon wurden unter Argon und bei 0 °C 2.0 ml einer 2.0 M Lösung von iso-Butylmagnesiumbromid in Tetrahydrofuran langsam zugetropft. Nach 15 min bei 0 °C wurde Diethylether zu der Reaktionsmischung gegeben und unter Eiskühlung wurde er mit 10 %iger wässriger Zitronensäure-Lösung versetzt. Die Phasen wurden getrennt, und die wässrige Phase wurde zweimal mit Diethylether extrahiert. Die vereinigten organischen Phasen wurden mit Wasser gewaschen, über Natriumsulfat getrocknet, filtriert und im Vakuum eingeengt. Aus dem Rohprodukt wurden nach Umfällung aus Acetonitril 0.80 g (51%) der Titelverbindung isoliert.
   LC/MS (Methode LC2): Rt = 1,60 min; m/z = 436,17 [M+H]⁺
(e) 4-[5-(4-Chloro-benzyl)-7-isobutyl-oxazolo[5,4-d]pyrimidin-2-yl]-2,6-dimethyl-phenol 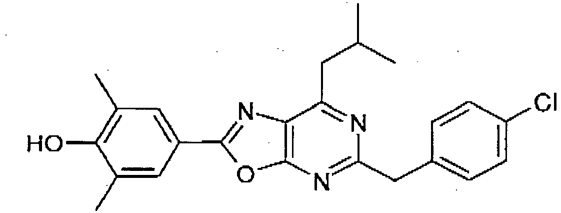 In Analogie zu Beispiel 1 (e) erhielt man aus der Umsetzung von 850 mg 5-(4-Chloro-benzyl)-7-isobutyl-2-(4-methoxy-3,5-dimethyl-phenyl)-oxazolo[5,4-d]pyrimidin mit Bortribromid 690 mg (84 %) der Titelverbindung.
   LC/MS (Methode LC2): Rt = 1,51 min; m/z = 422, 16 [M+H]⁺
(f) {4-[5-(4-Chloro-benzyl)-7-isobutyl-oxazolo[5,4-d]pyrimidin-2-yl]-2,6-dimethyl-phenoxy}-essigsäure tertbutylester 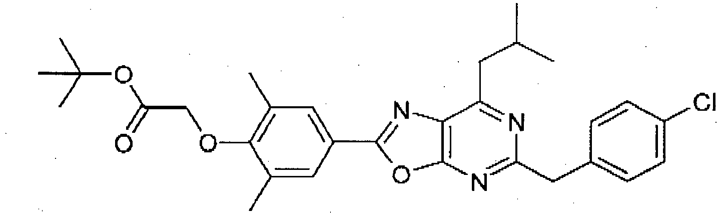 In Analogie zu Beispiel 1 (f) erhielt man aus der Umsetzung von 684 mg 4-[5-(4-Chloro-benzyl)-7-isobutyl-oxazolo[5,4-d]pyrimidin-2-yl]-2,6-dimethyl-phenol mit Bromessigsäure tert-Butylester 800 mg (92 %) der Titelverbindung.
   LC/MS (Methode LC2): Rt = 1,62 min; m/z = 536,22 [M+H]⁺
(g) {4-[5-(4-Chloro-benzyl)-7-isobutyl-oxazolo[5,4-d]pyrimidin-2-yl]-2,6-dimethyl-phenoxy}-essigsäure 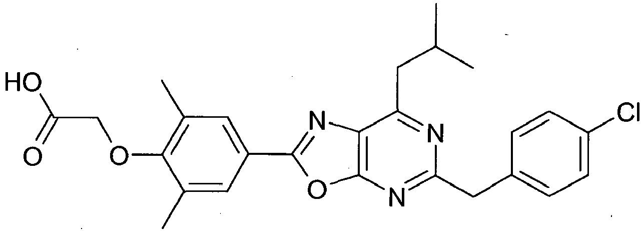 In Analogie zu Beispiel 1 (j) erhielt man aus der Umsetzung von 784 mg {4-[5-(4-Chloro-benzyl)-7-isobutyl-oxazolo[5,4-d]pyrimidin-2-yl]-2,6-dimethyl-phenoxy}-essigsäure tert-butyl ester mit Trifluoressigsäure 702 mg (100 %) der Titelverbindung.
   LC/MS (Methode LC2): Rt = 1,47 min; m/z = 480,16 [M+H]⁺

### Beispiel 8:

### [4-(5-Benzyl-7-isobutyl-oxazolo[5,4-d]pyrimidin-2-yl)-2,6-dimethyl-phenoxy]-essigsäure

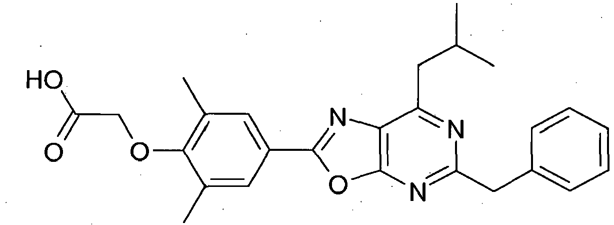

Eine Lösung von 50 mg {4-[5-(4-Chloro-benzyl)-7-isobutyl-oxazolo[5,4-d]pyrimidin-2-yl]-2,6-dimethyl-phenoxy}-essigsäure wurde mit 10 mg Palladium auf Kohle (10%) versetzt und über Nacht bei 5 bar hydriert. Es wurden dreimal 10 mg Palladium auf Kohle (10%) nachgegeben und jeweils für einen Tag hydriert. Zur Aufarbeitung wurde der Katalysator über Celite abfiltriert und die erhaltene Lösung eingeengt. Der Rückstand wurde mittels HPLC gereinigt. Man erhielt 7 mg (18 %) der Titelverbindung.
LC/MS (Methode LC2): Rt = 1,43 min; m/z = 446,30 [M+H]⁺

### Beispiel 9

### {4-[5-(4-Chloro-benzyl)-7-isopropyl-oxazolo[5,4-d]pyrimidin-2-yl]-2,6-dimethyl-phenoxy}-essigsäure

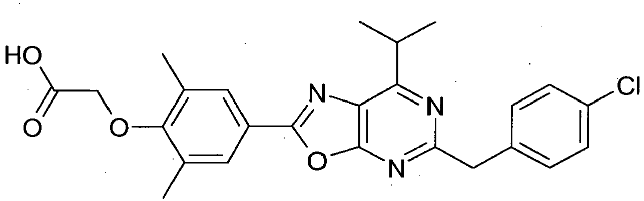

(a) 5-(4-Chloro-benzyl)-7-isopropyl-2-(4-methoxy-3,5-dimethyl-phenyl)-oxazolo[5,4-d]pyrimidin 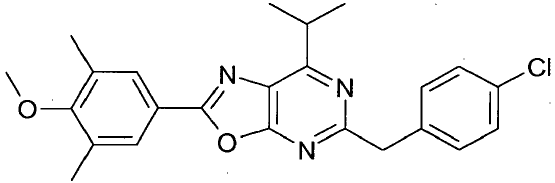 In Analogie zu Beispiel 7 (d) erhielt man aus der Umsetzung von 1,5 g 7-Chloro-5-(4-chloro-benzyl)-2-(4-methoxy-3,5-dimethyl-phenyl)-oxazolo[5,4-d]pyrimidin mit iso-Propylmagnesiumbromid 165 mg (11 %) der Titelverbindung.
   LC/MS (Methode LC2): Rt = 1,60 min; m/z = 422,16 [M+H]⁺
(b) 4-[5-(4-Chloro-benzyl)-7-isopropyl-oxazolo[5,4-d]pyrimidin-2-yl]-2,6-dimethyl-phenol 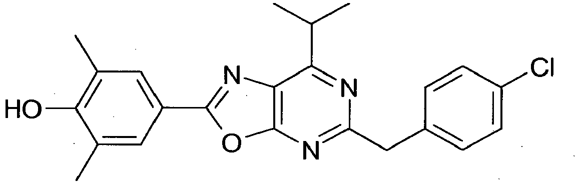 In Analogie zu Beispiel 1 (e) erhielt man aus der Umsetzung von 170 mg 5-(4-Chlorobenzyl)-7-isopropyl-2-(4-methoxy-3,5-dimethyl-phenyl)-oxazolo[5,4-d]pyrimidin mit Bortribromid 115 mg (70 %) der Titelverbindung.
   LC/MS (Methode LC2): Rt = 1,50 min; m/z = 408,14 [M+H]⁺
(c) {4-[5-(4-Chloro-benzyl)-7-isopropyl-oxazolo[5,4-d]pyrimidin-2-yl]-2,6-dimethylphenoxy}-essigsäure tert-Butylester 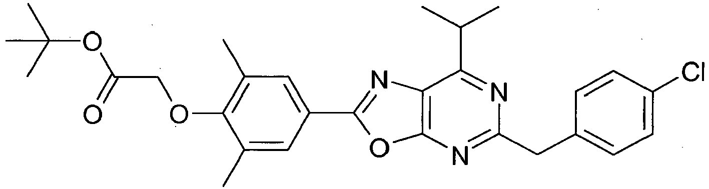 In Analogie zu Beispiel 1 (f) erhielt man aus der Umsetzung von 80 mg 4-[5-(4-Chlorobenzyl)-7-isopropyl-oxazolo[5,4-d]pyrimidin-2-yl]-2,6-dimethyl-phenol mit Bromessigsäure tert-Butylester 67 mg (65 %) der Titelverbindung.
   LC/MS (Methode LC4): Rt = 1,15 min; m/z = 522,15 [M+H]⁺
(d) {4-[5-(4-Chloro-benzyl)-7-isopropyl-oxazolo[5,4-d]pyrimidin-2-yl]-2,6-dimethylphenoxy}-essigsäure 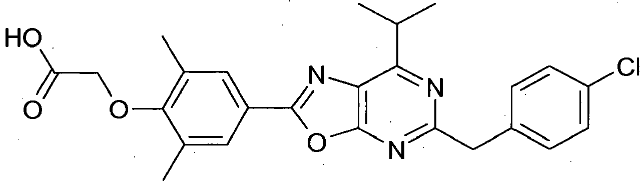 In Analogie zu Beispiel 1 (j) erhielt man aus der Umsetzung von 67 mg {4-[5-(4-Chlorobenzyl)-7-isopropyl-oxazolo[5,4-d]pyrimidin-2-yl]-2,6-dimethyl-phenoxy}-essigsäure tert-Butylester mit Trifluoressigsäure 74 mg (100 %) der Titelverbindung.
   LC/MS (Methode LC2): Rt = 1,47 min; m/z = 466,2 [M+H]⁺

### Bestimmung der pharmakologischen Wirkung

### A) GTP-γ-S-Assay mit humanen Edg-1-Rezeptoren

Zur Bestimmung der Edg-1-Rezeptor-Aktivierung durch die erfindungsgemäßen Verbindungen wurde ein GTP-γ-S-Assay (GTP-γ-S = Guanosin-5'-[thio]triphosphat) auf die Bindung an G-Protein gekoppeltem Rezeptor auf Basis des Szintillationsproximitätsassay-Prinzips verwendet, wobei ein Zellmembranpräparat einer CHO-Flp-In-Zelllinie, die den humanen Edg-1-Rezeptor konstitutiv überexprimiert, eingesetzt wurde.

### (a) Erzeugung der Zelllinie

Das Flp-In™-Expressionssystem (Invitrogen, Kat.-Nr. K6010-01) erlaubt die Erzeugung von stabilen Säugetierzelllinien, in die das interessierende Gen durch homologe Rekombination an einem spezifischen Genomort, der als FRT-Ort (FRT = Flp Recombination Target) bezeichnet wird, mit Hilfe einer durch das pOG44-Expressionsplasmid codierten Flp-Rekombinase integriert worden ist. Die Integration des pcDNA5/FRT-Expressionskonstrukts in das Flp-In-Wirtszellliniengenom führt zur Transkription des interessierenden Gens. Die stabil transfizierten Zellen werden hygromycinresistent.

Einen Tag vor der Transfektion wurden 200 000 Flp-In-CHO-Zellen in Ham-F-12-Medium (Invitrogen, Kat.-Nr. 31765) mit 10% fötalem Kälberserum (FCS; Perbio Science, Kat.-Nr. SH30068.03) in einer Platte mit 6 Vertiefungen ausgesät und über Nacht bei 37°C/5 % CO₂ inkubiert. Unter Verwendung von FuGENE^{®}-6-Transfektionsreagens (Roche, Kat.-Nr. 11988387001) wurden Zellen mit dem Flp-Rekombinase-Expressionsplasmid pOG44 und einem modifizierten Plasmid, das zusätzlich das edg-1-Gen (Zugangsnummer NM_001400) enthält und als pcDNA5-FRT-TO_nFLAG_DEST-EDG-1 bezeichnet wird, mit einem Verhältnis von 9:1 kotransfiziert. Zum Erhalt des modifizierten pcDNA5-FRT-TO_nFLAG_DEST-Plasmids wurde das Invitrogen-Plasmid pcDNA5/FRT/TO (Invitrogen, Kat.-Nr. V6520-20) durch Insertierung einer Gateway-Kassette mit ein ccdB-Gen und ein Chloramphenicolresistenzgen flankierenden attR-Rekombinationsstellen (Gateway Conversion System, Invitrogen, Kat.-Nr. 11828-029) auf das Gateway^{®}-Kloniersystem (Invitrogen) angepasst. Außerdem wurde vor der 5'-att-Rekombinationsstelle ein FLAG-Tag-Epitop hinzugefügt, um eine rekombinante Expression von Proteinen mit N-terminalem FLAG-Tag zu ermöglichen.

Für die Transfektion einer Vertiefung wurden 1,08 µg pOG44 und 0,12 µg pcDNA5-FRT-TO_nFLAG_DEST-EDG-1 mit 100 µl serumfreiem Ham-F-12-Medium mit 6 µl FuGENE^{®}-6-Transfektionsreagens gemischt. Nach 20 min Inkubation wurde der Transfektionsreagens/DNA-Komplex tropfenweise auf den Zellen verteilt. Die Zellen wurden 24 h bei 37°C inkubiert. Dann wurden die Zellen aus drei Vertiefungen in eine T75-Flasche (Greiner Cellstar^{®}, Kat.-Nr. 658175) mit Ham-F-12-Medium mit 10% FCS, aber ohne Antibiotikum, überführt und noch 24 h inkubiert. 48 h nach der Transfektion wurde das Medium durch Selektionsmedium (Ham F-12 mit 10 % FCS und 300 µg/ml Hygromycin B (Invitrogen, Kat.-Nr. 10687-010)) ersetzt. Das Medium wurde alle 2 bis 3 Tage ausgetauscht, bis eine resistente Population von Zellen herangewachsen war. Zellen wurden mehrmals aufgeteilt und in eine neue Flasche ausgesät, so daß die Zellen nicht mehr als 25% Konfluenz erreichten. Nach 2 Wochen Selektion wurden die Zellen in T175-Flaschen (Greiner Cellstar^{®}, Kat.-Nr. 660175) überführt und für die Batchproduktion kultiviert. Die Zellen wurden durch kurze Behandlung (2 bis 5 min) mit Accutase (PAA, Kat.-Nr. L11-007) aus den Kulturflaschen geerntet, in Selektionsmedium (siehe oben) resuspendiert und 5 min bei 200 x g zentrifugiert. Die Zellen wurden in einer Mischung von 90% FCS und 10% Dimethylsulfoxid resuspendiert und in flüssigem Stickstoff gefroren gelagert.

### (b) Membranpräparat

Aus der obenbeschriebenen CHO-Flp-In-Zelllinie, die den humanen Edg-1-Rezeptor konstitutiv überexprimiert, wurde nach Standardmethoden ein Membranpräparat erhalten. Kurz gesagt, wurden die kryokonservierten Zellen in Kultur genommen und in T175-Zellkulturflaschen (Becton Dickinson, Kat.-Nr. 35 5001) bis zur Konfluenz angezogen. Die Zellkultur wurde durch Waschen mit calciumfreier phosphatgepufferter Kochsalzlösung (PBS; Gibco, Kat.-Nr. 14190) gestoppt, und die Zellen wurden mit einem Gummischaber in 4°C kaltem und calciumfreiem PBS mit einem Proteaseinhibitorcocktail (Complete Protease Inhibitor; Roche, Kat.-Nr. 1697498; 1 Tablette pro 50 ml) geerntet und danach bei 4°C 15 min bei 1100 x g zentrifugiert (Heraeus Minifuge T). Zur Zelllyse wurde das Pellett in 4°C kaltem hypotonischem Puffer aus 5 mM HEPES (Sigma-Aldrich, Kat.-Nr. H-0981), 1 mM EDTA (Dinatriumsalz; Merck, Kat.-Nr. 8418) mit Proteaseinhibitorcocktail (wie oben) resuspendiert, in dem die Zellen noch 15 min auf Eis gelagert wurden. Nach der Lyse wurden die Zellen bei 4°C 10 min bei 400 x g zentrifugiert (Heraeus Minifuge T). Das Pellet wurde in einem Dounce-Homogenisator auseinandergebrochen, mit dem Überstand der vorhergehenden Zentrifugation verdünnt und danach bei 4°C 10 min bei 500 x g zentrifugiert (Heraeus Minifuge T), um Nuklei und noch intakte Zellen von den hauptsächlich im Überstand vorliegenden Membranen abzutrennen. Der Überstand wurde dann in hypotonischem Puffer verdünnt und bei 4°C bei ungefähr 18600 x g 2 h zentrifugiert (Beckmann, Avanti J251). Danach wurde das Membranpellet in einem Lagerpuffer aus 20 mM HEPES; 150 mM NaCl (Merck, Kat.-Nr. 6400), 1 mM EDTA (wie oben) mit Proteaseinhibitorcocktail (wie oben) resuspendiert. Das Membranpräparat wurde aliquotiert und bei -80°C gelagert. Die Proteinkonzentration des Membranpräparats wurde in einer Probe mit Hilfe eines kommerziellen Proteinassays (Bio-Rad, DC Protein Assay, Kat.-Nr. 500-0113, 500-0114, 500-0115) bestimmt.

### (c) GTP-γ-S-Assay

Das in (b) erhaltene Edg-1-Membranpräparat wurde in einem kommerziell erhältlichen Szintillationsproximitätsassay-Kit (SPA-Kit) auf die Bindung am G-Protein-gekoppelten Rezeptor von Amersham Biosciences/GE Healthcare (Code RPNQ0210) eingesetzt, indem die ligandeninduzierte Bindung von ³⁵S-radiomarkiertem GTP-γ-S an die rezeptorhaltige Membran, die an Szintillationsperlen gebunden ist, die Emission von Licht induziert und die Quantifizierung der in-vitro-Wirkung der Edg-1-agonistischen Verbindung erlaubt. Der Assay wurde auf einer Platte mit 96 Vertiefungen weitgehend nach den Anweisungen des Herstellers durchgeführt. Vor dem Beginn der Experimente wurden Szintillationsperlen in einem Rekonstitutionspuffer aus Tris-HCl (pH 7,4) mit 0,1% (w/v) Natriumazid suspendiert und dann auf Eis mit Assaypuffer (aus 20 mM HEPES, 100 mM NaCl, 1 mM EDTA (wie oben), 1 mM Dithiothreitol (DTT), auf pH 7,4 eingestellt) auf eine Perlenendkonzentration von 30 mg/ml verdünnt.

Die Vertiefungen wurden mit 10 µl des angegebenen Assaypuffers, 10 µl 100 µM Guanosindiphosphat-Lösung (GDP-Lösung) und 10 µl einer Lösung der Testverbindung in Assaypuffer/Dimethylsulfoxid versetzt, was eine Endkonzentration der Testverbindung von 10 µM ergibt. Anstelle der Lösung der Testverbindung wurden für die hohen Kontrollen 10 µl einer Lösung von Sphingosin-1-phosphat (S1 P; Sigma, Kat.-Nr. S-9666), was eine S1P-Endkonzentration von 10 µM ergab, und für die niedrigen Kontrollen 10 µl Assaypuffer in die jeweiligen Vertiefungen gegeben. Alle Vertiefungen enthielten äquivalente Mengen von Dimethylsulfoxid. Dann wurden in jede Vertiefung 10 µl einer [³⁵S]GTP-γ-S-Lösung (4 nM) und das in (b) erhaltene Edg-1-Membranpräparat (15 µg Membranprotein in 100 µl Assaypuffer) gegeben. Nach Inkubation der Platten bei Raumtemperatur über einen Zeitraum von 5 min wurden 50 µl der angegebenen Szintillationsperlensuspension (30 mg/ml) zugegeben. Nach einem weiteren Inkubationszeitraum von 45 min bei Raumtemperatur wurden die Platten 10 min bei 500 x g zentrifugiert. Die Quantifizierung der [³⁵S]GTP-γ-S-Bindung und somit der Rezeptoraktivierung wurde mit Hilfe eines Beta-Zählers (MicroBeta, Wallac) über einen Zeitraum von 1 min gemessen. Die Werte wurden durch Subtraktion der jeweiligen niedrigen Kontrolle hintergrundkorrigiert. Alle Messungen wurden dreifach durchgeführt. Die Rezeptoraktivierung durch die Testverbindung wird in % der jeweiligen hohen Kontrolle (10 µM S1P; wird als 100% Aktivierung erachtet) ausgedrückt. Die mit Beispielverbindungen bei 10 µM beobachteten Aktivierungen sind in Tabelle 2 aufgeführt.

**Tabelle 2: Edg-1-Rezeptor-Aktivierung durch Beispielverbindungen bei 10 µM in Prozent der Aktivierung durch 10 µM S1 P**

| Beispiel | % Aktivierung |
|---|---|
| 1 | 94 |
| 2 | 97 |
| 3 | 92 |
| 4 | 106 |
| 5 | 86 |
| 6 | 118 |
| 7 | 38 |
| 8 | 98 |
| 9 | 90 |

Aus den Messdaten ist ersichtlich, dass die Verbindungen gut zur Wundheilung und insbesondere zur Behandlung von Wundheilungsstörungen von Diabetes Patienten geeignet sind.

## Patentansprüche

1. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes, 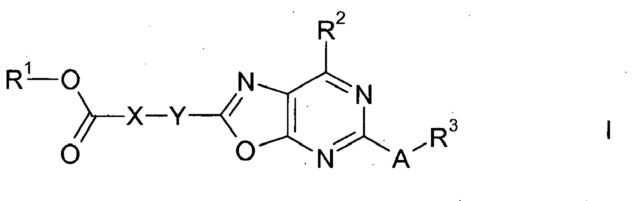 worin
A ausgewählt ist aus Bindung, -CH₂-, NH, O und S;
X ausgewählt ist aus (C₁-C₆)-Alkandiyl, (C₂-C₆)-Alkendiyl, (C₂-C₆)-Alkindiyl, (C₃-C₇)-Cycloalkandiyl, (C₁-C₆)-Alkandiyloxy und (C₃-C₇)-Cycloalkandiyloxy, die alle gegebenenfalls durch einen oder mehrere gleiche oder verschiedene Substituenten substituiert sind, die aus Fluor und Hydroxy ausgewählt sind, wobei das Sauerstoffatom der (C₁-C₆)-Alkandiyloxy- und (C₃-C₇)-Cycloalkandiyloxygruppen an die Gruppe Y gebunden ist;
Y ausgewählt ist aus Phenylen und einem zweiwertigen Rest eines aromatischen, 5-gliedrigen bis 6-gliedrigen monocyclischen Heterocyclus, der 1, 2 oder 3 gleiche oder verschiedene Ringheteroatome enthält, die aus N, O und S ausgewählt sind, wobei eines der Ringstickstoffatome ein Wasserstoffatom oder einen Substituenten R⁴ tragen kann und wobei das Phenylen und der zweiwertige Rest eines aromatischen Heterocyclus gegebenenfalls an einem oder mehreren Ringkohlenstoffatomen durch gleiche oder verschiedene Substituenten R⁵ substituiert sind;
R¹ ausgewählt ist aus Wasserstoff, (C₁-C₄)-Alkyl und (C₃-C₇)-Cycloalkyl-C_{z}H_{2z}-, worin z aus 0, 1 und 2 ausgewählt ist;
R² ausgewählt ist aus (C₁-C₆)-Alkyl, (C₃-C₅)-Cycloalkyl-CₓH₂ₓ-, Het¹-CₙH₂ₙ-, worin x und n aus 0, 1 und 2 ausgewählt sind;
R³ ausgewählt ist aus (C₁-C₆)-Alkyl, wobei der Alkylrest gegebenenfalls durch ein oder mehrere Fluoratome substituiert ist, (C₂-C₆)-Alkenyl, (C₂-C₆)-Alkinyl, (C₃-C₇)-Cycloalkyl-CᵤH₂ᵤ- und Het²-CᵥH₂ᵥ-, worin u und v aus 1 und 2 ausgewählt sind, oder R³ für einen Rest eines gesättigten oder ungesättigten, 3-gliedrigen bis 10-gliedrigen, monocyclischen oder bicyclischen Rings, der 0, 1, 2, 3 oder 4 gleiche oder verschiedene Ringheteroatome enthält, die aus N, O und S ausgewählt sind, steht, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist;
R⁴ ausgewählt ist aus (C₁-C₄)-Alkyl, (C₃-C₇)-Cycloalkyl-C_{w}H_{2w}- und Oxy, wobei w aus 0, 1 und 2 ausgewählt ist;
R⁵ ausgewählt ist aus Halogen, Hydroxy, (C₁-C₄)-Alkyl-, (C₃-C₅)-Cycloalkyl-C_{z}H_{2z}, (C₁-C₄)-Alkyloxy, (C₁-C₄)-Alkyl-S(O)ₘ-, Amino, Nitro, Cyano, Hydroxycarbonyl, (C₁-C₄)-Alkyloxycarbonyl, Aminocarbonyl und Aminosulfonyl, wobei z aus 0, 1 und 2 ausgewählt ist;
R³¹ ausgewählt ist aus Halogen, Hydroxy, (C₁-C₄)-Alkyl, (C₁-C₄)-Alkyloxy, (C₃-C₇)-Cycloalkyl, Oxo, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Nitro, Cyano, (C₁-C₄)-Alkylcarbonyl, Aminosulfonyl, (C₁-C₄)-Alkylaminosulfonyl und Di((C₁-C₄)-alkyl)aminosulfonyl;
Het¹ für einen Rest eines gesättigten, 4-gliedrigen bis 6-gliedrigen, monocyclischen gesättigten Heterocyclus steht, der 1 oder 2 gleiche oder verschiedene Ringheteroatome enthält, die unter O und S ausgewählt sind, und der über ein Ringkohlenstoffatom gebunden ist, wobei ein Ringschwefelatom eine oder zwei Oxogruppen tragen können und wobei der Rest eines Heterocyclus gegebenenfalls durch einen oder mehrere gleiche oder verschiedene Substituenten substituiert ist, die unter Fluor und (C₁-C₄)-Alkyl ausgewählt sind;
Het² für einen Rest eines gesättigten, 4-gliedrigen bis 7-gliedrigen, monocyclischen Heterocyclus steht, der 1 oder 2 gleiche oder verschiedene Ringheteroatome enthält, die unter N, O und S ausgewählt sind, und der über ein Ringkohlenstoffatom gebunden ist, wobei der Rest eines Heterocyclus gegebenenfalls durch einen oder mehrere gleiche oder verschiedene Substituenten substituiert ist, die unter Fluor und (C₁-C₄)-Alkyl ausgewählt sind;
m aus 0, 1 und 2 ausgewählt ist.

2. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach Anspruch 1, **dadurch gekennzeichnet, daß** darin bedeuten
A ausgewählt ist aus Bindung, -CH₂-, NH, O und S;
X ausgewählt ist aus (C₁-C₆)-Alkandiyl, (C₂-C₆)-Alkendiyl, (C₂-C₆)-Alkindiyl, (C₃-C₇)-Cycloalkandiyl, (C₁-C₆)-Alkandiyloxy und (C₃-C₇)-Cycloalkandiyloxy; die alle gegebenenfalls durch einen oder mehrere gleiche oder verschiedene Substituenten substituiert sind, die aus Fluor und Hydroxy ausgewählt sind, wobei das Sauerstoffatom der (C₁-C₆)-Alkandiyloxy- und (C₃-C₇)-Cycloalkandiyloxygruppen an die Gruppe Y gebunden ist;
Y ausgewählt ist aus Phenylen und einem zweiwertigen Rest eines aromatischen, 5-gliedrigen bis 6-gliedrigen monocyclischen Heterocyclus, der 1, 2 oder 3 gleiche oder verschiedene Ringheteroatome enthält, die aus N, O und S ausgewählt sind, wobei eines der Ringstickstoffatome ein Wasserstoffatom oder einen Substituenten R⁴ tragen kann und wobei das Phenylen und der zweiwertige Rest eines aromatischen Heterocyclus gegebenenfalls an einem oder mehreren Ringkohlenstoffatomen durch gleiche oder verschiedene Substituenten R⁵ substituiert sind;
R¹ ausgewählt ist aus Wasserstoff, (C₁-C₄)-Alkyl und (C₃-C₇)-Cycloalkyl-C_{z}H_{2z}-, worin z aus 0, 1 und 2 ausgewählt ist;
R² ausgewählt ist aus (C₁-C₆)-Alkyl, (C₃-C₅)-Cycloalkyl-CₓH₂ₓ- und Het¹-CₙH₂ₙ-, worin x und n aus 0, 1 und 2 ausgewählt sind;
R³ ausgewählt ist aus (C₁-C₆)-Alkyl, wobei der Alkylrest gegebenenfalls durch ein oder mehrere Fluoratome substituiert ist, (C₂-C₆)-Alkenyl, (C₂-C₆)-Alkinyl, (C₃-C₇)-Cycloalkyl-CᵤH₂ᵤ- und Het²-CₙH₂ₙ-, worin u und n aus 1 und 2 ausgewählt sind, oder R³ für einen Rest eines gesättigten oder ungesättigten, 3-gliedrigen bis 10-gliedrigen, monocyclischen oder bicyclischen Rings, der 0, 1, 2, 3 oder 4 gleiche oder verschiedene Ringheteroatome enthält, die aus N, O und S ausgewählt sind, steht, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist;
R⁴ ausgewählt ist aus (C₁-C₄)-Alkyl, (C₃-C₇)-Cycloalkyl-C_{w}H_{2w}- und Oxy, wobei w aus 0, 1 und 2 ausgewählt ist;
R⁵ ausgewählt ist aus Halogen, Hydroxy, (C₁-C₄)-Alkyl-, (C₃-C₅)-Cycloalkyl-C_{z}H_{2z}-, (C₁-C₄)-Alkyloxy, (C₁-C₄)-Alkyl-S(O)ₘ-, Amino, Nitro, Cyano, Hydroxycarbonyl, (C₁-C₄)-Alkyloxycarbonyl, Aminocarbonyl und Aminosulfonyl, wobei z aus 0, 1 und 2 ausgewählt ist;
R³¹ ausgewählt ist aus Halogen, Hydroxy, (C₁-C₄)-Alkyl, (C₁-C₄)-Alkyloxy, (C₃-C₇)-Cycloalkyl, Oxo, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Nitro, Cyano, (C₁-C₄)-Alkylcarbonyl, Aminosulfonyl, (C₁-C₄)-Alkylaminosulfonyl und Di((C₁-C₄)-alkyl)aminosulfonyl;
Het¹ für einen Rest eines gesättigten, 4-gliedrigen bis 6-gliedrigen, monocyclischen gesättigten Heterocyclus steht, der 1 oder 2 Sauerstoffatome enthält, und der über ein Ringkohlenstoffatom gebunden ist, und wobei der Rest eines Heterocyclus gegebenenfalls durch einen oder mehrere gleiche oder verschiedene Substituenten substituiert ist, die unter Fluor und (C₁-C₄)-Alkyl ausgewählt sind;
Het² für einen Rest eines gesättigten, 4-gliedrigen bis 7-gliedrigen, monocyclischen Heterocyclus steht, der 1 oder 2 gleiche oder verschiedene Ringheteroatome enthält, die unter N, O und S ausgewählt sind, und der über ein Ringkohlenstoffatom gebunden ist, wobei der Rest eines Heterocyclus gegebenenfalls durch einen oder mehrere gleiche oder verschiedene Substituenten substituiert ist, die unter Fluor und (C₁-C₄)-Alkyl ausgewählt sind;
m aus 0, 1 und 2 ausgewählt ist.

3. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes gemäß Anspruch 1 oder 2, **dadurch gekennzeichnet, daß** darin bedeuten
A -CH₂- oder O;
X (C₁-C₆)-Alkandiyloxy, wobei das Sauerstoffatom der (C₁-C₆)-Alkandiyloxygruppe an die Gruppe Y gebunden ist;
Y Phenylen, wobei das Phenylen gegebenenfalls an einem oder mehreren Ringkohlenstoffatomen durch gleiche oder verschiedene Substituenten R⁵ substituiert ist;
R¹ Wasserstoff oder (C₁-C₄)-Alkyl;
R² (C₁-C₆)-Alkyl;
R³ (C₁-C₆)-Alkyl, wobei der Alkylrest gegebenenfalls durch ein oder mehrere Fluoratome substituiert ist, oder Phenyl, wobei der Phenylrest gegebenenfalls an einem oder mehreren Ringkohlenstoffatomen durch gleiche oder verschiedene Substituenten R³¹ substituiert ist;
R⁵ Halogen, Hydroxy, (C₁-C₄)-Alkyl-, (C₃-C₅)-Cycloalkyl-C_{z}H_{2z}-, (C₁-C₄)-Alkyloxy, (C₁-C₄)-Alkyl-S(O)ₘ-, Amino, Nitro, Cyano, Hydroxycarbonyl, (C₁-C₄)-Alkyloxycarbonyl, Aminocarbonyl oder Aminosulfonyl, wobei z aus 0, 1 und 2 ausgewählt ist;
R³¹ Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Nitro, Cyano, (C₁-C₄)-Alkylcarbonyl, Aminosulfonyl, (C₁-C₄)-Alkylaminosulfonyl oder Di((C₁-C₄)-alkyl)aminosulfonyl;
m aus 0, 1 und 2 ausgewählt ist.

4. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes gemäß einem oder mehreren der Ansprüche 1 bis 3, **dadurch gekennzeichnet, daß** darin bedeuten
A -CH₂- oder O;
X (C₁-C₆)-Alkandiyloxy, wobei das Sauerstoffatom der (C₁-C₆)-Alkandiyloxygruppe an die Gruppe Y gebunden ist;
Y Phenylen, wobei das Phenylen gegebenenfalls an einem oder mehreren Ringkohlenstoffatomen durch gleiche oder verschiedene Substituenten R⁵ substituiert ist;
R¹ Wasserstoff;
R² (C₁-C₆)-Alkyl;
R³ (C₁-C₆)-Alkyl, wobei der Alkylrest gegebenenfalls durch ein oder mehrere Fluoratome substituiert ist, oder Phenyl, wobei der Phenylrest gegebenenfalls an einem oder mehreren Ringkohlenstoffatomen durch gleiche oder verschiedene Substituenten R³¹ substituiert ist;
R⁵ (C₁-C₄)-Alkyl;
R³¹ Halogen.

5. Pharmazeutische Zusammensetzung, enthaltend mindestens eine Verbindung der Formel I nach einem der Ansprüche 1 bis 4 oder ein physiologisch akzeptables Salz davon oder ein physiologisch akzeptables Solvat einer derartigen Verbindung oder eines derartigen Salzes und einen pharmazeutisch akzeptablen Träger.

6. Pharmazeutische Zusammensetzung, nach Anspruch 5, dadurch gekenzeichnet, dass es sich um eine Hydrogelzubereitung handelt.

7. Verbindung der Formel I nach einem der Ansprüche 1 bis 4 oder physiologisch akzeptables Salz davon oder physiologisch akzeptables Solvat einer derartigen Verbindung oder eines derartigen Salzes zur Verwendung als Arzneimittel.

8. Verbindung der Formel I nach einem der Ansprüche 1 bis 4 oder eines physiologisch akzeptablen Salzes davon oder eines physiologisch akzeptablen Solvats einer derartigen Verbindung oder eines derartigen Salzes zur Behandlung von Wundheilungsstörungen.

9. Verbindung der Formel I nach einem der Ansprüche 1 bis 4 oder eines physiologisch akzeptablen Salzes davon oder eines physiologisch akzeptablen Solvats einer derartigen Verbindung oder eines derartigen Salzes zur Wundheilung.

10. Verbindung der Formel I nach einem der Ansprüche 1 bis 4 oder eines physiologisch akzeptablen Salzes davon oder eines physiologisch akzeptablen Solvats einer derartigen Verbindung oder eines derartigen Salzes zur Wundheilung bei Diabetikern.

11. Verbindung der Formel I nach einem der Ansprüche 1 bis 4 oder eines physiologisch akzeptablen Salzes davon oder eines physiologisch akzeptablen Solvats einer derartigen Verbindung oder eines derartigen Salzes zur Behandlung des Diabetischen Fußsyndroms.

12. Verbindung der Formel I nach einem der Ansprüche 1 bis 4 oder eines physiologisch akzeptablen Salzes davon oder eines physiologisch akzeptablen Solvats einer derartigen Verbindung oder eines derartigen Salzes zur Behandlung von Herz-Kreislauf-Erkrankungen.

13. Verbindung der Formel I nach einem der Ansprüche 1 bis 4 oder eines physiologisch akzeptablen Salzes davon oder eines physiologisch akzeptablen Solvats einer derartigen Verbindung oder eines derartigen Salzes zur Cardioprotektion.

## Claims

1. Compound of the formula I, in any of its stereoisomeric forms, or mixture of stereoisomeric forms in any ratio, or physiologically acceptable salt thereof, or physiologically acceptable solvate of such a compound or such a salt, 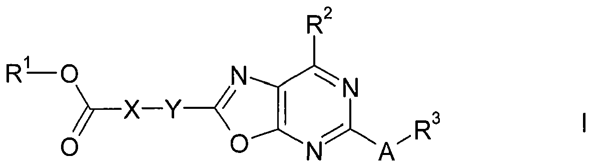 wherein
A is selected from the group consisting of a bond, -CH₂-, NH, O and S ;
X is selected from the group consisting of (C₁-C₆)-alkanediyl, (C₂-C₆)-alkenediyl, (C₂-C₆) -alkynediyl, (C₃-C₇)-cycloalkanediyl, (C₁-C₆)-alkanediyloxy and (C₃-C₇)-cycloalkanediyloxy, all of which are optionally substituted by one or more identical or different substituents selected from the group consisting of fluorine and hydroxyl, where the oxygen atom of the (C₁-C₆)-alkanediyloxy and (C₃-C₇)-cycloalkanediyloxy groups is attached to group Y;
Y is selected from the group consisting of phenylene and a bivalent radical of an aromatic 5-membered or 6-membered monocyclic heterocycle which contains 1, 2 or 3 identical or different ring heteroatoms selected from the group consisting of N, 0 and S, where one of the ring nitrogen atoms may carry a hydrogen atom or a substituent R⁴ and where the phenylene and the bivalent radical of an aromatic heterocycle are optionally substituted at one or more ring carbon atoms by identical or different substituents R⁵;
R¹ is selected from the group consisting of hydrogen, (C₁-C₄)-alkyl and (C₃-C₇)-cycloalkyl-C_{z}H_{2z}-, where z is selected from the group consisting of 0, 1 and 2;
R² is selected from the group consisting of (C₁-C₆)-alkyl, (C₃-C₅)-cycloalkyl-CₓH₂ₓ-, Het¹-CₙH₂ₙ-, where x and n are selected from the group consisting of 0, 1 and 2; R³ is selected from the group consisting of (C₁-C₆)-alkyl, where the alkyl radical is optionally substituted by one or more fluorine atoms, (C₂-C₆)-alkenyl, (C₂-C₆)-alkynyl, (C₃-C₇) -cycloalkyl-CᵤH₂ᵤ- and Het²-CᵥH₂ᵥ-, where u and v are selected from the group consisting of 1 and 2, or R³ is a radical of a saturated or unsaturated 3-membered to 10-membered monocyclic or bicyclic ring which contains 0, 1, 2, 3 or 4 identical or different ring heteroatoms selected from the group consisting of N, 0 and S, where one or two of the ring nitrogen atoms may carry a hydrogen atom or a (C₁-C₄)-alkyl substituent and one or two of the ring sulfur atoms may carry one or two oxo groups and where the radical of a ring is optionally substituted at one or more ring carbon atoms by identical or different substituents R³¹;
R⁴ is selected from the group consisting of (C₁-C₄)-alkyl, (C₃-C₇)-cycloalkyl-C_{w}H_{2w}- and oxy, where w is selected from the group consisting of 0, 1 and 2;
R⁵ is selected from the group consisting of halogen, hydroxyl, (C₁-C₄) -alkyl-, (C₃-C₅)-cycloalkyl-C_{z}H_{2z}-, (C₁-C₄)-alkyloxy, (C₁-C₄) -alkyl-S(O)ₘ-, amino, nitro, cyano, hydroxycarbonyl, (C₁-C₄)-alkyloxycarbonyl, aminocarbonyl and aminosulfonyl, where z is selected from the group consisting of 0, 1 and 2;
R³¹ is selected from the group consisting of halogen, hydroxyl, (C₁-C₄)-alkyl, (C₁-C₄)-alkyloxy, (C₃-C₇)-cycloalkyl, oxo, (C₁-C₄)-alkyl-S(O)ₘ-, amino, (C₁-C₄)-alkylamino, di((C₁-C₄)-alkyl)amino, (C₁-C₄)-alkylcarbonylamino, (C₁-C₄)-alkylsulfonylamino, nitro, cyano, (C₁-C₄) -alkylcarbonyl, aminosulfonyl, (C₁-C₄)-alkylaminosulfonyl and di((C₁-C₄)-alkyl)aminosulfonyl;
Het¹ is a radical of a saturated 4-membered to 6-membered monocyclic saturated heterocycle which contains 1 or 2 identical or different ring heteroatoms selected from the group consisting of O and S and which is attached via a ring carbon atom, where a ring sulfur atom may carry one or two oxo groups and where the radical of a heterocycle is optionally substituted by one or more identical or different substituents selected from the group consisting of fluorine and (C₁-C₄)-alkyl;
Het² is a radical of a saturated 4-membered to 7-membered monocyclic heterocycle which contains 1 or 2 identical or different ring heteroatoms selected from the group consisting of N, O and S and which is attached via a ring carbon atom, where the radical of a heterocycle is optionally substituted by one or more identical or different substituents selected from the group consisting of fluorine and (C₁-C₄)-alkyl;
m is selected from the group consisting of 0, 1 and 2.

2. Compound of the formula I, in any of its stereoisomeric forms, or a mixture of stereoisomeric forms in any ratio, or physiologically acceptable salt thereof, or physiologically acceptable solvate of such a compound or such a salt according to Claim 1, **characterized in that**
A is selected from the group consisting of a bond, -CH₂-, NH, O and S;
X is selected from the group consisting of (C₁-C₆)-alkanediyl, (C₂-C₆)-alkenediyl, (C₂-C₆)-alkynediyl, (C₃-C₇) -cycloalkanediyl, (C₁-C₆)-alkanediyloxy and (C₃-C₇)-cycloalkanediyloxy; all of which are optionally substituted by one or more identical or different substituents selected from the group consisting of fluorine and hydroxyl, where the oxygen atom of the (C₁-C₆)-alkanediyloxy and (C₃-C₇)-cycloalkanediyloxy groups is attached to group Y;
Y is selected from the group consisting of phenylene and a bivalent radical of an aromatic 5-membered or 6-membered monocyclic heterocycle which contains 1, 2 or 3 identical or different ring heteroatoms selected from the group consisting of N, O and S, where one of the ring nitrogen atoms may carry a hydrogen atom or a substituent R⁴ and where the phenylene and the bivalent radical of an aromatic heterocycle are optionally substituted at one or more ring carbon atoms by identical or different substituents R⁵;
R¹ is selected from the group consisting of hydrogen, (C₁-C₄)-alkyl and (C₃-C₇)-cycloalkyl-C_{z}H_{2z}-, where z is selected from the group consisting of O, 1 and 2;
R² is selected from the group consisting of (C₁-C₆)-alkyl, (C₃-C₅)-cycloalkyl-CₓH₂ₓ- and Het¹-CₙH₂ₙ-, where x and n are selected from the group consisting of 0, 1 and 2;
R³ is selected from the group consisting of (C₁-C₆)-alkyl, where the alkyl radical is optionally substituted by one or more fluorine atoms, (C₂-C₆)-alkenyl, (C₂-C₆)-alkynyl, (C₃-C₇) -cycloalkyl-CᵤH₂ᵤ- und Het²-CₙH₂ₙ-, where u and n are selected from the group consisting of 1 and 2, or R³ is a radical of a saturated or unsaturated 3-membered to 10-membered monocyclic or bicyclic ring which contains 0, 1, 2, 3 or 4 identical or different ring heteroatoms selected from the group consisting of N, O and S, where one or two of the ring nitrogen atoms may carry a hydrogen atom or a (C₁-C₄)-alkyl substituent and one or two of the ring sulfur atoms may carry one or two oxo groups and where the radical of a ring is optionally substituted at one or more ring carbon atoms by identical or different substituents R³¹;
R⁴ is selected from the group consisting of (C₁-C₄)-alkyl, (C₃-C₇)-cycloalkyl-C_{w}H_{2w}- and oxy, where w is selected from the group consisting of 0, 1 and 2;
R⁵ is selected from the group consisting of halogen, hydroxyl, (C₁-C₄)-alkyl-, (C₃-C₅)-cycloalkyl-C_{z}H_{2z}-, (C₁-C₄)-alkyloxy, (C₁-C₄) -alkyl-S (O)ₘ-, amino, nitro, cyano, hydroxycarbonyl, (C₁-C₄)-alkyloxycarbonyl, aminocarbonyl and aminosulfonyl, where z is selected from the group consisting of 0, 1 and 2;
R³¹ is selected from the group consisting of halogen, hydroxyl, (C₁-C₄) -alkyl, (C₁-C₄) -alkyloxy, (C₃-C₇)-cycloalkyl, oxo, (C₁-C₄)-alkyl-S(O)ₘ-, amino, (C₁-C₄)-alkylamino, di ((C₁-C₄)-alkyl) amino, (C₁-C₄)-alkylcarbonylamino, (C₁-C₄)-alkylsulfonylamino, nitro, cyano, (C₁-C₄) -alkylcarbonyl, aminosulfonyl, (C₁-C₄)-alkylaminosulfonyl and di((C₁-C₄)-alkyl)aminosulfonyl;
Het¹ is a radical of a saturated 4-membered to 6-membered monocyclic saturated heterocycle which contains 1 or 2 oxygen atoms and which is attached via a ring carbon atom, and where the radical of a heterocycle is optionally substituted by one or more identical or different substituents selected from the group consisting of fluorine and (C₁-C₄)-alkyl;
Het² is a radical of a saturated 4-membered to 7-membered monocyclic heterocycle which contains 1 or 2 identical or different ring heteroatoms selected from the group consisting of O and S and which is attached via a ring carbon atom, where the radical of a heterocycle is optionally substituted by one or more identical or different substituents selected from the group consisting of fluorine and (C₁-C₄)-alkyl;
m is selected from the group consisting of 0, 1 and 2.

3. Compound of the formula I, in any of its stereoisomeric forms, or a mixture of stereoisomeric forms in any ratio, or physiologically acceptable salt thereof, or physiologically acceptable solvate of such a compound or such a salt according to Claim 1 or 2, **characterized in that**
A is -CH₂- or O;
X is (C₁-C₆)-alkanediyloxy, where the oxygen atom of the (C₁-C₆)-alkanediyloxy group is attached to group Y;
Y is phenylene, where the phenylene is optionally substituted at one or more ring carbon atoms by identical or different substituents R⁵;
R¹ is hydrogen or (C₁-C₄)-alkyl;
R² is (C₁-C₆)-alkyl;
R³ is (C₁-C₆)-alkyl, where the alkyl radical is optionally substituted by one or more fluorine atoms, or phenyl, where the phenyl radical is optionally substituted at one or more ring carbon atoms by identical or different substituents R³¹;
R⁵ is halogen, hydroxyl, (C₁-C₄)-alkyl-, (C₃-C₅)-cycloalkyl-C_{z}H_{2z}-, (C₁-C₄)-alkyloxy, (C₁-C₄)-alkyl-S(O)ₘ-, amino, nitro, cyano, hydroxycarbonyl, (C₁-C₄)-alkyloxycarbonyl, aminocarbonyl or aminosulfonyl, where z is selected from the group consisting of 0, 1 and 2;
R³¹ is halogen, (C₁-C₄)-alkyl, (C₃-C₇)-cycloalkyl, hydroxyl, (C₁-C₄)-alkyloxy, oxo, (C₁-C₄)-alkyl-S(O)ₘ-, amino, (C₁-C₄)-alkylamino, di((C₁-C₄)-alkyl) amino, (C₁-C₄)-alkylcarbonylamino, (C₁-C₄)-alkylsulfonylamino, nitro, cyano, (C₁-C₄)-alkylcarbonyl, aminosulfonyl, (C₁-C₄)-alkylaminosulfonyl or di ((C₁-C₄)-alkyl) aminosulfonyl;
m is selected from the group consisting of 0, 1 and 2.

4. Compound of the formula I, in any of its stereoisomeric forms, or a mixture of stereoisomeric forms in any ratio, or physiologically acceptable salt thereof, or physiologically acceptable solvate of such a compound or such a salt according to one or more of Claims 1 to 3, **characterized in that**
A is -CH₂- or O;
X is (C₁-C₆)-alkanediyloxy, where the oxygen atom of the (C₁-C₆)-alkanediyloxy group is attached to group Y;
Y is phenylene, where the phenylene is optionally substituted at one or more ring carbon atoms by identical or different substituents R⁵;
R¹ is hydrogen;
R² is (C₁-C₆)-alkyl;
R³ is (C₁-C₆)-alkyl, where the alkyl radical is optionally substituted by one or more fluorine atoms, or phenyl, where the phenyl radical is optionally substituted at one or more ring carbon atoms by identical or different substituents R³¹;
R⁵ is (C₁-C₄)-alkyl;
R³¹ is halogen.

5. Pharmaceutical composition, comprising at least one compound of the formula I according to any of Claims 1 to 4 or a physiologically acceptable salt thereof, or a physiologically acceptable solvate of such a compound or such a salt, and a pharmaceutically acceptable carrier.

6. Pharmaceutical composition according to Claim 5, **characterized in that** the pharmaceutical composition is a hydrogel preparation.

7. Compound of the formula I according to any of Claims 1 to 4 or physiologically acceptable salt thereof, or physiologically acceptable solvate of such a compound or such a salt, for use as a medicament.

8. Compound of the formula I according to any of Claims 1 to 4 or a physiologically acceptable salt thereof, or a physiologically acceptable solvate of such a compound or such a salt, for the treatment of wound healing disorders.

9. Compound of the formula I according to any of Claims 1 to 4 or a physiologically acceptable salt thereof, or a physiologically acceptable solvate of such a compound or such a salt, for wound healing.

10. Compound of the formula I according to any of Claims 1 to 4 or a physiologically acceptable salt thereof, or a physiologically acceptable solvate of such a compound or such a salt, for wound healing in diabetics.

11. Compound of the formula I according to any of Claims 1 to 4 or a physiologically acceptable salt thereof, or a physiologically acceptable solvate of such a compound or such a salt, for the treatment of diabetic foot syndrome.

12. Compound of the formula I according to any of Claims 1 to 4 or a physiologically acceptable salt thereof, or a physiologically acceptable solvate of such a compound or such a salt, for the treatment of cardiovascular disorders.

13. Compound of the formula I according to any of Claims 1 to 4 or a physiologically acceptable salt thereof, or a physiologically acceptable solvate of such a compound or such a salt, for cardioprotection.

## Revendications

1. Composé de formule I sous l'une quelconque de ses formes stéréoisomères ou un mélange de formes stéréoisomères en un rapport quelconque ou sel physiologiquement acceptable de celui-ci ou solvate physiologiquement acceptable d'un tel composé ou d'un tel sel 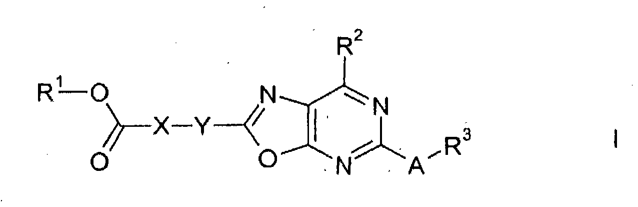 dans laquelle
A est choisi parmi une liaison, -CH₂-, NH, O et S ;
X est choisi parmi alcanediyle en (C₁-C₆), alcènediyle en (C₂-C₆), alcynediyle en (C₂-C₆), cycloalcanediyle en (C₃-C₇), alcanediyloxy en (C₁-C₆) et cycloalcanediyloxy en (C₃-C₇), qui sont tous éventuellement substitués par un ou plusieurs substituants identiques ou différents, choisis parmi fluor et hydroxy, l'atome d'oxygène des groupes alcanediyloxy en (C₁-C₆) et cycloalcanediyloxy en (C₃-C₇) étant relié au groupe Y ;
Y est choisi parmi phénylène et un radical bivalent d'un hétérocycle monocyclique aromatique de 5 à 6 éléments, qui contient 1, 2 ou 3 hétéroatomes de cycle identiques ou différents, choisis parmi N, O et S, un des atomes d'azote de cycle pouvant porter un atome d'hydrogène ou un substituant R⁴, et le phénylène et le radical bivalent d'un hétérocycle aromatique étant éventuellement substitués sur un ou plusieurs atomes de carbone de cycle par des substituants R⁵ identiques ou différents ;
R¹ est choisi parmi hydrogène, alkyle en (C₁-C₄) et cycloalkyle en (C₃-C₇)-C_{z}H_{2z}-, z étant choisi parmi 0, 1 et 2 ;
R² est choisi parmi alkyle en (C₁-C₆), cycloalkyle en (C₃-C₅) -CₓH₂ₓ-, Het¹-CₙH₂ₙ-, x et n étant choisis parmi 0, 1 et 2 ;
R³ est choisi parmi alkyle en (C₁-C₆), le radical alkyle étant éventuellement substitué par un ou plusieurs atomes de fluor, alcényle en (C₂-C₆), alcynyle en (C₂-C₆), cycloalkyle en (C₃-C₇)-CᵤH₂ᵤ- et Het²-CᵥH₂ᵥ-, u et v étant choisis parmi 1 et 2, ou R³ représente un radical d'un cycle monocyclique ou bicyclique saturé ou insaturé, de 3 à 10 éléments, qui contient 0, 1, 2, 3 ou 4 hétéroatomes de cycle identiques ou différents, choisis parmi N, O et S, un ou deux des atomes d'azote de cycle pouvant porter un atome d'hydrogène ou un substituant alkyle en (C₁-C₄), et un ou deux des atomes de soufre de cycle pouvant porter un ou deux groupes oxo, et le radical d'un cycle étant éventuellement substitué sur un ou plusieurs atomes de carbone de cycle par des substituants R³¹ identiques ou différents ;
R⁴ est choisi parmi alkyle en (C₁-C₄), cycloalkyle en (C₃-C₇)-C_{w}H_{2w}- et oxy, w étant choisi parmi 0, 1 et 2 ;
R⁵ est choisi parmi halogène, hydroxy, alkyle en (C₁-C₄), cycloalkyle en (C₃-C₅)-C_{z}H_{2z}-, alkyloxy en (C₁-C₄), alkyle en (C₁-C₄)-S(O)ₘ-, amino, nitro, cyano, hydroxycarbonyle, alkyloxycarbonyle en (C₁-C₄), aminocarbonyle et aminosulfonyle, z étant choisi parmi 0, 1 et 2 ;
R³¹ est choisi parmi halogène, hydroxy, alkyle en (C₁-C₄), alkyloxy en (C₁-C₄), cycloalkyle en (C₃-C₇), oxo, alkyle en (C₁-C₄)-S(O)ₘ-, amino, alkylamino en (C₁-C₄), di(alkyle en (C₁-C₄))amino, alkylcarbonylamino en (C₁-C₄), alkylsulfonylamino en (C₁-C₄), nitro, cyano, alkylcarbonyle en (C₁-C₄), aminosulfonyle, alkylaminosulfonyle en (C₁-C₄) et di (alkyle en (C₁-C₄))aminosulfonyle ;
Het¹ représente un radical d'un hétérocycle monocyclique saturé de 4 à 6 éléments, qui contient 1 ou 2 hétéroatomes de cycle identiques ou différents, choisis parmi O et S, et qui est relié par un atome de carbone de cycle, un atome de soufre de cycle pouvant porter un ou deux groupes oxo et le radical d'un hétérocycle étant éventuellement substitué par un ou plusieurs substituants identiques ou différents, choisis parmi fluor et alkyle en (C₁-C₄) ;
Het² représente un radical d'un hétérocycle monocyclique saturé de 4 à 7 éléments, qui contient 1 ou 2 hétéroatomes de cycle identiques ou différents, choisis parmi N, O et S, et qui est relié par un atome de carbone de cycle, le radical d'un hétérocycle étant éventuellement substitué par un ou plusieurs substituants identiques ou différents, choisis parmi fluor et alkyle en (C₁-C₄) ;
m est choisi parmi 0, 1 et 2.

2. Composé de formule I sous l'une quelconque de ses formes stéréoisomères ou un mélange de formes stéréoisomères en un rapport quelconque ou sel physiologiquement acceptable de celui-ci ou solvate physiologiquement acceptable d'un tel composé ou d'un tel sel selon la revendication 1, **caractérisé en ce que**
A est choisi parmi une liaison, -CH₂-, NH, O et S ;
X est choisi parmi alcanediyle en (C₁-C₆), alcènediyle en (C₂-C₆), alcynediyle en (C₂-C₆), cycloalcanediyle en (C₃-C₇), alcanediyloxy en (C₁-C₆) et cycloalcanediyloxy en (C₃-C₇), qui sont tous éventuellement substitués par un ou plusieurs substituants identiques ou différents, choisis parmi fluor et hydroxy, l'atome d'oxygène des groupes alcanediyloxy en (C₁-C₆) et cycloalcanediyloxy en (C₃-C₇) étant relié au groupe Y ;
Y est choisi parmi phénylène et un radical bivalent d'un hétérocycle monocyclique aromatique de 5 à 6 éléments, qui contient 1, 2 ou 3 hétéroatomes de cycle identiques ou différents, choisis parmi N, O et S, un des atomes d'azote de cycle pouvant porter un atome d'hydrogène ou un substituant R⁴, et le phénylène et le radical bivalent d'un hétérocycle aromatique étant éventuellement substitués sur un ou plusieurs atomes de carbone de cycle par des substituants R⁵ identiques ou différents ;
R¹ est choisi parmi hydrogène, alkyle en (C₁-C₄) et cycloalkyle en (C₃-C₇)-C_{z}H_{2z}-, z étant choisi parmi 0, 1 et 2 ;
R² est choisi parmi alkyle en (C₁-C₆), cycloalkyle en (C₃-C₅)-CₓH₂ₓ-, Het¹-CₙH₂ₙ-, x et n étant choisis parmi 0, 1 et 2 ;
R³ est choisi parmi alkyle en (C₁-C₆), le radical alkyle étant éventuellement substitué par un ou plusieurs atomes de fluor, alcényle en (C₂-C₆), alcynyle en (C₂-C₆), cycloalkyle en (C₃-C₇)-CᵤH₂ᵤ- et Het²-CₙH₂ₙ-, u et n étant choisis parmi 1 et 2, ou R³ représente un radical d'un cycle monocyclique ou bicyclique saturé ou insaturé, de 3 à 10 éléments, qui contient 0, 1, 2, 3 ou 4 hétéroatomes de cycle identiques ou différents, choisis parmi N, 0 et S, un ou deux des atomes d'azote de cycle pouvant porter un atome d'hydrogène ou un substituant alkyle en (C₁-C₄), et un ou deux des atomes de soufre de cycle pouvant porter un ou deux groupes oxo, et le radical d'un cycle étant éventuellement substitué sur un ou plusieurs atomes de carbone de cycle par des substituants R³¹ identiques ou différents ;
R⁴ est choisi parmi alkyle en (C₁-C₄), cycloalkyle en (C₃-C₇)-C_{w}H_{2w}- et oxy, w étant choisi parmi 0, 1 et 2 ;
R⁵ est choisi parmi halogène, hydroxy, alkyle en (C₁-C₄), cycloalkyle en (C₃-C₅)-C_{z}H_{2z}-, alkyloxy en (C₁-C₄), alkyle en (C₁-C₄)-S(O)ₘ-, amino, nitro, cyano, hydroxycarbonyle, alkyloxycarbonyle en (C₁-C₄), aminocarbonyle et aminosulfonyle, z étant choisi parmi 0, 1 et 2 ;
R³¹ est choisi parmi halogène, hydroxy, alkyle en (C₁-C₄), alkyloxy en (C₁-C₄), cycloalkyle en (C₃-C₇), oxo, alkyle en (C₁-C₄)-S(O)ₘ-, amino, alkylamino en (C₁-C₄), di(alkyle en (C₁-C₄))amino, alkylcarbonylamino en (C₁-C₄), alkylsulfonylamino en (C₁-C₄), nitro, cyano, alkylcarbonyle en (C₁-C₄), aminosulfonyle, alkylaminosulfonyle en (C₁-C₄) et di (alkyle en (C₁-C₄))aminosulfonyle ;
Het¹ représente un radical d'un hétérocycle monocyclique saturé de 4 à 6 éléments, qui contient 1 ou 2 atomes d'oxygène, et qui est relié par un atome de carbone de cycle, et le radical d'un hétérocycle étant éventuellement substitué par un ou plusieurs substituants identiques ou différents, choisis parmi fluor et alkyle en (C₁-C₄) ;
Het² représente un radical d'un hétérocycle monocyclique saturé de 4 à 7 éléments, qui contient 1 ou 2 hétéroatomes de cycle identiques ou différents, choisis parmi N, 0 et S, et qui est relié par un atome de carbone de cycle, le radical d'un hétérocycle étant éventuellement substitué par un ou plusieurs substituants identiques ou différents, choisis parmi fluor et alkyle en (C₁-C₄) ;
m est choisi parmi 0, 1 et 2.

3. Composé de formule I sous l'une quelconque de ses formes stéréoisomères ou un mélange de formes stéréoisomères en un rapport quelconque ou sel physiologiquement acceptable de celui-ci ou solvate physiologiquement acceptable d'un tel composé ou d'un tel sel selon la revendication 1 ou 2, **caractérisé en ce que**
A signifie -CH₂- ou O ;
X signifie alcanediyloxy en (C₁-C₆), l'atome d'oxygène du groupe alcanediyloxy en (C₁-C₆) étant relié au groupe Y ;
Y signifie phénylène, le phénylène étant éventuellement substitués sur un ou plusieurs atomes de carbone de cycle par des substituants R⁵ identiques ou différents ;
R¹ signifie hydrogène ou alkyle en (C₁-C₄) ;
R² signifie alkyle en (C₁-C₆) ;
R³ signifie alkyle en (C₁-C₆), le radical alkyle étant éventuellement substitué par un ou plusieurs atomes de fluor, ou phényle, le radical phényle étant éventuellement substitué sur un ou plusieurs atomes de carbone de cycle par des substituants R³¹ identiques ou différents ;
R⁵ signifie halogène, hydroxy, alkyle en (C₁-C₄), cycloalkyle en (C₃-C₅)-C_{z}H_{2z}-, alkyloxy en (C₁-C₄), alkyle en (C₁-C₄)-S(O)ₘ-, amino, nitro, cyano, hydroxycarbonyle, alkyloxycarbonyle en (C₁-C₄), aminocarbonyle ou aminosulfonyle, z étant choisi parmi 0, 1 et 2 ;
R³¹ signifie halogène, alkyle en (C₁-C₄), cycloalkyle en (C₃-C₇), hydroxy, alkyloxy en (C₁-C₄), oxo, alkyle en (C₁-C₄)-S(O)ₘ-, amino, alkylamino en (C₁-C₄), di (alkyle en (C₁-C₄) amino, alkylcarbonylamino en (C₁-C₄), alkylsulfonylamino en (C₁-C₄), nitro, cyano, alkylcarbonyle en (C₁-C₄), aminosulfonyle, alkylaminosulfonyle en (C₁-C₄) ou di (alkyle en (C₁-C₄))aminosulfonyle ;
m est choisi parmi 0, 1 et 2.

4. Composé de formule I sous l'une quelconque de ses formes stéréoisomères ou un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 3, **caractérisé en ce que**
A signifie -CH₂- ou O ;
X signifie alcanediyloxy en (C₁-C₆), l'atome d'oxygène du groupe alcanediyloxy en (C₁-C₆) étant relié au groupe Y ;
Y signifie phénylène, le phénylène étant éventuellement substitués sur un ou plusieurs atomes de carbone de cycle par des substituants R⁵ identiques ou différents ;
R¹ signifie hydrogène ;
R² signifie alkyle en (C₁-C₆) ;
R³ signifie alkyle en (C₁-C₆), le radical alkyle étant éventuellement substitué par un ou plusieurs atomes de fluor, ou phényle, le radical phényle étant éventuellement substitué sur un ou plusieurs atomes de carbone de cycle par des substituants R³¹ identiques ou différents ;
R⁵ signifie alkyle en (C₁-C₄) ;
R³¹ signifie halogène.

5. Composition pharmaceutique, contenant au moins un composé de formule I selon l'une quelconque des revendications 1 à 4 ou un sel physiologiquement acceptable de celui-ci ou un solvate physiologiquement acceptable d'un tel composé ou d'un tel sel, et un véhicule pharmaceutiquement acceptable.

6. Composition pharmaceutique selon la revendication 5, **caractérisée en ce qu'**il s'agit d'une préparation d'hydrogel.

7. Composé de formule I selon l'une quelconque des revendications 1 à 4 ou sel physiologiquement acceptable de celui-ci ou solvate physiologiquement acceptable d'un tel composé ou d'un tel sel, destiné à une utilisation en tant que médicament.

8. Composé de formule I selon l'une quelconque des revendications 1 à 4 ou sel physiologiquement acceptable de celui-ci ou solvate physiologiquement acceptable d'un tel composé ou d'un tel sel, pour le traitement de troubles de la cicatrisation.

9. Composé de formule I selon l'une quelconque des revendications 1 à 4 ou sel physiologiquement acceptable de celui-ci ou solvate physiologiquement acceptable d'un tel composé ou d'un tel sel, pour la cicatrisation.

10. Composé de formule I selon l'une quelconque des revendications 1 à 4 ou sel physiologiquement acceptable de celui-ci ou solvate physiologiquement acceptable d'un tel composé ou d'un tel sel, pour la cicatrisation chez les diabétiques.

11. Composé de formule I selon l'une quelconque des revendications 1 à 4 ou sel physiologiquement acceptable de celui-ci ou solvate physiologiquement acceptable d'un tel composé ou d'un tel sel, pour le traitement du syndrome du pied diabétique.

12. Composé de formule I selon l'une quelconque des revendications 1 à 4 ou sel physiologiquement acceptable de celui-ci ou solvate physiologiquement acceptable d'un tel composé ou d'un tel sel, pour le traitement de maladies cardiovasculaires.

13. Composé de formule I selon l'une quelconque des revendications 1 à 4 ou sel physiologiquement acceptable de celui-ci ou solvate physiologiquement acceptable d'un tel composé ou d'un tel sel, pour la cardioprotection.
